# EUROPEAN PATENT APPLICATION

(11) **EP 3 998 107 A1**
(43) Date of publication of application: **18.05.2022**
(21) Application number: 20836518.9
(22) Date of filing: 07.07.2020
(51) Int. Cl.: A61P 3/10, C07D 471/04, C12N 5/0735, C12N 5/10, A61K 35/39, A61K 35/545

(54) **METHOD FOR PRODUCING INSULIN-PRODUCING CELL USING DIHYDROINDOLIZINONE DERIVATIVES**

(30) Priority: 08.07.2019 JP 2019126861
(71) Applicant: Tokyo Institute of Technology, Tokyo 152-8550 (JP); Daiichi Sankyo Company, Limited, Tokyo 103-8426 (JP)
(72) Inventor: KUME, Shoen, Tokyo 152-8550 (JP); SHIRAKI, Nobuaki, Tokyo 152-8550 (JP); YANO, Tatsuya, Tokyo 103-8426 (JP); KIHO, Toshihiro, Tokyo 103-8426 (JP)
(74) Representative: Wallace, Sheila Jane
(86) International application number: PCT/JP2020/026543
(87) International publication number: WO 2021/006268

(57) **Abstract**

As an approach of efficiently inducing differentiation from pluripotent stem cells into insulin-producing cells, provided is a method comprising the step of three-dimensionally culturing cells in a medium containing a dihydroindolizinone derivative.

## Description

### [Technical Field]

The present invention relates to a method for producing insulin-producing cells from pluripotent stem cells by three-dimensional culture.

### [Background Art]

Diabetes develops with various genetic factors and environmental factors as the background and is a severe disease that significantly reduces the QOL of patients as a result of complications such as nephropathy induced by chronic hyperglycemia. Currently, the number of diabetics in the world exceeds 400 million, which is also problematic in view of medical care economics. Diabetes is roughly classified into type 1 and type 2 diabetes, and loss of pancreatic β cells having an insulin secretory function is a major cause in both pathological conditions. Administration of insulin preparations is a common method for treating type 1 diabetes and severe type 2 diabetes with significant loss of pancreatic β cells, but there are many problems such as side effects including hypoglycemia and the necessity of frequent self-injection. In recent years, transplantation of pancreatic islet cells isolated from an organ donor into a type 1 diabetic patient has become possible and is expected as a treatment method which replaces insulin treatment and enables complete remission of diabetes. However, wide adoption is difficult due to lack of pancreatic islet donors. Therefore, it is desired to urgently realize a technique for producing insulin-producing cells in a large amount from pluripotent stem cells.

As a technique for producing insulin-producing cells from pluripotent stem cells, a method of inducing differentiation of ES cells or iPS cells into insulin-producing cells through a 5-stage to 7-stage process using a compound represented by the following formula has been reported (Patent Document 1 and Non Patent Documents 1, 2, 3, 4, and 5). In the method of Shahjalal, et al. (Patent Document 1 and Non Patent Document 4), insulin-producing cells can be produced from human iPS cells stepwise through a 5-stage differentiation process. First, iPS cells grown in a maintenance medium are cultured in a medium containing activin A or the GSK3β inhibitor CHIR99201 for several days in stage 1, to induce Sox17-positive definitive endoderm cells. In stage 2, the definitive endoderm cells are treated with FGF10 or the sonic hedgehog inhibitor KAAD-cyclopamine for several days, to induce Foxa2-positive primitive gut tube cells. Further, in stage 3, the primitive gut tube cells are treated with a medium containing retinoic acid, KAAD-cyclopamine, the TGFβ receptor kinase inhibitor SB431542, and the BMP signal inhibitor Noggin for several days, to induce differentiation into PDX1-positive pancreatic progenitor cells. In stage 4, the pancreatic progenitor cells are stimulated with the protein kinase C activator indolactam V, ALk5 inhibitor II that is a TGFβ receptor kinase inhibitor, and Noggin, to induce Ngn3-positive pancreatic endocrine progenitor cells. In stage 5 of the final stage, the pancreatic endocrine progenitor cells are cultured in a medium containing a GLP-1 receptor agonist and nicotinamide for several days. Thereby, insulin-producing cells are obtained.

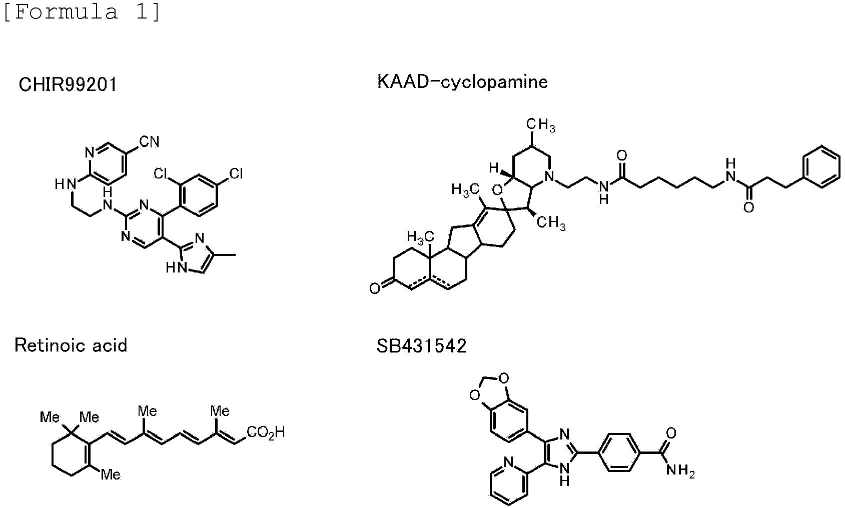

Further, there is a report using a compound represented by the following formula. There may be cases of using the small molecule inhibitor LDN193189, instead of Noggin used in the aforementioned differentiation culture, and SANT-1 (Non Patent Documents 2 and 6) or Dorsomorphin (Non Patent Document 5), instead of KAAD-cyclopamine. Further, in the final step of the differentiation culture, there may be cases of using Forskolin or Dexamethasone as a differentiation inducer (Non Patent Document 5). Other than the above, the AXL inhibitor R428 is reported as a compound that promotes the functional maturation of insulin-producing cells (Non Patent Document 3), and the AKT inhibitor AT7867 is reported as a compound that promotes the growth of PDX-1-positive pancreatic progenitor cells (Non Patent Document 7) .

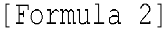

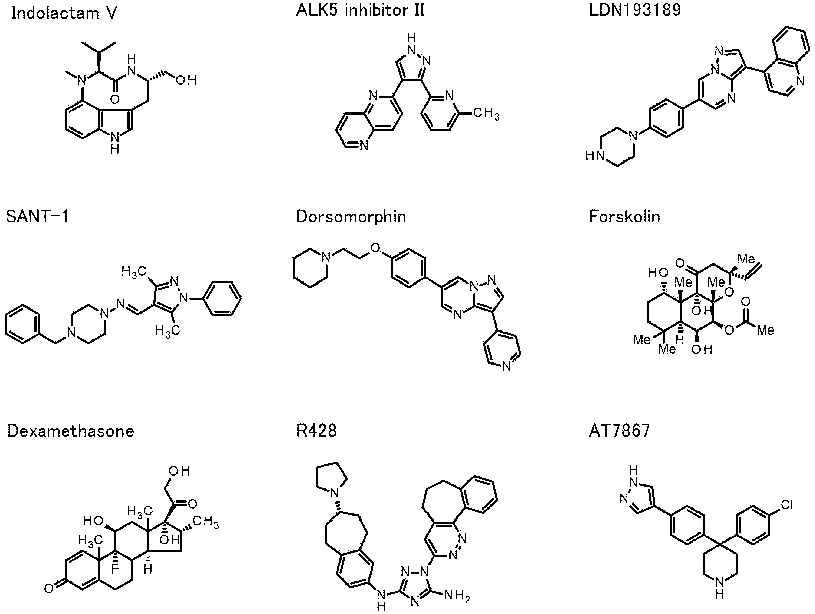

In order to apply insulin-producing cells derived from pluripotent stem cells to cell therapy, the stability of cell functions and the efficiency of the production method are important. The stability of cell functions means that the insulin-producing cells obtained exhibit good reproducibility and constant ability in the ability to secrete insulin in response to high glucose and the insulin secretion dynamics in each experiment. These abilities vary depending on the production lot or cell line in the cells obtained by a conventional method, and it is thus difficult to ensure a stable quality, which is a problem. Concerning the efficiency of the production method, there is a problem of poor cost efficiency in conventional methods due to the small number of insulin-producing cells capable of inducing differentiation.

### [Citation List]

### [Patent Documents]

[Patent Document 1] International Application Publication No. 2015/178397

### [Non Patent Documents]

[Non Patent Document 1] Kroon, E. et al., Pancreatic endoderm derived from human embryonic stem cells generates glucose-responsive insulin-secreting cells in vivo. Nature Biotechnology, 26: 443-452, 2008.
[Non Patent Document 2] Pagliuca F.W., et al., Generation of functional human pancreatic β cells in vitro. Cell, 159: 428-439, 2014.
[Non Patent Document 3] Rezania A. et al., Reversal of diabetes with insulin-producing cells derived in vitro from human pluripotent stem cells. Nature Biotechnology, 32: 1122-1133, 2014.
[Non Patent Document 4] Shahjalal H. et al., Generation of insulin-producing β-like cells from human iPS cells in a defined and completely xeno-free culture system. Journal of Molecular Cell Biology, 6: 394-408, 2014.
[Non Patent Document 5] Kunisada Y. et al., Small molecules induce efficient differentiation into insulin-producing cells from human induced pluripotent stem cells. Stem Cell Research, 8: 274-284, 2012.
[Non Patent Document 6] Nakashima R. et al., Neural cells play an inhibitory role in pancreatic differentiation of pluripotent stem cells. Genes Cells, 20: 1028-1045, 2015.
[Non Patent Document 7] Kimura A. et al., Small molecule AT7867 proliferates PDX1-expressing pancreatic progenitor cells derived from human pluripotent stem cells. Stem Cell Research, 24: 61-68, 2017.

### [Summary of Invention]

### [Technical Problem]

An object of the present invention is to provide an approach achieving high differentiation efficiency from stem cells into insulin-producing cells, which has been difficult with conventional techniques.

### [Solution to Problem]

As a result of diligent studies, the inventors have found that a compound represented by formula (I) or a salt thereof has a remarkable effect of promoting induction of differentiation from pluripotent stem cells into insulin-producing cells, and further the compound or a salt thereof is useful for producing insulin-producing cells, thereby accomplishing the present invention. The compound represented by formula (I) has a new structure that is completely different from known differentiation inducers and exerts an effect of further enhancing the efficiency of the induction of differentiation in the later steps of the differentiation process more than known differentiation-promoting compounds and growth factors.

That is, the present invention relates to [1] to [21] described below.
[1] A method for producing insulin-producing cells by differentiating pluripotent stem cells into insulin-producing cells, comprising the step of three-dimensionally culturing cells in a medium containing a compound represented by formula (I): 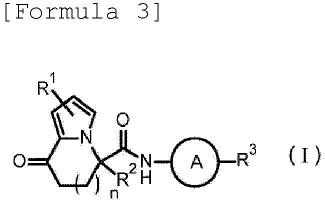 wherein each substituent is defined as follows:
   R¹ represents a hydrogen atom, a halogen atom, or a C1-C6 alkyl group;
   R² represents a hydrogen atom or a C1-C6 alkyl group;
   R³ represents an aryl group optionally substituted with one to four substituents independently selected from a substituent group α, a C5-C10 cycloalkenyl group optionally substituted with one to four substituents independently selected from the substituent group α, or a heterocyclyl group optionally substituted with one to four substituents independently selected from the substituent group α;
   the substituent group α includes a halogen atom, a cyano group, a carboxy group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, a phenoxy group optionally substituted with one to four substituents independently selected from a substituent group β, a phenyl group optionally substituted with one to four substituents independently selected from the substituent group β, and a benzoyl group optionally substituted with one to four substituents independently selected from the substituent group β;
   the substituent group β includes a halogen atom, a C1-C6 alkyl group, a C1-C6 alkoxy group, a halo-C1-C6 alkyl group, a halo-C1-C6 alkoxy group, and a (C1-C6 alkoxy)carbonyl group;
   n represents 0 or 1; and
   A represents a group represented by any one of formulae (i) to (iv) below: 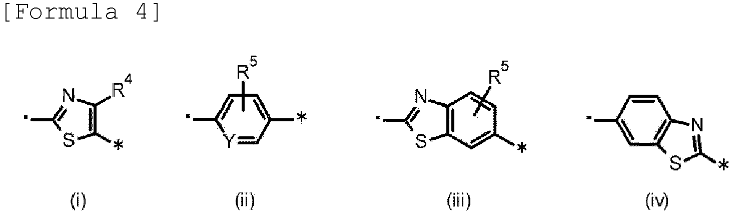 wherein each substituent is defined as follows:
      • and * each represent a bond, where · is bonded to a nitrogen atom in an amido group of formula (I), and * is bonded to R³;
      R⁴ represents a hydrogen atom, a C1-C6 alkyl group, a halo-C1-C6 alkyl group, or a (C1-C6 alkoxy)carbonyl group;
      R⁵ represents a hydrogen atom, a halogen atom, or a C1-C6 alkyl group; and
      Y represents N or CH; or a salt thereof.
[2] A method for producing insulin-producing cells according to [1], wherein
   R³ in the compound represented by formula (I) represents a naphthyl group, a 1,3-benzodioxolyl group, a 2,2-dihalo-1,3-benzodioxolyl group, a C5-C10 cycloalkenyl group, a phenyl group optionally substituted with one or two substituents independently selected from a substituent group α1, or a 5- or 6-membered heterocyclyl group optionally substituted with one or two substituents independently selected from the substituent group α1;
   the substituent group α1 includes a halogen atom, a cyano group, a carboxy group, a phenoxy group, a benzoyl group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a 5- or 6-membered non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, and a phenyl group optionally substituted with one or two substituents independently selected from a substituent group β1; and
   the substituent group β1 includes a halogen atom, a C1-C6 alkyl group, a C1-C6 alkoxy group, and a (C1-C6 alkoxy)carbonyl group.
[3] A method for producing insulin-producing cells according to [1], wherein
   R³ in the compound represented by formula (I) represents a naphthyl group, a 1,3-benzodioxolyl group, a 2,2-dihalo-1,3-benzodioxolyl group, a C5-C10 cycloalkenyl group, a phenyl group optionally substituted with one or two substituents independently selected from a substituent group α2, or a 5- or 6-membered heterocyclyl group optionally substituted with one or two substituents independently selected from a substituent group γ2;
   the substituent group α2 includes a halogen atom, a cyano group, a carboxy group, a phenoxy group, a benzoyl group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a 5- or 6-membered non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, and a phenyl group optionally substituted with one or two substituents independently selected from a substituent group β2;
   the substituent group β2 includes a halogen atom, a C1-C6 alkyl group, and a C1-C6 alkoxy group; and
   the substituent group γ2 includes a halogen atom, a C1-C6 alkyl group, a C1-C6 alkoxy group, a (C1-C6 alkyl)carbonyl group, and a (C1-C6 alkoxy)carbonyl group, or a salt thereof.
[4] A method for producing insulin-producing cells according to [1], wherein
   R³ in the compound represented by formula (I) represents a naphthyl group, a 1,3-benzodioxolyl group, a 2,2-difluoro-1,3-benzodioxolyl group, a C5-C8 cycloalken-1-yl group, a phenyl group optionally substituted with one or two substituents independently selected from a substituent group α3, or a 5- or 6-membered heterocyclyl group optionally substituted with one or two substituents independently selected from a substituent group γ3;
   the substituent group α3 includes a halogen atom, a cyano group, a carboxy group, a phenoxy group, a benzoyl group, a C1-C4 alkyl group, a C1-C4 alkoxy group, a halo-C1-C2 alkyl group, a halo-C1-C2 alkoxy group, a hydroxy C1-C4 alkyl group, a C1-C2 alkoxy C1-C2 alkoxy group, a (C1-C4 alkyl)carbonyl group, a (C1-C4 alkoxy)carbonyl group, a (C1-C4 alkoxy)carbonyloxy group, a phenyl C1-C4 alkoxy group, a morpholin-1-yl group, a carbamoyl group optionally substituted with one or two C1-C4 alkyl groups, a C1-C2 alkoxy group substituted by a carbamoyl group optionally substituted with one or two C1-C4 alkyl groups, a sulfamoyl group substituted with one or two C1-C4 alkyl groups, and a phenyl group optionally substituted with one or two substituents independently selected from a substituent group β3;
   the substituent group β3 includes a fluorine atom, a chlorine atom, a C1-C4 alkyl group, and a C1-C4 alkoxy group; and
   the substituent group γ3 includes a halogen atom, a C1-C4 alkyl group, a C1-C4 alkoxy group, a (C1-C4 alkyl)carbonyl group, and a (C1-C4 alkoxy)carbonyl group.
[5] A method for producing insulin-producing cells according to any one of [1] to [4], wherein R¹ in the compound represented by formula (I) represents a hydrogen atom, a chlorine atom, or a methyl group.
[6] A method for producing insulin-producing cells according to any one of [1] to [5], wherein R² in the compound represented by formula (I) represents a hydrogen atom or a methyl group.
[7] A method for producing insulin-producing cells according to any one of [1] to [6], wherein A in the compound represented by formula (I) represents a group represented by formula (i), and R⁴ represents a hydrogen atom, a C1-C6 alkyl group, a halo-C1-C6 alkyl group, or a (C1-C6 alkoxy)carbonyl group.
[8] A method for producing insulin-producing cells according to [7], wherein R⁴ in the compound represented by formula (I) represents a hydrogen atom, a methyl group, or a trifluoromethyl group.
[9] A method for producing insulin-producing cells according to any one of [1] to [6], wherein A in the compound represented by formula (I) represents a group represented by formula (ii), and R⁵ represents a hydrogen atom, a halogen atom, or a C1-C6 alkyl group.
[10] A method for producing insulin-producing cells according to [9], wherein R⁵ in the compound represented by formula (I) represents a hydrogen atom, a fluorine atom, or a methyl group.
[11] A method for producing insulin-producing cells according to any one of [1] to [6], wherein A in the compound represented by formula (I) represents a group represented by formula (iii), and R⁵ represents a hydrogen atom, a fluorine atom, or a methyl group.
[12] A method for producing insulin-producing cells according to any one of [1] to [6], wherein A in the compound represented by formula (I) represents a group represented by formula (iv).
[13] A method for producing insulin-producing cells according to any one of [1] to [12], wherein n in the compound represented by formula (I) represents 1.
[14] A method for producing insulin-producing cells according to any one of [1] to [13], wherein R³ in the compound represented by formula (I) represents a 2,2-difluoro-1,3-benzodioxolyl group, a 1-tert-butoxycarbonyl-3,6-dihydro-2H-pyridin-4-yl group, or a phenyl group optionally substituted with one or two substituents independently selected from the group consisting of a fluorine atom, a chlorine atom, a trifluoromethyl group, a tert-butoxy group, a trifluoromethoxy group, a 2,2,2-trifluoroethoxy group, a benzyloxy group, and a phenoxy group.
[15] A method for producing insulin-producing cells according to any one of [1] to [13], wherein R³ in the compound represented by formula (I) represents a phenyl group, or a phenyl group substituted at m position or p position with any one substituent selected from the group consisting of a fluorine atom, a chlorine atom, a trifluoromethyl group, a tert-butoxy group, a trifluoromethoxy group, a 2,2,2-trifluoroethoxy group, a benzyloxy group, and a phenoxy group.
[16] A method for producing insulin-producing cells according to [1], wherein the compound represented by formula (I) is any one selected from the compound group shown below: 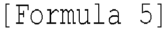 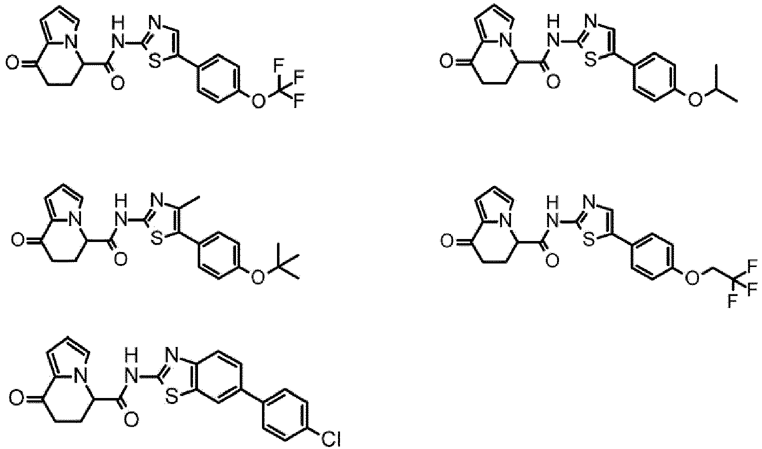
[17] A method for producing insulin-producing cells according to [1], wherein the pluripotent stem cells are human ES cells or human iPS cells.
[18] A method for producing insulin-producing cells according to [1], wherein the three-dimensional culture is performed in a low-adhesive or non-adhesive culture container.
[19] A method for producing insulin-producing cells according to any one of [1] to [18], wherein the differentiation process from pluripotent stem cells into insulin-producing cells comprises steps 1 to 5 below, and at least one step selected from the group consisting of step 3, step 4 and step 5 comprises culturing cells in a medium containing the compound represented by formula (I) or a salt thereof:
   step 1 of inducing definitive endoderm cells from pluripotent stem cells;
   step 2 of inducing primitive gut tube cells from the definitive endoderm cells;
   step 3 of inducing pancreatic progenitor cells from the primitive gut tube cells;
   step 4 of inducing pancreatic endocrine progenitor cells from the pancreatic progenitor cells; and
   step 5 of inducing insulin-producing cells from the pancreatic endocrine progenitor cells.
[20] A method for producing insulin-producing cells according to [19], wherein in step 3, step 4 and step 5, cells are cultured in a medium containing the compound represented by formula (I) or a salt thereof.
[21] A method for producing insulin-producing cells according to [19], wherein in step 1, pluripotent stem cells pretreated with a methionine-depleted medium is used.
[22] Insulin-producing cells derived from pluripotent stem cells, the insulin-producing cells being produced by a method according to any one of [1] to [21].
[23] A therapeutic drug for a disease caused by abnormal insulin secretion or insulin secretory disorder, comprising insulin-producing cells derived from pluripotent stem cells, the insulin-producing cells being produced by a method according to any one of [1] to [21].
[24] The therapeutic drug according to [23], wherein the disease caused by abnormal insulin secretion or insulin secretory disorder is type 1 diabetes or type 2 diabetes.

This description encompasses the contents described in the description and/or drawings of Japanese Patent Application No. 2019-126861 on which the priority of the present application is based.

### [Advantageous Effects of Invention]

A compound represented by formula (I) of the present invention or a salt thereof has a remarkable effect when differentiating pluripotent stem cells derived from mammals into insulin-producing cells, as compared with known differentiation induction methods. Accordingly, a method of the present invention using this compound can efficiently produce insulin-producing cells. Further, a method of the present invention three-dimensionally cultures cells and can therefore culture cells in an environment closer to an *in vivo* environment, as compared with two-dimensional culture methods.

### [Brief Description of Drawings]

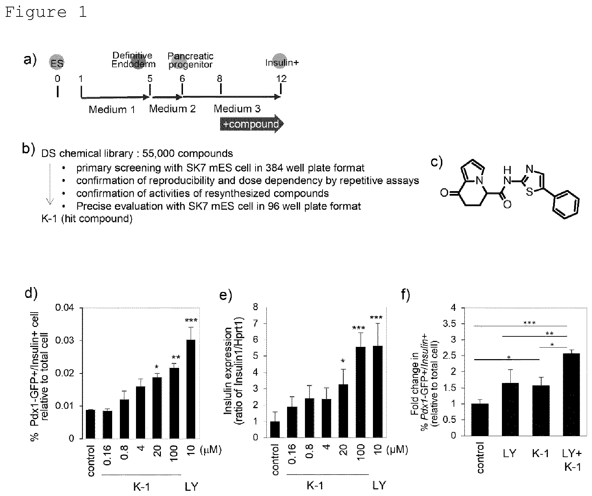

[Figure 1] Screening of a novel small-molecule compound that promotes differentiation of β cells. Figure 1a) is a schematic view of a culture system for screening for a compound that enhances β cell differentiation using SK7 mES cells. Figure 1b) summarizes a screening flow of a β cell differentiation promoter. Figure 1c) shows the chemical structure of K-1. Figure 1d) shows the effect of K-1 on the ratio of Pdx1-GFP+Ins+ double-positive cells to all cells. Figure 1e) shows an insulin 1 gene expression level. In Figures 1d) and 1e), significance in Dunnett' s' multiple comparison test is indicated by *p < 0.05, **p < 0.01, or ***p < 0.001. Figure 1f) shows the additive effect of a γ-secretase inhibitor LY411575 and K-1. The ratio of Pdx1-GFP+ and insulin+ double-positive cells to all cells (DAPI+) was calculated. An insulin 1 mRNA expression level was normalized with a Hprt1 expression level. The value is indicated by mean ± SEM of three experiments. Significance in one-tailed independent t-test is indicated by *p < 0.05, **p < 0.01, or ***p < 0.001.

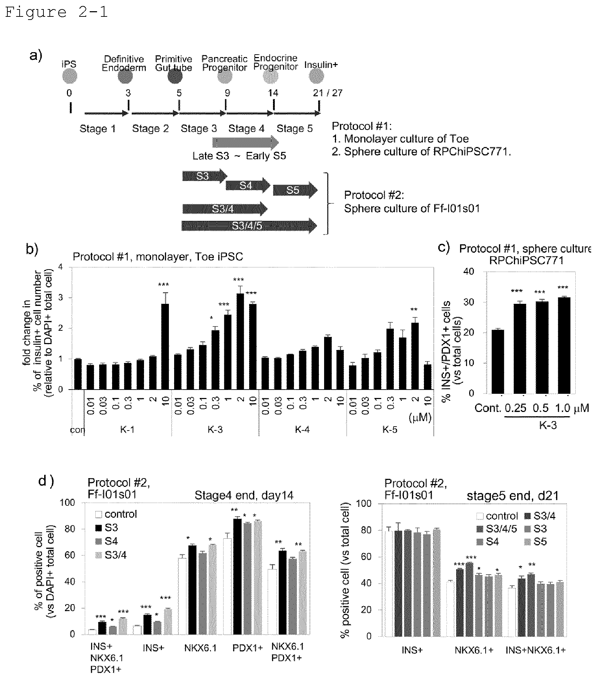

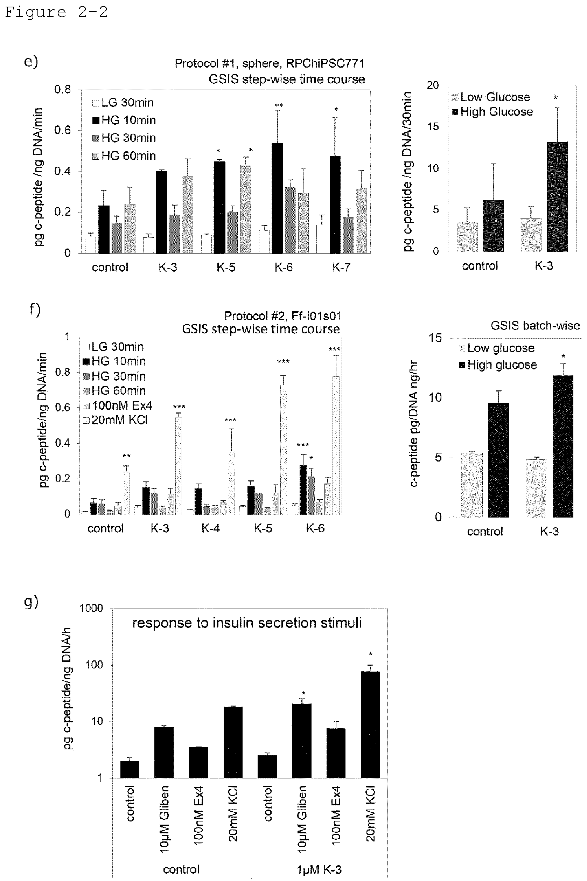

[Figure 2-1] K-1 and derivatives thereof promote differentiation of β cells derived from human iPS cells.
Figure 2-1a) summarizes a culture system for evaluating the effectiveness of a compound for β cell differentiation using a human iPS cell line (Toe, RPChiPS771, and Ffl-01s01). Figure 2-1b) shows fold change in the ratio of the number of obtained INS+ cells to the total number of cells from a DMSO control in monolayer culture of Toe hiPS cells. Figure 2-1c) shows the ratio of produced INS+PDX1+ double-positive cells to all cells in sphere culture according to differentiation protocol #1 using RPChiPSC771 iPS cells. Figure 2-1d) shows the positivity of a molecular marker at the end of stage 4 (left) or the end of stage 5 (right) in iPS-derived cells. Treatment with K-3 was performed in different time windows, i.e., stage 3 (S3), stage 4 (S4), stage 5 (S5), stages 3 & 4 (S3/4), or stages 3, 4 & 5 (S3/4/5). Culture was performed according to differentiation protocol #2 using Ff-I01s01. In Figures 2-1b), 2-1c) and 2-1d), significance in Dunnett' s' multiple comparison test is indicated by *p < 0.05, **p < 0.01, or ***p < 0.001.
[Figure 2-2] K-1 and derivatives thereof promote differentiation of β cells derived from human iPS cells. The left graph of Figure 2-2e) shows the time-dependent GSIS activity of iPS-β cells treated with 1 µM K-3 or a derivative thereof or a negative control. The treatment was performed between stages 3 and 4. Sphere culture was performed according to differentiation protocol #1. RPChiPSC771 iPS cells were used. Measurement was performed by a step-wise time-course method. In the right graph of Figure 2-2e), the GSIS activity of iPS-β cells treated with K-3 or a negative control was measured in a batch-wise manner between stages 3 and 4. The left graph of Figure 2-2f) shows the time-dependent GSIS activity of iPS-β cells. The iPS-β cells were treated with 1 µM K-3, -5 or -6 or 2 µM K-4 or a negative control between stages 3 and 4. Sphere culture was performed according to differentiation protocol #2. Ff-I01s01 iPS cells were used. Measurement was performed by a step-wise time-course method. In the right graph of Figure 2-2f), the GSIS activity of iPS-β cells treated with K-3 or a negative control was measured in a batch-wise manner between stages 3 and 4. Figure 2-2g) shows a test on the response of iPS-β cells treated with K-3 or a negative control (DMSO) to different secretagogues (100 nM exendin 4, 10 µM glibenclamide, and 20 mM KCl). Significance in Dunnett' s' multiple comparison test is indicated by *p < 0.05, **p < 0.01, or ***p < 0.001. In the left graphs of Figures 2-2e) and 2-2f), significance in Dunnett' s' multiple comparison test is indicated by *p < 0.05, **p < 0.01, or ***p < 0.001. In the right graphs of Figures 2-2e) and 2-2f) and Figure 2-2g), significance in one-tailed independent t-test is indicated by *p < 0.05, **p < 0.01, or ***p < 0.001.

### [Description of Embodiments]

Hereinafter, the present invention will be described in detail.

In this description, the terms described below will be used.

A "halogen atom" is a fluorine atom, a chlorine atom, a bromine atom, or an iodine atom.

A "C1-C6 alkyl group" is a linear or branched alkyl group having 1 to 6 carbon atoms. Specific examples thereof include a methyl group, an ethyl group, a propyl group, an isopropyl group, a butyl group, an isobutyl group, a sec-butyl group, a tert-butyl group, a pentyl group, an isopentyl group, a 2-methylbutyl group, a neopentyl group, a 1-ethylpropyl group, a hexyl group, an isohexyl group, a 3-methylpentyl group, a 2-methylpentyl group, a 1-methylpentyl group, a 3,3-dimethylbutyl group, a 2,2-dimethylbutyl group, a 1,1-dimethylbutyl group, or a 1,2-dimethylbutyl group.

An "aryl group" is a monocyclic or bicyclic aromatic carbocycle having 6 to 10 carbon atoms, which may be condensed with a non-aromatic heterocycle or cycloalkane. Specific examples thereof include a phenyl group, a naphthyl group, a tetralinyl group, an indanyl group, a chromanyl group, a 2,3-dihydrobenzofuranyl group, a 1,3-benzodioxolyl group, a 2,3-dihydro-1,4-benzodioxinyl group, a 1,2,3,4-tetrahydroquinolinyl group, a 1,2,3,4-tetrahydroisoquinolinyl group, an indolinyl group, or a 3,4-dihydro-2H-1,4-benzoxazinyl group. A phenyl group, a naphthyl group, or a 1,3-benzodioxolyl group is preferred.

A "C5-C10 cycloalkenyl group" is a hydrocarbon ring having one double bond within the ring having 5 to 10 carbon atoms, which may be crosslinked with an alkylene group. Specific examples thereof include a cyclopentenyl group, a cyclohexenyl group, a cycloheptenyl group, a cyclooctenyl group, a bicyclo[2.2.1]heptenyl group, or a bicyclo[2.2.2]octenyl group.

A "heterocyclyl group" is a 4- to 10-membered ring group in which the atoms constituting the ring are one to four heteroatoms independently selected from nitrogen, oxygen, and sulfur, other than carbon, which may be aromatic or non-aromatic, or may be crosslinked with an alkylene group in the case of being non-aromatic. Specific examples of a non-aromatic heterocyclyl group include an azetidinyl group, a pyrrolidinyl group, a piperidinyl group, an azepanyl group, a diazepanyl group, an azocanyl group, a piperazinyl group, a homopiperazinyl group, a morpholinyl group, an oxazepanyl group, a thiomorpholinyl group, a thiazepanyl group, a tetrahydropyranyl group, a tetrahydrofuryl group, a dioxanyl group, a dioxolanyl group, a 2-azabicyclo[2.2.1]heptyl group, a 2,5-diazabicyclo[2.2.1]heptyl group, a 3-azabicyclo[3.2.1]octyl group, an 8-azabicyclo[3.2.1]octyl group, a 9-azabicyclo[3.3.1]nonyl group, a 3,9-diazabicyclo[3.3.1]nonyl group, a dihydropyranyl group, a dihydropyrrolyl group, a dihydropyridyl group, a tetrahydropyridyl group, a tetrahydropyrazyl group, a 3,9-diazaspiro[5.5]undec-3-yl group, a 1,9-diazaspiro[5.5]undec-9-yl group, a 1,8-diazaspiro[4.5]dec-8-yl group, or a 1,4-dioxa-8-aza spiro[4.5]dec-8-yl group. Examples of an aromatic heterocyclyl group include a furyl group, a pyrrolyl group, a thienyl group, an oxazolyl group, a thiazolyl group, an imidazolyl group, a pyrazolyl group, an oxadiazolyl group, a thiadiazolyl group, a triazolyl group, a tetrazolyl group, a pyridyl group, a pyrazyl group, a pyrimidyl group, a pyridazinyl group, a triazinyl group, an indolyl group, an isoindolyl group, an indazolyl group, a purinyl group, a quinolyl group, an isoquinolyl group, or a naphthyridinyl group.

A "C1-C6 alkoxy group" is a group in which the C1-C6 alkyl group is bonded to an oxygen atom. Specific examples thereof include a methoxy group, an ethoxy group, a n-propoxy group, an isopropoxy group, a n-butoxy group, a sec-butoxy group, an isobutoxy group, a tert-butoxy group, a pentyloxy group, an isopentyloxy group, a neopentyloxy group, a hexyloxy group, or an isohexyloxy group.

A "halo-C1-C6 alkyl group" is a group in which the C1-C6 alkyl group is substituted with 1 to 7 halogen atoms. Specific examples thereof include a trifluoromethyl group, a difluoromethyl group, a 1,1-difluoroethyl group, a 2,2-difluoroethyl group, or a 2,2,2-trifluoroethyl group.

A "halo-C1-C6 alkoxy group" is a group in which the C1-C6 alkoxy group is substituted with 1 to 7 halogen atoms. Specific examples thereof include a fluoromethoxy group, a difluoromethoxy group, a dichloromethoxy group, a dibromomethoxy group, a trifluoromethoxy group, a trichloromethoxy group, a 2-fluoroethoxy group, a 2-bromoethoxy group, a 2-chloroethoxy group, a 2-iodoethoxy group, a 2,2-difluoroethoxy group, a 2,2,2-trifluoroethoxy group, a 2,2,2-trichloroethoxy group, a pentafluoroethoxy group, a 3-fluoropropoxy group, a 3-chloropropoxy group, a 4-fluorobutoxy group, a 5-fluoropentyloxy group, or a 6-fluorohexyloxy group.

A "hydroxy C1-C6 alkyl group" is a group in which the C1-C6 alkyl group is substituted with one hydroxyl group. Specific examples thereof include a hydroxymethyl group, a hydroxyethyl group, a hydroxypropyl group, a hydroxybutyl group, a hydroxypentyl group, or a hydroxyhexyl group.

A "C1-C6 alkoxy C1-C6 alkoxy group" is a group in which the C1-C6 alkoxy group is substituted with the C1-C6 alkoxy group. Specific examples thereof include a methoxymethoxy group, a methoxyethoxy group, a methoxypropoxy group, an ethoxymethoxy group, an ethoxyethoxy group, an ethoxypropoxy group, or a propoxypropoxy group.

A "(C1-C6 alkyl)carbonyl group" is a group in which the C1-C6 alkyl group is bonded to a carbonyl group. Specific examples thereof include an acetyl group, a propionyl group, a butyryl group, an isobutyryl group, or a pivaloyl group.

A "(C1-C6 alkoxy)carbonyl group" is a group in which the C1-C6 alkoxy group is bonded to a carbonyl group. Specific examples thereof include a methoxycarbonyl group, an ethoxycarbonyl group, a n-propoxycarbonyl group, an isopropoxycarbonyl group, a n-butoxycarbonyl group, an isobutoxycarbonyl group, a sec-butoxycarbonyl group, a tert-butoxycarbonyl group, a n-pentyloxycarbonyl group, an isopentyloxycarbonyl group, a neopentyloxycarbonyl group, a n-hexyloxycarbonyl group, or an isohexyloxycarbonyl group.

A "(C1-C6 alkoxy)carbonyloxy group" is a group in which the C1-C6 alkoxy group is bonded to a carbonyloxy group. Specific examples thereof include a methoxycarbonyloxy group, an ethoxycarbonyloxy group, a n-propoxycarbonyloxy group, an isopropoxycarbonyloxy group, a n-butoxycarbonyloxy group, an isobutoxycarbonyloxy group, a sec-butoxycarbonyloxy group, a tert-butoxycarbonyloxy group, a n-pentyloxycarbonyloxy group, an isopentyloxycarbonyloxy group, a neopentyloxycarbonyloxy group, a n-hexyloxycarbonyloxy group, or an isohexyloxycarbonyloxy group.

A "phenyl C1-C6 alkoxy group" is a group in which the C1-C6 alkoxy group is substituted with a phenyl group at any position. Specific examples thereof include a benzyloxy group, a 1-phenylethyloxy group, a 2-phenylethyloxy group, a 1-phenylpropyloxy group, a 2-phenylpropyloxy group, or a 3-phenylpropyloxy group.

A "carbamoyl group optionally substituted with one or two C1-C6 alkyl groups" is a carbamoyl group or a group in which the one or two C1-C6 alkyl groups are bonded to a carbamoyl group. Specific examples thereof include a carbamoyl group, a methylcarbamoyl group, a dimethylcarbamoyl group, an ethylcarbamoyl group, a diethylcarbamoyl group, an ethylmethylcarbamoyl group, a propylcarbamoyl group, or a dipropylcarbamoyl group.

A "C1-C6 alkoxy group substituted by a carbamoyl group optionally substituted with one or two C1-C6 alkyl groups" is a group in which the C1-C6 alkoxy group is substituted by the carbamoyl group optionally substituted with the one or two C1-C6 alkyl groups. Specific examples thereof include a carbamoylmethyloxy group, a carbamoylethyloxy group, a methylcarbamoylmethyloxy group, a methylcarbamoylethyloxy group, a dimethylcarbamoylmethyloxy group, a dimethylcarbamoylethyloxy group, an ethylcarbamoylmethyloxy group, an ethylcarbamoylethyloxy group, a diethylcarbamoylmethyloxy group, a diethylcarbamoylethyloxy group, an ethylmethylcarbamoylmethyloxy group, an ethylmethylcarbamoylethyloxy group, a propylcarbamoylmethyloxy group, a propylcarbamoylethyloxy group, a dipropylcarbamoylmethyloxy group, or a dipropylcarbamoylethyloxy group.

A "sulfamoyl group substituted with one or two C1-C6 alkyl groups" is a group in which the one or two C1-C6 alkyl groups are bonded to a sulfamoyl group. Specific examples thereof include a methylsulfamoyl group, a dimethylsulfamoyl group, an ethylsulfamoyl group, an ethylmethylsulfamoyl group, a diethylsulfamoyl group, a propylsulfamoyl group, or a dipropylsulfamoyl group.

A "C1-C4 alkyl group" is a linear or branched alkyl group having one to four carbon atoms. Specific examples thereof include a methyl group, an ethyl group, a propyl group, an isopropyl group, a butyl group, an isobutyl group, a sec-butyl group, or a tert-butyl group.

A "C1-C4 alkoxy group" is a group in which the C1-C4 alkyl group is bonded to an oxygen atom. Specific examples thereof include a methoxy group, an ethoxy group, a n-propoxy group, an isopropoxy group, a n-butoxy group, a sec-butoxy group, an isobutoxy group, or a tert-butoxy group.

A "C1-C2 alkyl group" is a linear alkyl group having 1 or 2 carbon atoms, such as a methyl group and an ethyl group.

A "halo-C1-C2 alkyl group" is a group in which the C1-C2 alkyl group is substituted with 1 to 5 halogen atoms. Specific examples thereof include a trifluoromethyl group, a difluoromethyl group, a 1,1-difluoroethyl group, a 2,2-difluoroethyl group, or a 2,2,2-trifluoroethyl group.

A "C1-C2 alkoxy group" is a group in which the C1-C2 alkyl group is bonded to an oxygen atom, such as a methoxy group and an ethoxy group.

A "halo-C1-C2 alkoxy group" is a group in which the C1-C2 alkoxy group is substituted with 1 to 5 halogen atoms. Specific examples thereof include a fluoromethoxy group, a difluoromethoxy group, a dichloromethoxy group, a dibromomethoxy group, a trifluoromethoxy group, a trichloromethoxy group, a 2-fluoroethoxy group, a 2-bromoethoxy group, a 2-chloroethoxy group, a 2-iodoethoxy group, a 2,2-difluoroethoxy group, a 2,2,2-trifluoroethoxy group, a 2,2,2-trichloroethoxy group, or a pentafluoroethoxy group.

A "hydroxy C1-C4 alkyl group" is a group in which the C1-C4 alkyl group is substituted by one hydroxyl group. Specific examples thereof include a hydroxymethyl group, a hydroxyethyl group, a hydroxypropyl group, or a hydroxybutyl group.

A "C1-C2 alkoxy C1-C2 alkoxy group" is a group in which the C1-C2 alkoxy group is substituted by the C1-C2 alkoxy group. Specific examples thereof include a methoxymethoxy group, a methoxyethoxy group, an ethoxymethoxy group, or an ethoxyethoxy group.

A "(C1-C4 alkyl)carbonyl group" is a group in which the C1-C4 alkyl group is bonded to a carbonyl group. Specific examples thereof include an acetyl group, a propionyl group, a butyryl group, an isobutyryl group, or a pivaloyl group.

A "(C1-C4 alkoxy)carbonyl group" is a group in which the C1-C4 alkoxy group is bonded to a carbonyl group. Specific examples thereof include a methoxycarbonyl group, an ethoxycarbonyl group, a n-propoxycarbonyl group, an isopropoxycarbonyl group, a n-butoxycarbonyl group, an isobutoxycarbonyl group, a sec-butoxycarbonyl group, or a tert-butoxycarbonyl group.

A "(C1-C4 alkoxy)carbonyloxy group" is a group in which the C1-C4 alkoxy group is bonded to a carbonyloxy group. Specific examples thereof include a methoxycarbonyloxy group, an ethoxycarbonyloxy group, a n-propoxycarbonyloxy group, an isopropoxycarbonyloxy group, a n-butoxycarbonyloxy group, an isobutoxycarbonyloxy group, a sec-butoxycarbonyloxy group, or a tert-butoxycarbonyloxy group.

A "phenyl C1-C4 alkoxy group" is a group in which the C1-C4 alkoxy group is substituted with a phenyl group at any position. Specific examples thereof include a benzyloxy group, a 1-phenylethyloxy group, a 2-phenylethyloxy group, a 1-phenylpropyloxy group, a 2-phenylpropyloxy group, or a 3-phenylpropyloxy group.

A "carbamoyl group optionally substituted with one or two C1-C4 alkyl groups" is a carbamoyl group or a group in which the one or two C1-C4 alkyl groups are bonded to a carbamoyl group. Specific examples thereof include a carbamoyl group, a methylcarbamoyl group, a dimethylcarbamoyl group, an ethylcarbamoyl group, a diethylcarbamoyl group, an ethylmethylcarbamoyl group, a propylcarbamoyl group, or a dipropylcarbamoyl group.

A "C1-C2 alkoxy group substituted by a carbamoyl group optionally substituted with one or two C1-C4 alkyl groups" is a group in which the C1-C2 alkoxy group is substituted by a carbamoyl group optionally substituted with the one or two C1-C4 alkyl groups. Specific examples thereof include a carbamoylmethyloxy group, a carbamoylethyloxy group, a methylcarbamoylmethyloxy group, a methylcarbamoylethyloxy group, a dimethylcarbamoylmethyloxy group, a dimethylcarbamoylethyloxy group, an ethylcarbamoylmethyloxy group, an ethylcarbamoylethyloxy group, a diethylcarbamoylmethyloxy group, a diethylcarbamoylethyloxy group, an ethylmethylcarbamoylmethyloxy group, an ethylmethylcarbamoylethyloxy group, a propylcarbamoylmethyloxy group, a propylcarbamoylethyloxy group, a dipropylcarbamoylmethyloxy group, or a dipropylcarbamoylethyloxy group.

A "sulfamoyl group substituted with one or two C1-C4 alkyl groups" is a group in which the one or two C1-C4 alkyl groups are bonded to a sulfamoyl group. Specific examples thereof include a methylsulfamoyl group, a dimethylsulfamoyl group, an ethylsulfamoyl group, an ethylmethylsulfamoyl group, a diethylsulfamoyl group, a propylsulfamoyl group, or a dipropylsulfamoyl group.

"Stem cells" are cells having self-replication ability and pluripotency, and examples thereof include ES cells, iPS cells, and adult stem cells.

"Pluripotent stem cells" are cells capable of differentiating into various cells of living organisms and are preferably ES cells or iPS cells.

"Insulin-producing cells" are cells that secrete insulin upon reaction with hyperglycemia or the like and have a superior ability to express insulin as compared with other pancreatic hormones such as glucagon or somatostatin.

"Three-dimensional culture" is culture on the condition that cells do not adhere to a culture container, and means culture in the state of a sphere (also called "embryoid body" for pluripotent stem cells) formed by adhesion among cells. In this description, it is also referred to as "sphere culture".

### <Method for producing insulin cells>

The present invention provides a method for producing insulin-producing cells by differentiating pluripotent stem cells into insulin-producing cells, comprising the step of three-dimensionally culturing cells in a medium containing a compound represented by formula (I) or a salt thereof. The effect of a compound represented by formula (I) or a salt thereof to promote the differentiation into insulin-producing cells can be confirmed by the method of Reference Example 1 or Example 1, which will be described below.

Various stem cells can be employed as the pluripotent stem cells targeted, as long as they are stem cells capable of differentiating into endoderm cells, but the pluripotent stem cells are preferably ES cells or iPS cells, more preferably iPS cells. Various pluripotent stem cells derived from mammals can be employed but are preferably derived from humans, mice, rats, pet animals such as dogs and cats, livestock animals such as bovines, horses, pigs, and sheep, more preferably humans.

The differentiation process from pluripotent stem cells into insulin-producing cells is, for example, divided into five stages as described in Non Patent Document 4. That is, the five stages are: stage 1 in which Sox17-positive definitive endoderm cells are induced from pluripotent stem cells, stage 2 in which Foxa2-positive primitive gut tube cells are induced from the definitive endoderm cells, stage 3 in which PDX1-positive pancreatic progenitor cells are induced from the primitive gut tube cells, stage 4 in which Ngn3-positive pancreatic endocrine progenitor cells are induced from the pancreatic progenitor cells, and stage 5 in which insulin-producing cells are finally induced from the pancreatic endocrine progenitor cells. In some cases, stage 1 may be further subdivided into stage 1-1 in which the state of mesendoderm cells is generated and stage 1-2 in which definitive endoderm cells are induced. Stage 2 may be further subdivided into stage 2-1 and stage 2-2 depending on difference in zinc concentration in a differentiation medium. Stage 5 may be further subdivided into stage 5-1 and stage 5-2 depending on difference in retinoic acid concentration in a differentiation medium. In this description, the cell differentiation may be expressed with these stages.

The medium to be used for such culture is not specifically limited, as long as it is a medium generally used for cell culture, and various media can be used, such as DMEM medium, α-MEM medium, RPMI medium, StemFit medium (Ajinomoto Co., Inc., AK03, AK03N, etc.), Essential 8 (Thermo Fisher, A1517001), TeSR1 (Stem Cell Technologies, 85850), NutriStem (stemgent, 01-0005), and CMRL (Thermo Fisher, 11530037). The medium may be supplemented with serum, and a serum replacement such as KO-Serum or B-27 supplement can be added to a serum-free medium for use. Also, the medium may be supplemented with various culture additives generally used for cell culture, such as sugar (glucose, etc.), an antioxidant (β mercaptoethanol, vitamin C, etc.), various amino acids (essential amino acids, non-essential amino acids (NEAA), etc.), various metal ion sources (Ca²⁺, Mg²⁺, Zn²⁺, etc.), and various growth factors (EGF, FGF, KGF, IGF, etc.).

In such culture for differentiation induction or pretreatment thereof, a medium in which the concentration of a specific component such as an amino acid or a metal ion is reduced or depleted from general medium composition can also be used. For example, a methionine-depleted or -reduced medium is used in culture treatment of pluripotent stem cells for a short time (for example, 1 hour, 5 hours, 10 hours, or 24 hours) and induces differentiation of cells thus treated. In other aspects, a medium in which a zinc ion concentration is reduced (for example, 1 µM or lower, preferably 0.5 µM or lower) or depleted may be used only in the first half (for example, at least a portion of stage 1 or stage 2, preferably a period including stage 1, more preferably only stage 1, stage 1 to the first half of stage 2, or the whole period from stage 1 to stage 2) of a differentiation culture step. Such a medium having an adjusted concentration of a specific component can be produced without adding a desired component, or by reducing the amount to be added, on the basis of information on known medium composition. Alternatively, a medium containing no desired component is obtained, and a specific component may be added thereto so as to have a desired concentration. The methionine-reduced or - depleted medium can be produced on the basis of, for example, WO2015/125662. In Examples, AKM medium used as a zinc-depleted medium was obtained from Research institute For Bioscience Products & Fine Chemicals, Ajinomoto Co., Inc.

In the differentiation process from pluripotent stem cells into insulin-producing cells, the differentiation stage proceeds by culture in a medium containing an additive for signal induction suitable for each stage. Hereinafter, the additive to be used in a medium in each stage will be listed. For such an additive, an appropriate type may be selected and used, or all additives for a stage concerned may be used. Also, an additive for a preceding or subsequent stage may be used.

In stage 1, at least one selected from an activin receptor and Wnt signal activation is necessary. As the additive for a medium to be used, an activin receptor agonist (for example, activin A), a WNT signal activator (for example, Wnt protein such as Wnt3A, or a GSK3β inhibitor (for example, CHIR990221)), or the like is generally used. A culture period of stage 1 is 1 to 4 days, preferably 2 to 3 days. The GSK3β inhibitor may be added through the period of stage 1 or may be added only in stage 1-1, and a medium for stage 1 in which the GSK3β inhibitor is reduced or not contained may be used in subsequent stage 1-2. An example of the medium for stage 1 is a medium containing 10 to 1000 ng/mL (preferably 100 ng/mL) activin A and/or 1 to 10 µM (preferably 3 µM) CHIR99021.

In stage 2, FGF receptor signal activation is necessary. As the additive for a medium to be used, a FGF receptor agonist (for example, FGF10, KGF) or the like is generally used. As an additive, a sonic hedgehog inhibitor (for example, KAAD-cyclopamine or SANT-l) can also be used. A culture period of stage 2 is 1 to 7 days, preferably 2 to 6 days. The sonic hedgehog inhibitor may be added through the period of stage 2 or may be added only in the first half of stage 2. An example of the medium for stage 2 is a medium containing 5 to 500 ng/mL (preferably 50 ng/mL) FGF10 or KGF and/or 0.05 to 5 µM (preferably 0.25 µM) SANT1.

In stage 3, at least one selected from retinoic acid receptor signal activation, BMP receptor signal inhibition, sonic hedgehog signal inhibition, and FGF receptor signal activation is necessary. As the additive for a medium to be used, a retinoic acid receptor agonist (for example, retinoic acid), a sonic hedgehog inhibitor (for example, KAAD-cyclopamine or SANT-l), a BMP signal inhibitor (for example, Noggin or LDN193189), a protein kinase C activator (for example, indolactam V), or the like is generally used. As an additive, a TGFβ receptor kinase inhibitor (for example, SB431542) can also be used. A culture period of stage 3 is 1 to 8 days, preferably 2 to 6 days. Examples of the medium for stage 3 include a medium containing at least one of 0.05 to 5 µM (preferably 0.15 µM) SANT1, 0.1 to 10 µM (preferably 2 µM) retinoic acid, and 0.01 to 1 µM (preferably 0.1 µM) LDN193189, and a medium containing at least one of 0.05 to 5 µM (preferably 0.25 µM) SANT1, 0.1 to 10 µM (preferably 2 µM) retinoic acid, 5 to 500 ng/mL (preferably 50 ng/mL) KGF or FGF10, and 5 to 500 nM (preferably 50 nM) indolactam V.

In stage 4, at least one selected from BMP receptor signal inhibition, sonic hedgehog signal inhibition, FGF receptor signal activation, and retinoic acid receptor signal activation is necessary. As the additive for a medium to be used, a BMP signal inhibitor (for example, Noggin or LDN193189), a sonic hedgehog inhibitor (for example, SANT-l), or the like is generally used. As an additive, a protein kinase C activator (for example, indolactam V) or a TGFβ receptor kinase inhibitor (for example, ALK5 inhibitor II) can also be used. A culture period of stage 4 is 1 to 7 days, preferably 2 to 5 days. Examples of the medium for stage 4 include a medium containing at least one of 0.5 to 50 µM (preferably 5 µM) ALK5 inhibitor (Calbiochem, 616452), 0.01 to 10 µM (preferably 0.3 µM) indolactam V, and 0.01 to 1 µM (preferably 0.1 µM) LDN193189, and a medium containing at least one of 0.05 to 5 µM (preferably 0.25 µM) SANT1, 0.01 to 1 µM (preferably 0.1 µM) retinoic acid, 5 to 500 ng/mL (preferably 50 ng/mL) KGF or FGF10, and 10 to 1000 nMM (preferably 100 nM LDN193189.

In stage 5, at least one selected from TGFβ receptor signal inhibition, NOTCH signal inhibition, thyroid hormone receptor signal activation, and retinoic acid receptor signal activation is necessary. As the additive for a medium to be used, a TGFβ receptor kinase inhibitor (for example, ALK5 inhibitor II), a γ-secretase inhibitor (for example, DAPT), thyroid hormone T3, an EGFR agonist (for example, EGF), a retinoic acid receptor agonist (for example, retinoic acid), vitamin C, or the like is generally used. As an additive, a, GLP-1 receptor agonist (for example, GLP-1 peptide or exendin-4), nicotinamide, an adenylate cyclase activator (for example, Forskolin), or a glucocorticoid receptor agonist (for example, Dexamethasone) can also be used. A culture period of stage 5 is 5 to 20 days, preferably 7 to 15 days. Examples of the medium for stage 5 include a medium containing 5 to 500 ng/mL(preferably 50 ng/mL) exendin 4 and/or 1 to 100 nM (preferably 10 mM) nicotinamide, a medium containing at least one of 0.5 to 100 µM (preferably 10 µM) ALK5 inhibitor (Calbiochem, 616452), 0.01 to 1 µM (preferably 0.1 µM) retinoic acid, 1 to 100 µM (preferably 10 µM) DAPT, 1 to 500 ng/mL (preferably 33.3 ng/mL) EGF, and 0.1 to 10 µM (preferably 1 µM) T3, and a medium in which the concentration of the aforementioned retinoic acid is reduced (for example, 0.05 µM or lower, preferably 0.025 µM). The medium in which the retinoic acid concentration is reduced is preferably used in the second half of stage 5.

A method for producing insulin-producing cells of the present invention comprises the step of three-dimensionally culturing cells in a medium containing a compound represented by formula (I) or a salt thereof. As a three-dimensional culture method, an approach generally used by a person skilled in the art can be appropriately employed, but examples thereof can include a culture method using a low-adhesive or non-adhesive culture container, a stirring culture method, a hanging drop culture method, and a culture method using a hydrogel or a porous scaffold. As the low-adhesive or non-adhesive culture container, a general culture container may be coated at its bottom or side with a polymer reagent or the like that inhibits cell adhesion, for use, or a culture container in which a low-cell adhesive material is employed in a base material may be used. Various shapes of the container can be used, and use of a round-bottom container can promote sphere formation of cells. In the case of employing a flat-bottom container, sphere formation can be promoted by culture on a shaker. Sphere formation can also be promoted by stirring culture using a spinner flask or a culture reactor, which may be adapted to large-scale culture.

In a method for producing insulin-producing cells of the present invention, the differentiation of pluripotent stem cells into insulin-producing cells is generally performed by three-dimensional culture throughout all steps, but three-dimensional culture may be performed only in one or some steps (for example, one or more steps selected from the step of inducing definitive endoderm cells from pluripotent stem cells, the step of inducing primitive gut tube cells from the definitive endoderm cells, the step of inducing pancreatic progenitor cells from the primitive gut tube cells, the step of inducing pancreatic endocrine progenitor cells from the pancreatic progenitor cells, and the step of inducing insulin-producing cells from the pancreatic endocrine progenitor cells). In the case of employing three-dimensional culture in one or some steps, the second half (for example, primitive gut tube cells or later) of the differentiation process is desirable. A compound represented by formula (I) or a salt thereof may be added in at least one or some steps in which three-dimensional culture is employed, and does not have to be added in all steps, and its addition in a step other than three-dimensional culture (generally adhesion culture) is not hindered.

A compound represented by formula (I) or a salt thereof can be added, for example, in a three-dimensional culture step of the differentiation stage after the primitive gut tube cells, in a differentiation process into insulin-producing cells, thereby remarkably promoting the differentiation into insulin-producing cells. The compound represented by formula (I) may be added in any step of the differentiation process from pluripotent stem cells into insulin-producing cells, but is preferably added during culture of the primitive gut tube cells, pancreatic progenitor cells, and/or pancreatic endocrine progenitor cells derived from pluripotent stem cells. In the aforementioned differentiation stages, the compound may be added at any one stage or two stages out of stages 3 to 5, or at all the three stages, but is preferably added at all the stages from stage 3 to stage 5.

A compound represented by formula (I) or a salt thereof may be added instead of the aforementioned additive for each stage or may be added in addition thereto. A compound represented by formula (I) or a salt thereof can be added to the medium in solid form as it is, in powder form, or after being dissolved in an organic solvent such as dimethylsulfoxide. The amount to be added is not specifically limited but is set by a person skilled in the art, so that the differentiation from pluripotent stem cells into insulin-producing cells proceeds efficiently. In several embodiments of the present invention, a compound represented by formula (I) is added so as to be present in the medium in an amount of 1 ng/mL to 5 mg/mL, preferably 10 ng/mL to 5 mg/mL, more preferably 50 ng/mL to 5 mg/mL, even more preferably 100 ng/mL to 1 mg/mL.

Examples of a method for producing insulin-producing cells of the present invention include methods shown below and can specifically include protocol #1 and protocol #2 employed in Example 1. In the following protocols, the medium to be used in each stage will be listed below, but is not limited thereto.

### [Treatment of cells and culture method]

Pretreatment: Pluripotent stem cells are transferred at a concentration of 1 × 10⁶ cells/mL in a medium to maintain an undifferentiated state (for example, StemFit AK03N medium (Ajinomoto Co., Inc.)) to a low-attachment 6-well plate and cultured for 24 hours or longer on a rotary shaker (95 rpm). Three-dimensional culture under these conditions is also applied to subsequent culture steps. On a differentiation induction start day, the medium is replaced with an AK03N-based methionine-depleted medium (KA01, Ajinomoto Co., Inc.), and the cells are cultured for 5 hours.

Stage 1: Pluripotent stem cells (which may be pretreated) are cultured for 1 to 4 days in a medium for stage 1 which may contain a compound represented by formula (I) or a salt thereof to induce definitive endoderm cells. Preferably, the cells are cultured for 24 hours in differentiation medium 1-1 or M1-1 AKM medium, then the medium is replaced with differentiation medium 1-2 or M1-2 AKM medium, and the cells are cultured for 1 to 2 days.

Stage 2: The cultures containing the definitive endoderm cells are cultured for 2 to 6 days in a medium for stage 2 which may contain a compound represented by formula (I) or a salt thereof to induce primitive gut tube cells. Preferably, the cells are cultured for 2 to 3 days in differentiation medium 2, M2 AKM medium or S2 medium. As another example, the cells are cultured for 2 days in a medium for stage 2 in which a zinc concentration is reduced (for example, M2 AKM medium) and then cultured for 2 to 3 days in a medium for stage 2 with a general zinc concentration (for example, S2 medium or differentiation medium 2).

Stage 3: The cultures containing the primitive gut tube cells are cultured for 2 to 8 days in a medium for stage 3 which may contain a compound represented by formula (I) or a salt thereof to induce pancreatic progenitor cells. Preferably, the cells are cultured for 5 to 7 days in differentiation medium 3 or for 2 to 4 days in S3 medium.

Stage 4: The cultures containing the pancreatic progenitor cells are cultured for 1 to 7 days in a medium for stage 4 which may contain a compound represented by formula (I) or a salt thereof to induce pancreatic endocrine progenitor cells. Preferably, the cells are cultured for 2 to 3 days in differentiation medium 4 or for 4 to 6 days in S4 medium.

Stage 5: The cultures containing the pancreatic endocrine progenitor cells are cultured for 5 to 20 days in a medium for stage 5 which may contain a compound represented by formula (I) or a salt thereof to induce insulin-producing cells. Preferably, the cells are cultured for 10 to 15 days in differentiation medium 5. In another aspect, the cells are cultured for 3 to 5 days in a medium for stage 5 containing 0.1 µM or higher retinoic acid (for example, S5-1 medium) and then cultured for 2 to 4 days in a medium for stage 5 containing retinoic acid at a low concentration of 0.05 µM or lower (for example, S5-2 medium).

### [Medium]

AKM medium is insulin- and Zn²⁺-depleted StemFit Basic 03 medium (Ajinomoto Co., Inc.). (Example of medium for stage 1)

Differentiation medium 1-1; DMEM (high glucose), L-Gln, NEAA, 0.01 mM β-mercaptoethanol, 100 ng/mL activin A, B27 supplement, 3 µM CHIR99021

Differentiation medium 1-2: DMEM (high glucose), L-Gln, NEAA, 0.01 mM β-mercaptoethanol, 100 ng/mL activin A, B27 supplement

M1-1 AKM medium: AKM medium supplemented with 100 ng/mL activin A and 3 µM CHIR990221 and further supplemented with 100 ng/mL IGF1 and 0.5 µM Zn

M1-2 AKM medium: AKM medium supplemented with 100 ng/mL activin A and further supplemented with 100 ng/mL IGF1 and 0.5 µM Zn

### (Example of medium for stage 2)

Differentiation medium 2: RPMI, L-Gln, NEAA, 0.01 mM β-mercaptoethanol, insulin-depleted B27 supplement, 50 ng/mL FGF10, 0.25 µM SANT1

M2 AKM medium: AKM medium supplemented with 50 ng/mL FGF10 and 250 nM SANT1 and further supplemented with 0.5 µM Zn

S2 medium: StemFit Basic 03 supplemented with 50 ng/mL KGF and 44 µg/mL vitamin C

### (Example of medium for stage 3)

Differentiation medium 3: DMEM (high glucose), L-Gln, NEAA, 0.01 mM β-mercaptoethanol, 0.15 µM SANT1, 2 µM retinoic acid, 0.1 µM LDN193189, B27 supplement

S3 medium: StemFit Basic 03 supplemented with 50 ng/mL KGF, 50 nM indolactam V, 2 µM retinoic acid, 250 nM SANT1, and 44 µg/mL vitamin C

### (Example of medium for stage 4)

Differentiation medium 4: DMEM (high glucose), L-Gln, NEAA, 0.01 mM β-mercaptoethanol, 5 µM ALK5 inhibitor (Calbiochem, 616452), 0.3 µM indolactam V, 0.1 µM LDN193189, B27 supplement

S4 medium: StemFit Basic 03 supplemented with 50 ng/mL KGF, 100 nM retinoic acid, 250 nM SANT1, 44 µg/mL vitamin C, and 100 nM LDN193189

### (Example of medium for stage 5)

Differentiation medium 5: KO DMEM/F12, L-Gln, NEAA, 0.01 mM β-mercaptoethanol, 50 ng/mL exendin 4, 10 mM nicotinamide, 10 µM ZnSO₄, 1 mM N-acetyl-L-cysteine, B27 supplement

S5-1 medium: StemFit Basic 03 supplemented with 10 µM ALK5 inhibitor, 10 µM DAPT, 33.3 ng/mL EGF, 100 nM retinoic acid, 1 µM T3, and 44 µg/mL vitamin C

S5-2 medium: StemFit Basic 03 supplemented with 10 µM ALK5 inhibitor, 10 µM DAPT, 33.3 ng/mL EGF, 25 nM retinoic acid, and 1 µM T3

In the case of evaluating the effectiveness of a compound for a differentiation rate and a cell function, a test compound and a negative control (0.01% DMSO) were treated between stages 3 and 4 (days 5 to 13), and immunocytochemical staining was carried out on days 14 and 21 in order to confirm an effect on positivity to insulin, NKX6.1, and PDX1. GSIS was carried out on day 21.

### <Compound>

Preferred aspects of the compound represented by formula (I) in the present invention will be described below.

Examples of the substituent R¹ in the present invention can include a hydrogen atom, a halogen atom, and a C1-C6 alkyl group. R¹ is preferably a hydrogen atom, a chlorine atom, a bromine atom, or a methyl group, more preferably a hydrogen atom, a chlorine atom, or a methyl group. The R¹ substitution can be at any position shown below in (I-i) to (I-iii).

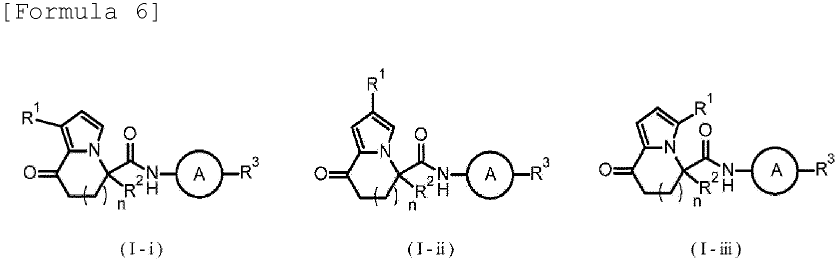

Examples of the substituent R² in the present invention can include a hydrogen atom or a C1-C6 alkyl group. R² is preferably a hydrogen atom or a C1-C2 alkyl group, more preferably a hydrogen atom or a methyl group.

Examples of the substituent R³ in the present invention include an aryl group optionally substituted with one to four substituents independently selected from a substituent group α, a C5-C10 cycloalkenyl group optionally substituted with one to four substituents independently selected from the substituent group α, or a heterocyclyl group optionally substituted with one to four substituents independently selected from the substituent group α. R³ is preferably a naphthyl group, a 1,3-benzodioxolyl group, a 2,2-dihalo-1,3-benzodioxolyl group, a C5-C10 cycloalkenyl group, a phenyl group optionally substituted with one or two substituents independently selected from a substituent group α1, or a 5- or 6-membered heterocyclyl group optionally substituted with one or two substituents independently selected from the substituent group α1, more preferably a naphthyl group, a 1,3-benzodioxolyl group, a 2,2-dihalo-1,3-benzodioxolyl group, a C5-C10 cycloalkenyl group, a phenyl group optionally substituted with one or two substituents independently selected from a substituent group α2, or a 5- or 6-membered heterocyclyl group optionally substituted with one or two substituents independently selected from a substituent group γ2. R³ is even more preferably a naphthyl group, a 1,3-benzodioxolyl group, a 2,2-difluoro-1,3-benzodioxolyl group, a C5-C8 cycloalken-1-yl group, a phenyl group optionally substituted with one or two substituents independently selected from a substituent group α3, or a 5- or 6-membered heterocyclyl group optionally substituted with one or two substituents independently selected from a substituent group γ3, particularly preferably a 2,2-difluoro-1,3-benzodioxolyl group, a 1-tert-butoxycarbonyl-3,6-dihydro-2H-pyridin-4-yl group, or a phenyl group optionally substituted with one or two substituents independently selected from the group consisting of a fluorine atom, a chlorine atom, a trifluoromethyl group, a tert-butoxy group, a trifluoromethoxy group, a 2,2,2-trifluoroethoxy group, a benzyloxy group, and a phenoxy group. Further, a phenyl group, or a phenyl group substituted at m position or p position with any one substituent selected from the group consisting of a fluorine atom, a chlorine atom, a trifluoromethyl group, a tert-butoxy group, a trifluoromethoxy group, a 2,2,2-trifluoroethoxy group, a benzyloxy group, and a phenoxy group is particularly preferred. The substituent groups α to γ are as described below.

Examples of the substituent group α in the present invention include a halogen atom, a cyano group, a carboxy group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, a phenoxy group optionally substituted with one to four substituents independently selected from a substituent group β, a phenyl group optionally substituted with one to four substituents independently selected from the substituent group β, and a benzoyl group optionally substituted with one to four substituents independently selected from the substituent group β. Preferably, the substituent group α1 is a halogen atom, a cyano group, a carboxy group, a phenoxy group, a benzoyl group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a 5- or 6-membered non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, or a phenyl group optionally substituted with one or two substituents independently selected from a substituent group β1. More preferably, the substituent group α2 is a halogen atom, a cyano group, a carboxy group, a phenoxy group, a benzoyl group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1 to C6 alkoxy group, a 5- or 6-membered non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, or a phenyl group optionally substituted with one or two substituents independently selected from a substituent group β2. Even more preferably, the substituent group α3 is a halogen atom, a cyano group, a carboxy group, a phenoxy group, a benzoyl group, a C1-C4 alkyl group, a C1-C4 alkoxy group, a halo-C1-C2 alkyl group, a halo-C1-C2 alkoxy group, a hydroxy C1-C4 alkyl group, a C1-C2 alkoxy C1-C2 alkoxy group, a (C1-C4 alkyl)carbonyl group, a (C1-C4 alkoxy)carbonyl group, a (C1-C4 alkoxy)carbonyloxy group, a phenyl C1-C4 alkoxy group, a morpholin-1-yl group, a carbamoyl group optionally substituted with one or two C1-C4 alkyl groups, a C1-C2 alkoxy group substituted by a carbamoyl group optionally substituted with one or two C1-C4 alkyl groups, a sulfamoyl group substituted with one or two C1-C4 alkyl groups, or a phenyl group optionally substituted with one or two substituents independently selected from a substituent group β3, particularly preferably, a fluorine atom, a chlorine atom, a trifluoromethyl group, a tert-butoxy group, a trifluoromethoxy group, a 2,2,2-trifluoroethoxy group, a benzyloxy group, or a phenoxy group.

Examples of the substituent group β in the present invention include a halogen atom, a C1-C6 alkyl group, a C1-C6 alkoxy group, a halo-C1-C6 alkyl group, a halo-Cl-C6 alkoxy group, and a (C1-C6 alkoxy)carbonyl group. Preferably, the substituent group β1 is a halogen atom, a C1-C6 alkyl group, a C1-C6 alkoxy group, or a (C1-C6 alkoxy)carbonyl group. More preferably, the substituent group β2 is a halogen atom, a C1-C6 alkyl group, or a C1-C6 alkoxy group. Even more preferably, the substituent group β3 is a fluorine atom, a chlorine atom, a C1-C4 alkyl group, or a C1-C4 alkoxy group, particularly preferably, a methyl group, or a methoxy group.

Examples of the substituent group γ in the present invention include the substituent group γ2 including a halogen atom, a C1-C6 alkyl group, a C1-C6 alkoxy group, a (C1-C6 alkyl)carbonyl group, and a (C1-C6 alkoxy)carbonyl group. Preferably, the substituent group γ3 is a halogen atom, a C1-C4 alkyl group, a C1-C4 alkoxy group, a (C1-C4 alkyl)carbonyl group, and a (C1-C4 alkoxy)carbonyl group, more preferably a fluorine atom, a chlorine atom, a methyl group, an isobutoxy group, or a tert-butoxycarbonyl group.

In the present invention, n can represent a numerical value of 0 or 1. When n = 0, the compound represented by formula (I) is a compound having a dihydropyrrolizinone structure represented by formula (II) below: 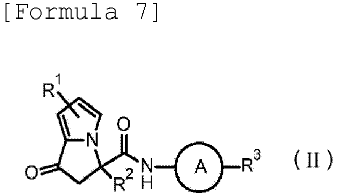 wherein R¹ to R³ have the same meanings as described above. When n = 1, the compound represented by formula (I) is a compound having a dihydroindolizinone structure represented by formula (III) below: 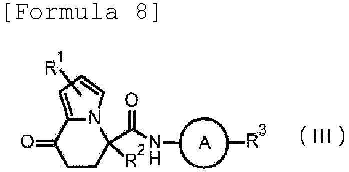 wherein R¹ to R³ have the same meanings as described above. In the present invention, n is preferably 1, and a compound having a dihydroindolizinone structure is more preferred.

In the present invention, A represents a group represented by formulae (i) to (iv) below: 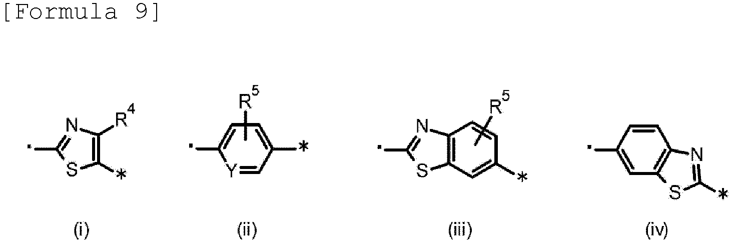 wherein ·, *, R⁴, R⁵, and Y have the same meanings as described above.

When A represents a group represented by formula (i), the compound represented by formula (I) is a compound represented by formula (IV) below: 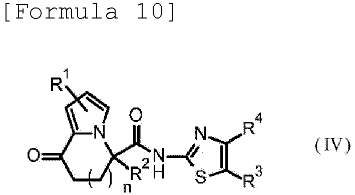 wherein R¹ to R⁴ and n have the same meanings as described above.

When A is a group represented by formula (ii), the compound represented by formula (I) is a compound represented by formula (V) below: 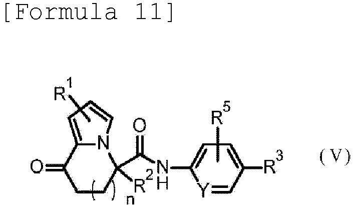 wherein R¹ to R³, R⁵, n, and Y have the same meanings as described above. The R⁵ substitution can be at any position shown below in (V-i) to (V-iii).

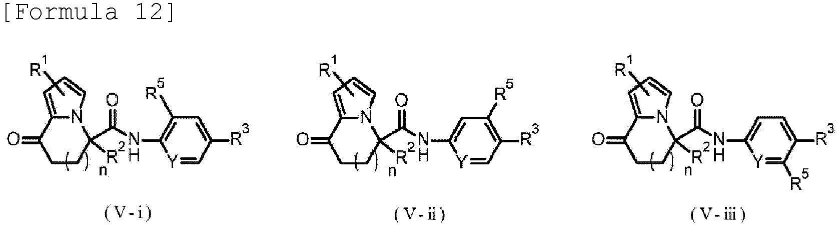

When A is a group represented by formula (iii), the compound represented by formula (I) is a compound represented by formula (VI) below: 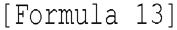 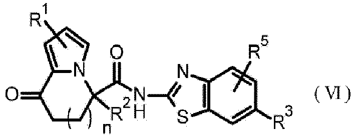 wherein R¹ to R³, R⁵, and n have the same meanings as described above. The R⁵ substitution can be at any position shown below in (VI-i) to (VI-iii).

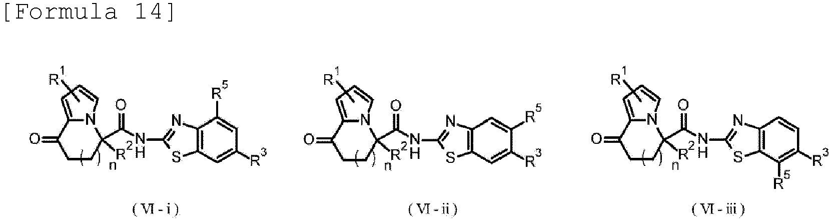

When A is a group represented by formula (iv), the compound represented by formula (I) is a compound represented by formula (VII) below: 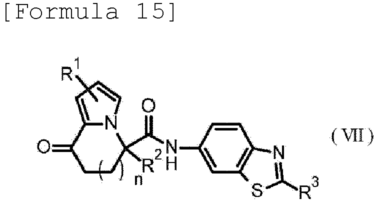 wherein R¹ to R³ and n have the same meanings as described above.

Examples of the substituent R⁴ in the present invention include a hydrogen atom, a C1-C6 alkyl group, a halo-C1-C6 alkyl group, or a (C1-C6 alkoxy)carbonyl group. Preferably, R⁴ is a hydrogen atom, a C1-C4 alkyl group, or a halo-C1-C2 alkyl group, more preferably a hydrogen atom, a methyl group, or a trifluoromethyl group.

Examples of the substituent R⁵ in the present invention include a hydrogen atom, a halogen atom, or a C1-C6 alkyl group. Preferably, R⁵ is a hydrogen atom, a fluorine atom, or a methyl group.

Examples of Y in the present invention include N or CH. When Y is N, (ii) in A represents a pyridine ring, and when Y is CH, (ii) in A represents a benzene ring. Y in (ii) of A is preferably CH.

The compound having formula (I) is preferably a compound described in the Synthesis Examples, more preferably the following compounds:
8-oxo-N-[5-[4-(trifluoromethoxy)phenyl]thiazol-2-yl]-6,7-dihydro-5H-indolizine-5-carboxamide
N-[5-(4-isopropoxyphenyl)thiazol-2-yl]-8-oxo-6,7-dihydro-5H-indolizine-5-carboxamide
N-[5-(4-tert-butoxyphenyl)-4-methylthiazol-2-yl]-8-oxo-6,7-dihydro-5H-indolizine-5-carboxamide
8-oxo-N-[5-[4-(2,2,2-trifluoroethoxy)phenyl]thiazol-2-yl]-6,7-dihydro-5H-indolizine-5-carboxamide
N-[6-(4-chlorophenyl)-1,3-benzothiazol-2-yl]-8-oxo-6,7-dihydro-5H-indolizine-5-carboxamide

The structural formulae thereof are as shown below in order.

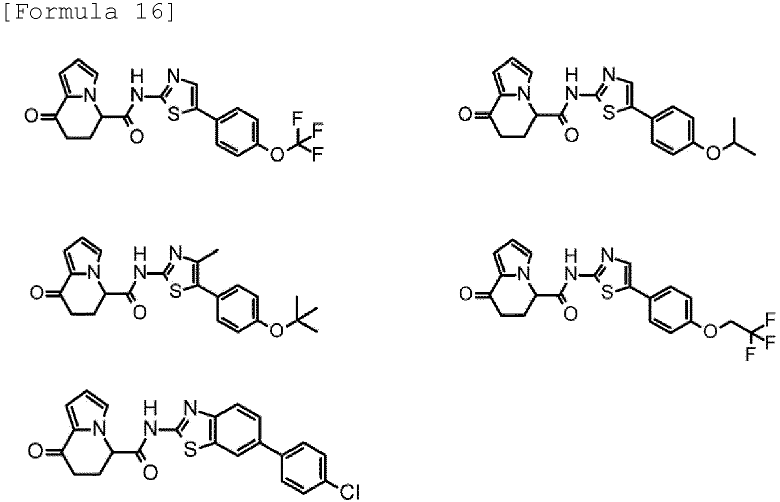

The aforementioned 8-oxo-N-[5-[4-(trifluoromethoxy)phenyl]thiazol-2-yl]-6,7-dihydro-5H-indolizine-5-carboxamide, and N-[5-(4-tert-butoxyphenyl)-4-methylthiazol-2-yl]-8-oxo-6,7-dihydro-5H-indolizine-5-carboxamide include their optically active forms.

The optically active form of 8-oxo-N-[5-[4-(trifluoromethoxy)phenyl]thiazol-2-yl]-6,7-dihydro-5H-indolizine-5-carboxamide is (5R)-8-oxo-N-[5-[4-(trifluoromethoxy)phenyl]thiazol-2-yl]-6,7-dihydro-5H-indolizine-5-carboxamide, or (5S)-8-oxo-N-[5-[4-(trifluoromethoxy)phenyl]thiazol-2-yl]-6,7-dihydro-5H-indolizine-5-carboxamide. The optically active form of N-[5-(4-tert-butoxyphenyl)-4-methylthiazol-2-yl]-8-oxo-6,7-dihydro-5H-indolizine-5-carboxamide is (5R)-N-[5-(4-tert-butoxyphenyl)-4-methylthiazol-2-yl]-8-oxo-6,7-dihydro-5H-indolizine-5-carboxamide, or (5S)-N-[5-(4-tert-butoxyphenyl)-4-methylthiazol-2-yl]-8-oxo-6,7-dihydro-5H-indolizine-5-carboxamide.

The structural formulae thereof are as shown below in order.

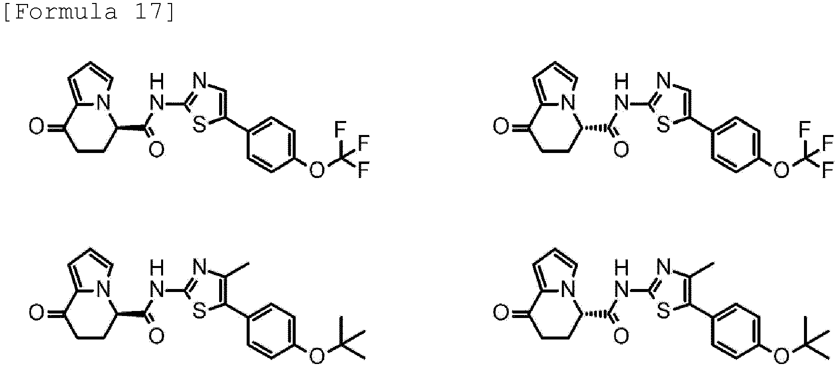

The separation and analysis of optical isomers from racemic compounds can be achieved by high-performance liquid chromatography (HPLC) using chiral columns. The identification of the optical isomers by HPLC can be performed with reference to the retention time but can be preferably performed by analyzing a mixture of a standard sample of such a racemic compound or optical isomer with an analysis sample because there may be cases where the retention time is affected by deterioration of columns, reproducibility between devices, and the like. Further, when measuring optical isomers by HPLC, there is no variation in the order in which the optical isomers are eluted under the same measurement conditions. Therefore, there may be cases where the optical isomers are characterized by a first peak in which the retention time is relatively short and a second peak in which the retention time is relatively long under specific conditions.

### (Salt)

A "salt thereof" means "a salt with a base" or "an acid addition salt" of a compound that can be obtained by reaction with a base or an acid in the case where the compound has an acidic group or a basic group. In use for treating warm-blooded animals (particularly humans), the salt thereof is preferably a pharmaceutically acceptable salt. Further, a "salt thereof" and a "pharmaceutically acceptable salt" also include hydrates thereof.

A "salt with a base" of the compound is preferably an alkali metal salt such as a sodium salt, potassium salt, and lithium salt; an alkaline earth metal salt such as a magnesium salt and calcium salt; an organic base salt such as a N-methylmorpholine salt, triethylamine salt, tributylamine salt, diisopropylethylamine salt, dicyclohexylamine salt, N-methylpiperidine salt, pyridine salt, 4-pyrrolidinopyridine salt, and picoline salt; or an amino acid salt such as a glycine salt, lysine salt, arginine salt, ornithine salt, glutamate, and aspartate, more preferably, an alkali metal salt or alkaline earth metal salt.

An "acid addition salt" of the compound is preferably a hydrohalide such as a hydrofluoric acid salt, hydrochloride, hydrobromide, and hydroiodide; an inorganic acid salt such as a nitrate, perchlorate, sulfate, and phosphate; a lower alkanesulfonate such as a methanesulfonate, trifluoromethanesulfonate, and ethanesulfonate; an arylsulfonate such as a benzenesulfonate and p-toluenesulfonate; an organic acid salt such as an acetate, malate, fumarate, succinate, citrate, ascorbate, tartrate, oxalate, and maleate; and an amino acid salt such as a glycine salt, lysine salt, arginine salt, ornithine salt, glutamate, and aspartate, more preferably, a hydrohalide (particularly, a hydrochloride).

### (Hydrates, etc.)

The compound represented by formula (I) or a salt thereof may absorb moisture, adhere to the adsorbed water, or become a hydrate by standing in the atmosphere or recrystallizing. The present invention includes such various hydrates, solvates, and crystalline polymorphic compounds.

### (Isomers)

There can be tautomers or geometric isomers of the compound represented by formula (I) corresponding to the types of substituents. In this description, the compound represented by formula (I) may be described as only one embodiment of such an isomer, but the present invention also includes other isomers than above, separated isomers, or mixtures thereof.

The compound represented by formula (I) may have asymmetric carbon atoms or axial asymmetry, and optical isomers based on these may exist. The present invention also includes separated optical isomers and mixtures thereof.

### (Isotopes)

The compound represented by formula (I) also includes label bodies, that is, compounds in which one or more atoms of the compound are substituted with isotopes (such as ²H, ³H, ¹³C, ¹⁴C, and ³⁵S).

### (Prodrugs)

The present invention also includes pharmacologically acceptable prodrugs of the compound represented by formula (I). Such a pharmacologically acceptable prodrug is a compound having a group that can be converted into an amino group, a hydroxyl group, a carboxy group, or the like by solvolysis or under physiological conditions. Examples of a group forming a prodrug include the group described in Prog. Med, 5, 2157-2161 (1985).

More specifically, in the case where an amino group is present in the compound, examples of a prodrug can include a compound with the amino group acylated or phosphorylated (for example, a compound with the amino group eicosanoylated, alanylated, pentylaminocarbonylated, (5-methyl-2-oxo-1,3-dioxolen-4-yl)methoxycarbonylated, tetrahydrofuranylated, pyrrolidylmethylated, or pivaloyloxymethylated).

In the case where a hydroxyl group is present in the compound, examples thereof can include a compound with the hydroxyl group acylated, alkylated, phosphorylated, or borated (for example, a compound with the hydroxyl group acetylated, palmitoylated, propanoylated, pivaloylated, succinylated, fumarylated, alanylated, or dimethylaminomethylcarbonylated).

In the case where a carboxy group is present in the compound, examples thereof include a compound with the carboxy group esterified or amidated (for example, a compound with the carboxy group ethyl esterified, phenyl esterified, carboxymethyl esterified, dimethylaminomethyl esterified, pivaloyloxymethyl esterified, ethoxycarbonyloxyethyl esterified, amidated, or methylamidated).

### (Production method)

Next, typical methods for producing a compound represented by formula (I) will be described. A compound represented by formula (I) can be produced by various production methods, and the production methods shown below are just examples. Therefore, the present invention should not be construed as being limited to these examples.

A compound represented by formula (I), a salt thereof, and a synthetic intermediate thereof can be produced by applying various known production methods using characteristics based on their basic skeletons or the types of substituents. As known methods, there are methods disclosed in "ORGANIC FUNCTIONAL GROUP PREPARATIONS", the second edition, ACADEMIC PRESS, INC., 1989 and "Comprehensive Organic Transformations", VCH Publishers Inc., 1989, for example.

Further, a compound represented by formula (I) and a salt thereof can be synthesized according to methods A to C described below. In the synthesis of a compound represented by formula (I), it may be effective as a manufacturing technology to protect functional groups with suitable protecting groups (groups that can be easily converted into the functional groups) at the transient stage from raw materials into an intermediate, depending on the types of functional groups. Examples of protecting groups can include the protecting groups disclosed in P. G. M. Wuts and T. W. Greene, Protective Groups in Organic Synthesis (the third edition, 1999), and these reaction conditions may be appropriately selected for use. In general, once a synthetic route is set by a person skilled in the art, protecting groups optimal for the synthetic route are appropriately set by a person skilled in the art.

In such a method, a desired compound can be obtained by introducing the protecting groups and performing reactions, and then removing the protecting groups, as required. Further, prodrugs of the compound represented by formula (I) can be produced by introducing specific groups at the transient stage from raw materials into an intermediate or further performing reactions using a compound obtained above, in the same manner as the aforementioned protecting groups. Each reaction can be performed by applying a general method such as esterification, amidation, and dehydration.

A compound represented by formula (I) can be produced using an intermediate that can be synthesized by a known method or a modification thereof. In particular, an intermediate containing a group represented by formulae (ii) to (iv), which corresponds to A, can be produced using commercially available raw materials by applying a known method or a modification thereof.

The compound to be obtained in each step of methods A to C below may be a salt formed with the compound. For example, hydrochloride, sulfate, sodium salt, potassium salt, or the like can be mentioned.

The solvent to be used in the reaction in each step of methods A to C below is not specifically limited, as long as it does not inhibit the reaction and partially dissolves the starting materials, and is selected, for example, from the following solvent group. The solvent group is composed of aliphatic hydrocarbons such as hexane, pentane, petroleum ether, and cyclohexane; aromatic hydrocarbons such as benzene, toluene, and xylenes; hydrocarbon halides such as methylene chloride (chlorinated methylene), chloroform, carbon tetrachloride, dichloroethane, chlorobenzene, and dichlorobenzene; ethers such as diethyl ether, diisopropyl ether, tetrahydrofuran, dioxane, dimethoxyethane, and diethylene glycol dimethyl ether; ketones such as acetone, methyl ethyl ketone, methyl isobutyl ketone, and cyclohexanone; esters such as ethyl acetate, propyl acetate, and butyl acetate; nitriles such as acetonitrile, propionitrile, butyronitrile, and isobutyronitrile; carboxylic acids such as acetic acid and propionic acid; alcohols such as methanol, ethanol, 1-propanol, 2-propanol, 1-butanol, 2-butanol, 1-methyl-1-propanol, and 2-methyl-2-propanol; amides such as formamide, dimethylformamide, dimethylacetamide, N-methyl-2-pyrrolidone, and hexamethylphosphate triamide; sulfoxides such as dimethylsulfoxide and sulfolane; water; and mixtures thereof.

The acid to be used in the reaction in each step of methods A to C below is not specifically limited, as long as it does not inhibit the reaction, and is selected from the following acid group. The acid group is composed of inorganic acids such as hydrochloric acid, hydrobromic acid, hydriodic acid, phosphoric acid, sulfuric acid, and nitric acid; organic acids such as acetic acid, propionic acid, trifluoroacetic acid, and pentafluoropropionic acid; and organic sulfonic acids such as methanesulfonic acid, trifluoromethanesulfonic acid, p-toluenesulfonic acid, and camphorsulfonic acid.

The base to be used in the reaction in each step of methods A to C below is not specifically limited, as long as it does not inhibit the reaction, and is selected from the following base group. The base group is composed of alkali metal carbonates such as lithium carbonate, sodium carbonate, potassium carbonate, and cesium carbonate; alkali metal bicarbonates such as lithium bicarbonate, sodium bicarbonate, and potassium bicarbonate; alkali metal hydroxides such as lithium hydroxide, sodium hydroxide, and potassium hydroxide; alkaline earth metal hydroxides such as calcium hydroxide and barium hydroxide; alkali metal hydrides such as lithium hydride, sodium hydride, and potassium hydride; alkali metal amides such as lithium amide, sodium amide, and potassium amide; alkali metal alkoxides such as lithium methoxide, sodium methoxide, sodium ethoxide, sodium tert-butoxide, and potassium tert-butoxide; lithium alkylamides such as lithium diisopropylamide; silylamides such as lithium bistrimethylsilylamide and sodium bistrimethylsilylamide; alkyl lithiums such as n-butyl lithium, sec-butyl lithium, and tert-butyl lithium; alkylmagnesium halides such as methylmagnesium chloride, methylmagnesium bromide, methylmagnesium iodide, ethylmagnesium chloride, ethylmagnesium bromide, isopropylmagnesium chloride, isopropylmagnesium bromide, and isobutylmagnesium chloride; and organic amines such as triethylamine, tributylamine, diisopropylethylamine, N-methylpiperidine, N-methylmorpholine, N-ethylmorpholine, pyridine, picoline, 4-(N,N-dimethylamino)pyridine, 4-pyrrolidinopyridine, 2,6-di(tert-butyl)-4-methylpyridine, quinoline, N,N-dimethylaniline, N,N-diethyl aniline, 1,5-diazabicyclo[4,3,0]non-5-ene (DBN), 1, 4-diazabicyclo[2,2,2]octane (DABCO), and 1, 8-diazabicyclo[5,4,0]undec-7-ene (DBU).

The reaction temperature in the reaction in each step of methods A to C below differs depending on the solvent, the starting materials, the reagent, and the like, and the reaction time differs depending on the solvent, the starting materials, the reagent, the reaction temperature, and the like.

In the reaction in each step of methods A to C below, the target compound of the step is isolated from the reaction mixture after the completion of the reaction according to a conventional method. The target compound is obtained, for example, by (i) leaking insoluble matter such as catalysts, as required, (ii) adding water and a solvent immiscible with water (such as methylene chloride, diethyl ether, and ethyl acetate) to the reaction mixture to extract the target compound, (iii) washing organic layers with water, followed by drying using a drying agent such as anhydrous magnesium sulfate, and (iv) distilling off the solvent. The target compound obtained can be further purified by a conventional method, such as recrystallization, reprecipitation, distillation, or column chromatography (including a normal phase and a reverse phase) using silica gel or alumina, as required. The target compound obtained can be identified by a standard analytical technique such as elemental analysis, NMR, mass spectrometry, and IR analysis, to analyze the composition and purity thereof. Alternatively, the target compound of each step can be used for the next reaction as it is without purification.

An optical isomer can be separated and purified in each step of methods A to C below by fractional recrystallization using optically active amines such as (R)- or (S)-phenethyl amine or separation using optically active columns.

Hereinafter, methods for producing a compound represented by formula (I) will be described. However, the production methods are not limited to the following methods at all.

### [Method A]

Method A is a method for producing a compound (A2) that can be used as a synthetic intermediate when producing the compound represented by formula (I). The compound (A2) can be produced by a known method or a modification thereof other than the synthesis methods shown in this method and Examples. 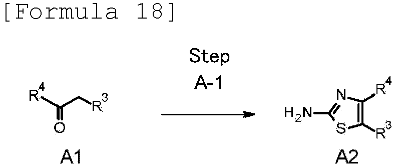 wherein R³ and R⁴ have the same meanings as described above.

### (Step A-1) Formation of thiazole ring

Step A-1 is a step of allowing an equal amount or excess amount of a halogenating agent or bromotrimethylsilane and thiourea to act on a compound (A1) to produce the compound (A2). Examples of the halogenating agent include chlorine and bromine. The solvent in the reaction is not specifically limited, as long as the reaction proceeds, but dichloromethane, chloroform, ethanol, acetonitrile, N,N-dimethylformamide, acetic acid, or the like is used. The reaction temperature is generally 0 to 100°C, and the reaction time is generally about 0.5 hours to 2 days.

### (Method B)

Method B is a method for producing a compound (B3) that can be used as a synthetic intermediate when producing the compound represented by formula (I). In the following figure, P¹ and P² each represent a protecting group of the amino group or a hydrogen atom. Specific examples of the protecting group include a Boc group (tert-butoxycarbonyl group), a Cbz group (benzyloxycarbonyl group), a benzylidene group, or a diphenylmethylene group. In the case where P¹ represents a benzylidene group or a diphenylmethylene group, P² represents the same protecting group as P¹. R³ and A may each have a protecting group on a substituent contained therein, and each step includes a step of protecting the substituent or removing the protecting group, as required. 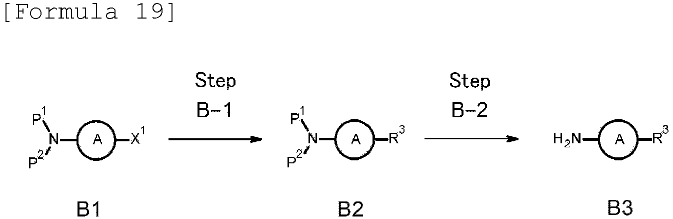 wherein R³ has the same meanings as described above, X¹ represents a halogen atom or a leaving group such as a methanesulfonyloxy group, a trifluoromethanesulfonyloxy group, or a p-toluenesulfonyloxy group, and P¹ and P² each represent any protecting group.

### (Step B-1) Coupling reaction

Step B-1 is a step of introducing the substituent R³ into a substituent X¹ on A of a compound (B1) in the presence of a palladium catalyst under conditions using an equal amount or an excess amount of boronic acid or boronic acid ester (R³-B(OH)₂ or R³-B(OR)₂, where R represents any alkyl group) (Suzuki-Miyaura coupling); conditions using an organic tin reagent (R³-SnR₃) (Stille coupling); or conditions using an organic zinc reagent (R³-ZnX, where X represents a halogen atom) (Negishi coupling), to obtain a compound (B2). In the aforementioned reaction, a base can be added, as required. Examples of the palladium catalyst include tetrakis (triphenylphosphine) palladium, [1,1'-bis(diphenylphosphino)ferrocene] palladium (II) dichloride dichloromethane complex (1:1), chloro(2-dicyclohexylphosphino-2'4'6'-triisopropyl-1,1'-biphenyl)[2-(2'-amino-1,1'-biphenyl)] palladium (II), tris(dibenzylideneacetone) dipalladium, palladium (II) acetate, palladium (II) acetylacetonate, or bis(triphenylphosphine) palladium (II) dichloride. Further, examples of the base include organic bases such as triethylamine, diisopropylethylamine, 1,8-diazabicyclo[5.4.0] undec-7-ene (DBU), or 1,5-diazabicyclo[4.3.0] non-5-ene (DBN), and inorganic bases such as potassium bicarbonate, sodium bicarbonate, potassium carbonate, sodium carbonate, potassium hydroxide, sodium hydroxide, potassium phosphate, or sodium phosphate. The reaction solvent is not specifically limited as long as the reaction proceeds, but examples thereof can include methanol, ethanol, tetrahydrofuran, 1,2-dimethoxyethane, 1,4-dioxane, water, N,N-dimethylformamide, dimethylsulfoxide, benzene, toluene, xylenes, or mixtures thereof. The reaction temperature is generally about 20 to 150°C. The reaction time is generally about 1 hour to 2 days. This coupling reaction can be performed according to the method described in A. Meijere and F. Diederich, "Metal-Catalyzed Cross-Coupling Reactions (the second edition, 2004)".

### (Step B-2) Deprotection

Step B-2 is a step of removing the protecting groups P¹ and P² in the compound (B2) to produce the compound (B3). In this step, deprotection of the protecting groups in R³ can be performed, as required. The reaction conditions thereof differ depending on the types of the protecting groups P¹ and P². The reaction can be performed, for example, according to the method described in T. W. Greene and P. G. Wuts,"Protective Groups in Organic Synthesis (the third edition, 1999)".

### (Method C)

Method C is a method for producing a compound represented by formula (I) from a compound (C1) that can be synthesized using a known method or a modification thereof. 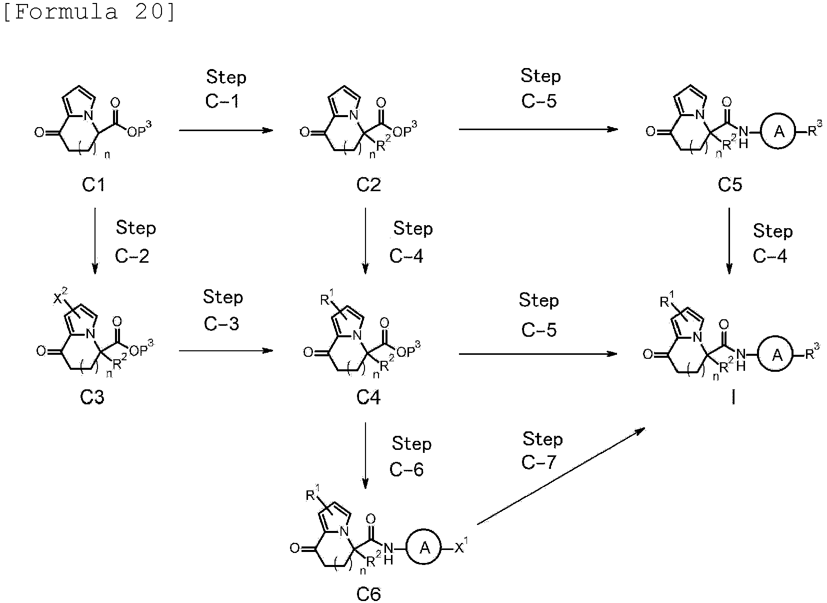 wherein R¹ to R³, n, A and X¹ have the same meanings as described above, X² represents a halogen atom, and P³ represents any protecting group.

### (Step C-1) Alkylation reaction

Step C-1 is a step of treating a compound (C1) with a base in the presence of an equal amount or an excess amount of an alkylating agent, thereby introducing the substituent R², to produce a compound (C2). As the alkylating agent, an alkyl halide, methanesulfonic acid alkyl ester, p-toluenesulfonic acid alkyl ester, or the like can be used. Examples of the base include potassium hexamethyldisilazide and sodium hexamethyldisilazide. The reaction solvent is not specifically limited, as long as the reaction proceeds, but is preferably tetrahydrofuran. The reaction temperature is generally - 78 to 0°C. The reaction time is generally 0.5 to 24 hours.

### (Step C-2) Halogenation and alkylation

Step C-2 is a step of halogenating a compound (C1) with an equal amount or an excess amount of a halogenating agent, followed by alkylation, to produce a compound (C3). The order of halogenation and alkylation can be appropriately replaced. Examples of the halogenating agent include N-bromosuccinimide and N-iodosuccinimide. The reaction solvent is not specifically limited, as long as the reaction proceeds, but is preferably dichloromethane or dimethylformamide. The reaction temperature is generally about 0 to 50°C. The reaction time is generally 0.5 to 24 hours. The alkylation can be performed in the same manner as in step C-1.

### (Step C-3) Coupling reaction

Step C-3 is a step of subjecting a compound (C3) to a coupling reaction, to produce a compound (C4). Step C-3 can be performed in the same manner as in step B-1.

### (Step C-4)

Step C-4 is a step of subjecting a compound (C2) or a compound (C5) to halogenation or a coupling reaction following the halogenation, to obtain a compound (C4) or a compound represented by formula (I). The halogenation can be performed in the same manner as in step C-2, and the coupling reaction can be performed in the same manner as in step B-1.

### (Step C-5) Condensation reaction

Step (C-5) is a step of removing the protecting groups of the carboxy group in a compound (C2) or a compound (C4) to form a carboxylic acid, followed by condensation using an equal amount or an excess amount of a compound (A2) of method A or a compound (B3) of method B, to produce a compound (C5) or a compound represented by formula (I). The deprotection of the carboxy group can be performed according to the method described in T. W. Greene and P. G. Wuts, "Protective Groups in Organic Synthesis (the third edition, 1999)". The condensation reaction is performed by allowing a suitable sulfonylating agent or a suitable condensing agent to act on the carboxylic acid obtained from the compound (C2) and the compound (C4) in the presence of a base. In the condensation reaction, an additive that promotes the reaction can be added, as required. Examples of the sulfonylating agent include 2,4,6-triisopropylbenzenesulfonyl chloride, p-toluenesulfonyl chloride, and benzenesulfonyl chloride. Examples of the condensing agent include WSC (1-ethyl-3-(3-dimethylaminopropyl)carbodiimide), DCC (1,3-dicyclohexylcarbodiimide), DMT-MM (4-(4,6-dimethoxy-1,3,5-triazin-2-yl)-4-methylmorpholinium chloride), CDI (1,1'-carbonyldiimidazole), DEPC (diethyl phosphorocyanidate), and DPPA (diphenylphosphorylazide). Examples of the base include aromatic amines such as pyridine and lutidine, and tertiary amines such as triethylamine, N,N-diisopropylethylamine, and DMAP (4-dimethylaminopyridine). Typical examples of the additive include HOAt (3H-[1,2,3]triazolo[4,5-b]pyridin-3-ol), HOBt (1H-benzotriazol-1-ol), and HOSu (N-hydroxysuccinimide). The reaction solvent is not specifically limited, as long as the reaction proceeds, but is preferably dichloromethane or N,N-dimethylformamide. The reaction temperature is generally 0 to 50°C. The reaction time is generally 0.5 to 24 hours.

### (Step C-6) Condensation reaction

Step (C-6) is a step of removing the protecting groups of the carboxy group in the compound (C4), followed by condensation with an amine derivative, to produce a compound (C6). The same method as in step (C-5) can be used.

### (Step C-7) Coupling reaction

Step (C-7) is a step of subjecting the compound (C6) to a coupling reaction, to produce a compound represented by formula (I). The same method as in step (B-1) can be used.

### <Insulin-producing cells>

Insulin-producing cells produced by a method of the present invention exhibit functions similar to those of native pancreatic β cells and are therefore useful for treating diabetes. Cells produced by a production method of the present invention have favorable ability to produce insulin as well as properties of decreasing the amount of insulin to be secreted in a situation with a low sugar level in an ambient environment and increasing the amount of insulin to be secreted in an environment with a high sugar level. Hence, the cells produced by a production method of the present invention have a low risk of hypoglycemia and provide a highly safe treatment method.

Cells produced by a method of the present invention can be confirmed to be cells targeted by evaluating the expression of pancreatic β cell marker genes (for example, insulin, NKX6.1, and PDX1) at the mRNA level or the protein level in the cells. Further, the ability of the cells produced by a method of the present invention to secrete insulin can be evaluated by time-dependent GSIS activity measurement described in the method (6) of Example 1 below.

Cells produced by a method of the present invention have properties of exhibiting rapid first-phase insulin secretion and/or long-lasting second-phase insulin secretion in time-dependent GSIS activity measurement. Further, the cells produced by a method of the present invention have properties of exhibiting insulin secretion enhancement, c-peptide secretion enhancement, or the like in response to stimulation with an insulin secretagogue (for example, a SU agent such as glibenclamide, a GLP-1 receptor agonist such as exendin 4, or KCl). Further, the cells have properties of having a high positive ratio of NKX6.1, a marker that exhibits functional maturation, and having a large amount of insulin to be secreted per cell. Owing to these properties, insulin-producing cells produced by a method of the present invention are considered as cells with enhanced functional maturation as pancreatic β cells, and are cells clearly differentiated from known insulin-producing cells derived from pluripotent stem cells.

A subject to be treated with such insulin-producing cells is a patient having a disease caused by abnormal insulin secretion or secretory disorder in pancreatic β cells, and specifically includes a patient with type 1 diabetes or type 2 diabetes.

In the case of using such cells in treatment, the cells can be transplanted subcutaneously, intraperitoneally, into the pancreas, to the surface of the pancreas, or to the neighborhood thereof in a patient.

Cells produced by a method of the present invention may be transplanted as an aggregate (sphere) formed by three-dimensional culture or may be transplanted as dispersed cells. Alternatively, an aggregate in which cells dispersed once are reaggregated, or a sheet processed therefrom may be transplanted.

Hereinafter, the present invention will be described further in detail by way of Reference Synthesis Examples, Synthesis Examples, Reference Examples, and Examples, but the scope of the present invention is not limited to these examples.

In the Reference Synthesis Examples and Synthesis Examples, elution in column chromatography was performed under observation by TLC (Thin Layer Chromatography). In the TLC observation, silica gel 60F₂₅₄ available from Merck KGaA was employed as a TLC plate, a solvent used as an elution solvent in column chromatography was employed as a developing solvent, and a UV detector was employed as a detection method. As a silica gel for the column, silica gel SK-85 also available from Merck KGaA (230 to 400 mesh) or Chromatorex NH available from FUJI SILYSIA CHEMICAL LTD. (200-350 mesh) was used. Other than normal column chromatography devices, an automatic chromatography device available from Shoko Science Co., Ltd. (Purif-α2 or Purif-espoir2) was appropriately used. An elution solvent was determined based on the TLC observation.

The abbreviations used in the Reference Synthesis Examples, Synthesis Examples, Reference Examples, and Examples below have the following meanings:
mg: milligram, g: gram, µL: microliter, mL: milliliter,
mmol: millimole, mM: millimolar concentration, µM: micromolar concentration, µm: micrometer, mm: millimeter, and MHz: megahertz.

In the nuclear magnetic resonance (which will be hereinafter referred to as ¹H NMR) spectrum in the Reference Synthesis Examples and Synthesis Examples below, chemical shift values were described in terms of δ values (ppm) using tetramethylsilane as a standard substance. The splitting pattern is indicated by s for singlet, d for doublet, t for triplet, q for quadruplet, m for multiplet, and br for broad. Mass spectrometry (which will be hereinafter referred to as MS) was performed by EI (Electron Ionization), ESI (Electrospray Ionization), or FAB (Fast Atom Bombardment).

### [Examples]

### Reference Synthesis Example 1

### N-(5-Bromothiazol-2-yl)-8-oxo-6,7-dihydro-5H-indolizine-5-carboxamide

To a solution of commercially available 8-oxo-6,7-dihydro-5H-indolizine-5-carboxylic acid (1.51 g, 8.43 mmol), commercially available 5-bromothiazol-2-amine (2.64 g, 10.2 mmol) and 3-hydroxytriazolo[4,5-b]pyridine (1.23 g, 9.04 mmol) in dichloromethane (30 mL) were added 1-ethyl-3-(3-dimethylaminopropyl)carbodiimide hydrochloride (2.43 g, 12.6 mmol) and N,N-diisopropylethylamine (2.94 mL, 16.9 mmol), followed by stirring at room temperature for 2 hours. The reaction solution was washed with 1N hydrochloric acid and a saturated aqueous solution of sodium chloride, dried over anhydrous sodium sulfate, and then filtered to distil off the solvent under reduced pressure. The residue was purified by silica-gel column chromatography (n-hexane/10% methanol-ethyl acetate solution = 3/1-0/1) to obtain 1.88 g (yield: 66%) of the title compound as a solid.

### Reference Synthesis Example 2

### N-(4-Bromophenyl)-8-oxo-6,7-dihydro-5H-indolizine-5-carboxamide

Using commercially available 4-bromoaniline, 4.66 g (yield: 84%) of the title compound was obtained as a solid according to the method of Reference Synthesis Example 1.

### Reference Synthesis Example 3

### N-(4-Bromo-2-fluorophenyl)-8-oxo-6,7-dihydro-5H-indolizine-5-carboxamide

Using commercially available 4-bromo-2-fluoroaniline, 345 mg (yield: 58%) of the title compound was obtained as a solid according to the method of Reference Synthesis Example 1.

### Reference Synthesis Example 4

### N-(5-Bromo-4-methylthiazol-2-yl)-8-oxo-6,7-dihydro-5H-indolizine-5-carboxamide

Using commercially available 5-bromo-4-methyl-thiazol-2-amine, 252 mg (yield: 25%) of the title compound was obtained as a solid according to the method of Reference Synthesis Example 1.

### Reference Synthesis Example 5

### N-(6-Bromo-1,3-benzothiazol-2-yl)-8-oxo-6,7-dihydro-5H-indolizine-5-carboxamide

Using commercially available 2-amino-6-bromobenzothiazole, 585 mg (yield: 42%) of the title compound was obtained as a solid according to the method of Reference Synthesis Example 1.

### Reference Synthesis Example 6

### N-(4-Bromo-2-methylphenyl)-8-oxo-6,7-dihydro-5H-indolizine-5-carboxamide

Using commercially available 4-bromo-2-methylaniline, 515 mg (yield: 66%) of the title compound was obtained as a solid according to the method of Reference Synthesis Example 1.

### Reference Synthesis Example 7

### N-(5-Bromo-2-pyridyl)-8-oxo-6,7-dihydro-5H-indolizine-5-carboxamide

Using commercially available 5-bromopyridin-2-amine, 455 mg (yield: 58%) of the title compound was obtained as a solid according to the method of Reference Synthesis Example 1.

### Reference Synthesis Example 8

### N-(2-Chloro-1,3-benzothiazol-6-yl)-8-oxo-6,7-dihydro-5H-indolizine-5-carboxamide

Using commercially available 2-chloro-1,3-benzothiazol-6-amine, 637 mg (yield: 76%) of the title compound was obtained as a solid according to the method of Reference Synthesis Example 1.

### Reference Synthesis Example 9

### 5-[4-(Trifluoromethoxy)phenyl]thiazol-2-amine

To a mixture of N-(5-bromothiazol-2-yl)-1,1-diphenylmethanimine disclosed in International Publication No. WO 2003014095 (1.46 g, 4.25 mmol), commercially available 4-(trifluoromethoxy)phenylboronic acid (4.30 g, 20.0 mmol), potassium carbonate (2.97 g, 21.5 mmol), water (3 mL) and 1,4-dioxane (15 mL) was added a [1,1'-bis(diphenylphosphino)ferrocene]palladium(II) dichloride-dichloromethane complex (1:1) (338 mg, 0.414 mmol), followed by stirring at 100°C under a nitrogen atmosphere for 4 hours. After water was added to the reaction mixture, it was extracted with ethyl acetate. The organic layers were combined, washed with water and saturated saline, and then dried over anhydrous sodium sulfate. The solvent was distilled off under reduced pressure, and the residue obtained was purified by silica-gel column chromatography (n-hexane/ethyl acetate = 9/1-3/1). To a solution of an oil material obtained in methanol (20 mL) was added 1N hydrochloric acid (5 mL, 5.0 mmol), followed by stirring at room temperature for 5.5 hours. The reaction mixture was concentrated under reduced pressure, and the precipitated solid was washed with dichloromethane to obtain 897 mg (yield: 81%) of the title compound as a solid.

### Reference Synthesis Example 10

### tert-Butyl N-[5-[4-(dimethylcarbamoyl)phenyl]thiazol-2-yl]carbamate

To a solution of commercially available tert-butyl N-(5-bromothiazol-2-yl) carbamate (500 mg, 1.89 mmol) in 1,4-dioxane (30 mL) were added [4-(dimethylcarbamoyl)phenyl]boronic acid (519 mg, 2.69 mmol) and a [1,1'-bis(diphenylphosphino)ferrocene]palladium(II) dichloride-dichloromethane complex (1:1) (74 mg, 0.09 mmol) at room temperature, followed by stirring. To the reaction mixture were added potassium carbonate (743 mg, 5.38 mmol) and water (3.0 mL), followed by stirring at 100°C in an argon atmosphere for 4.5 hours. After water was added to the reaction mixture, it was extracted with ethyl acetate. The organic layers were combined, washed with water and saturated saline, and then dried over anhydrous sodium sulfate. The solvent was distilled off under reduced pressure, and the residue obtained was purified by silica-gel column chromatography (dichloromethane/methanol = 100/0-93/7, 90/10-70/30) to obtain 172 mg (yield: 28%) of the title compound as a solid.

### Reference Synthesis Example 11

### 4-(2-Aminothiazol-5-yl)-N,N-dimethylbenzamide

To a solution of the tert-butyl N-[5-[4-(dimethylcarbamoyl)phenyl]thiazol-2-yl]carbamate obtained in Reference Synthesis Example 10 (172 mg, 0.495 mmol) in dichloromethane (10 mL) was added trifluoroacetic acid (2.0 mL, 26 mmol) at room temperature, followed by stirring and thereafter standing. After the reaction solution was concentrated under reduced pressure, and a saturated aqueous solution of sodium bicarbonate was added thereto, the mixture was extracted with dichloromethane. After the organic layers were dried over anhydrous sodium sulfate, the solvent was distilled off under reduced pressure to obtain 118 mg (yield: 96%) of the title compound as a solid.

### Reference Synthesis Example 12

### 5-(1,3-Benzodioxol-5-yl)thiazol-2-amine

Using commercially available 1,3-benzodioxol-5-ylboronic acid, a product was obtained according to the method of Reference Synthesis Example 10. Thereafter, 123 mg (31%, 2 steps) of the title compound was obtained as a solid according to the method of Reference Synthesis Example 11.

### Reference Synthesis Example 13

### 4-(2-Aminothiazol-5-yl)benzonitrile

A solution of bromine (56 µL, 1.09 mmol) in dichloromethane (0.56 mL) was added to a solution of commercially available 4-(2-oxoethyl)benzonitrile (142 mg, 0978 mmol) in dichloromethane (10 mL) under ice cooling. After the reaction solution was warmed to room temperature, the mixture was stirred for 3 hours. After neutralization by adding a saturated aqueous solution of sodium bicarbonate to the reaction solution under ice cooling, the mixture was extracted with dichloromethane. After the organic layers were combined and dried over anhydrous sodium sulfate, the solvent was distilled off under reduced pressure. To a solution of the residue obtained in ethanol (30 mL) was added thiourea (150 mg, 1.97 mmol), followed by stirring under heating reflux for 4.5 hours. After cooling, the solvent was distilled off under reduced pressure, and the residue obtained was purified by silica-gel column chromatography (dichloromethane/methanol = 99/1-95/5) to obtain 8.3 mg (4.2%) of the title compound as a solid.

### Reference Synthesis Example 14

### tert-Butyl 2-[4-[2-(tert-butoxycarbonylamino)thiazol-5-yl]phenoxy]acetate

Using commercially available tert-butyl 2-[4-(4,4,5,5-tetramethyl-1,3,2-dioxaborolan-2-yl) phenoxy]acetate (2.4 g, 7.2 mmol), 248 mg (11%) of the title compound was obtained as a solid according to the method of Reference Synthesis Example 10.

### Reference Synthesis Example 15

### Methyl 2-[4-(2-aminothiazol-5-yl)phenoxy]acetate

Using the tert-butyl 2-[4-[2-(tert-butoxycarbonylamino)thiazol-5-yl]phenoxy]acetate (193 mg, 0.475 mmol) obtained in Reference Synthesis Example 14, a solid was obtained according to the method of Reference Synthesis Example 11. To a mixed solution of the solid obtained in tetrahydrofuran (10 mL) and methanol (3 mL) was added trimethylsilyldiazomethane (0.6M hexane solution, 1.0 mL) at room temperature, followed by stirring for 6 hours. After the reaction solution was concentrated under reduced pressure and a saturated aqueous solution of sodium bicarbonate was added thereto, the mixture was extracted with dichloromethane. After the organic layers were dried over anhydrous sodium sulfate, the solvent was distilled off under reduced pressure, and the residue obtained was purified by silica-gel column chromatography (dichloromethane/methanol = 99/1-97/3) to obtain 92.5 mg (yield: 74%) of the title compound as a solid.

### Reference Synthesis Example 16

### Methyl 2-[4-[2-[(8-oxo-6,7-dihydro-5H-indolizine-5-carbonyl)amino]thiazol-5-yl]phenoxy]acetate

Using the methyl 2-[4-(2-aminothiazol-5-yl)phenoxy]acetate obtained in Reference Synthesis Example 15, 85.7 mg (yield: 89%) of the title compound was obtained as a solid according to the method of Synthesis Example 1.

### Reference Synthesis Example 17

### Methyl 2-bromo-8-oxo-6,7-dihydro-5H-indolizine-5-carboxylate

To a solution of commercially available methyl 8-oxo-6,7-dihydro-5H-indolizine-5-carboxylate (261 mg, 1.35 mmol) in dichloromethane (5 mL) was added N-bromosuccinimide (228 mg, 1.28 mmol) at room temperature, followed by stirring for 4 hours. The solvent was distilled off under reduced pressure, and the residue obtained was purified by silica-gel column chromatography (n-hexane/ethyl acetate = 4/1-2/1) to obtain 56.9 mg (yield: 16%) of the title compound as a solid.

### Reference Synthesis Example 18

### 2-Bromo-8-oxo-N-[5-[4-(trifluoromethoxy)phenyl]thiazol-2-yl]-6,7-dihydro-5H-indolizine-5-carboxamide

To a solution of the methyl 2-bromo-8-oxo-6,7-dihydro-5H-indolizine-5-carboxylate obtained in Reference Synthesis Example 17 (56.9 mg, 0.209 mmol) in ethanol (2 mL) was added a 1N sodium hydroxide aqueous solution (0.5 mL, 0.50 mmol), followed by stirring at 60°C for 2 hours. After acidification with 1N hydrochloric acid, the mixture was extracted with ethyl acetate. After the organic layers were dried over anhydrous sodium sulfate, the solvent was distilled off under reduced pressure, to obtain a yellow solid (52.5 mg). Using the solid obtained (52.5 mmol) and the 5-[4-(trifluoromethoxy)phenyl]thiazol-2-amine obtained in Reference Synthesis Example 9, 48.0 mg (yield: 55%) of the title compound was obtained as a solid according to the method of Synthesis Example 9.

### Reference Synthesis Example 19

### N-(5-Bromothiazol-2-yl)-2-methyl-8-oxo-6,7-dihydro-5H-indolizine-5-carboxamide

To a mixture of the methyl 2-bromo-8-oxo-6,7-dihydro-5H-indolizine-5-carboxylate (65.2 mg, 0.24 mmol) obtained in Reference Synthesis Example 17, commercially available 2,4,6-trimethyl-1,3,5,2,4,6-trioxatriborinane (0,201 mL, 1.44 mmol) and potassium carbonate (171 mg, 1.24 mmol) were added 1,4-dioxane (1.5 mL) and water (0.5 mL). Thereafter, [1,1'-bis(di-tert-butylphosphino)ferrocene]palladium(II) dichloride (15.6 mg, 0.024 mmol) was further added thereto, followed by stirring at 100°C under a nitrogen atmosphere for 2.5 hours. After water and 1N hydrochloric acid were added to the reaction solution, it was extracted with ethyl acetate. After the organic layers were washed with saturated saline and dried over anhydrous sodium sulfate, the solvent was distilled off under reduced pressure. Using the residue obtained, 23.9 mg (yield: 22%) of the title compound was obtained as a solid according to the method of Reference Synthesis Example 1.

### Reference Synthesis Example 20

### Methyl 2-chloro-8-oxo-6,7-dihydro-5H-indolizine-5-carboxylate

Using commercially available N-chlorosuccinimide, 56.3 mg (yield: 5.2%) of the title compound was obtained as a solid according to the method of Reference Synthesis Example 17.

### Reference Synthesis Example 21

### Methyl 5-methyl-8-oxo-6,7-dihydroindolizine-5-carboxylate

To a solution of commercially available methyl 8-oxo-6,7-dihydro-5H-indolizine-5-carboxylate (987 mg, 4.89 mmol) and methyl iodide (1.27 mL, 20.4 mmol) in tetrahydrofuran was added a 1.0M potassium bis(trimethylsilyl)amide tetrahydrofuran solution (11.2 mL, 11.2 mmol) dropwise over 30 minutes or more at -78°C. After the mixture was stirred at the same temperature for 1 hour, a saturated aqueous solution of ammonium chloride was added thereto, followed by quenching. After water was added to the reaction solution, it was extracted with ethyl acetate. The organic layers were washed with saturated saline and dried over anhydrous sodium sulfate. The solvent was distilled off under reduced pressure, and the residue obtained was purified by silica-gel column chromatography (n-hexane/ethyl acetate = 6/1-1/1) to obtain 592 mg (yield: 56%) of the title compound as an oil material.

### Reference Synthesis Example 22

### 5-Methyl-8-oxo-6,7-dihydroindolizine-5-carboxylic acid

To a solution of the methyl 5-methyl-8-oxo-6,7-dihydroindolizine-5-carboxylate obtained in Reference Synthesis Example 21 (63.0 mg, 0.304 mmol) in ethanol (1.0 mL) was added a 1N sodium hydroxide aqueous solution (0.91 mL, 0.91 mmol), followed by stirring at 80°C for 2 hours. After neutralization by adding 1N hydrochloric acid to the reaction solution, it was extracted with ethyl acetate. The organic layers were washed with saturated saline and dried over anhydrous sodium sulfate. The solvent was distilled off under reduced pressure to obtain 48.3 mg (yield: 82%) of the title compound as a solid.

### Reference Synthesis Example 23

### N-(5-Bromothiazol-2-yl)-5-methyl-8-oxo-6,7-dihydroindolizine-5-carboxamide

Using the 5-methyl-8-oxo-6,7-dihydroindolizine-5-carboxylic acid (472 mg, 2.44 mmol) obtained in Reference Synthesis Example 22, 628 mg (yield: 73%) of the title compound was obtained as a solid according to the method of Reference Synthesis Example 1.

### Reference Synthesis Example 24

### N-(5-Bromo-4-isopropylthiazol-2-yl)-8-oxo-6,7-dihydro-5H-indolizine-5-carboxamide

Using commercially available 5-bromo-4-isopropylthiazol-2-amine, 287 mg (yield: 43%) of the title compound was obtained as a solid according to the method of Reference Synthesis Example 1.

### Reference Synthesis Example 25

### Methyl 5-bromo-2-[(8-oxo-6,7-dihydro-5H-indolizine-5-carbonyl)amino]thiazol-4-carboxylate

Using commercially available methyl 2-amino-5-bromothiazole-4-carboxylate, 219 mg (yield: 29%) of the title compound was obtained as an amorphous material according to the method of Reference Synthesis Example 1.

### Synthesis Example 1

### 8-Oxo-N-(5-phenylthiazol-2-yl)-6,7-dihydro-5H-indolizine-5-carboxamide

To a solution of commercially available 8-oxo-6,7-dihydro-5H-indolizine-5-carboxylic acid (1.5 g, 8.4 mmol), 5-phenylthiazol-2-amine described in Journal of Medicinal Chemistry 1983, 26, 1158-1163 (1.0 g, 5.7 mmol), N,N-dimethylpyridin-4-amine (0.14 g, 1.1 mmol) and N,N-diisopropylethylamine (2.5 mL, 14 mmol) in dichloromethane (50 mL) was added commercially available 2,4,6-triisopropylsulfonyl chloride (2.5 g, 8.3 mmol) at room temperature, followed by stirring for 3 hours. After the reaction solution was diluted by adding dichloromethane, a saturated aqueous solution of sodium bicarbonate was added for neutralization and washing. After organic layers were dried over anhydrous sodium sulfate, the solvent was distilled off under reduced pressure. The residue was purified by silica-gel column chromatography (dichloromethane/methanol = 99/1-95/5) to obtain 1.59 g (yield: 83%) of the title compound as a solid.

### Synthesis Example 2

### Methyl 4-[2-[(8-oxo-6,7-dihydro-5H-indolizine-5-carbonyl)amino]thiazol-5-yl]benzoate

Using the methyl 4-(2-aminothiazol-5-yl)benzoate described in International Publication No. WO 2012121168, 18.4 g (yield: 54%) of the title compound was obtained as a solid according to the method of Synthesis Example 1.

### Synthesis Example 3

### N-[5-[4-(Dimethylcarbamoyl)phenyl]thiazol-2-yl]-8-oxo-6,7-dihydro-5H-indolizine-5-carboxamide

Using the 4-(2-aminothiazol-5-yl)-N,N-dimethylbenzamide obtained in Reference Synthesis Example 11, 7.17 g (yield: 75%) of the title compound was obtained as a solid according to the method of Synthesis Example 1.

### Synthesis Example 4

### N-[5-(1,3-Benzodioxol-5-yl)thiazol-2-yl]-8-oxo-6,7-dihydro-5H-indolizine-5-carboxamide

Using the 5-(1,3-benzodioxol-5-yl)thiazol-2-amine obtained in Reference Synthesis Example 12, 130 mg (yield: 62%) of the title compound was obtained as a solid according to the method of Synthesis Example 1.

### Synthesis Example 5

### 4-[2-[(8-Oxo-6,7-dihydro-5H-indolizine-5-carbonyl)amino]thiazol-5-yl]benzoic acid

To a mixed solution of the methyl 4-[2-[(8-oxo-6,7-dihydro-5H-indolizine-5-carbonyl)amino]thiazol-5-yl]benzoate (383 mg, 0.969 mmol) obtained in Synthesis Example 2 with ethanol (3 mL) and water (2 mL) was added a 1N sodium hydroxide aqueous solution (3.8 mL, 3.8 mmol), followed by stirring at 50°C for 4 hours. Water was added to the reaction solution, followed by neutralization with 1N hydrochloric acid, and the precipitated solid was collected by filtration. The solid obtained was washed with water and thereafter dried to obtain 326 mg (yield: 88%) of the title compound as a solid.

### Synthesis Example 6

### N-[5-(4-Carbamoylphenyl)thiazol-2-yl]-8-oxo-6,7-dihydro-5H-indolizine-5-carboxamide

To a solution of the 4-[2-[(8-oxo-6,7-dihydro-5H-indolizine-5-carbonyl)amino]thiazol-5-yl]benzoic acid obtained in Synthesis Example 5 (17 mg, 0.045 mmol) in N,N-dimethylformamide (1 mL) were added [dimethylamino(triazolo[4,5-b]pyridin-3-yloxy)methylene]-dimethyl-ammonium hexafluorophosphate (25 mg, 0.067 mmol), N,N-diisopropylethylamine (39 µL, 0.22 mmol) and a 7M ammonia methanol solution (64 µL, 0.45 mmol) at room temperature, followed by stirring for 15 hours. The reaction solution was purified by direct silica-gel column chromatography (dichloromethane/50% methanol ethyl acetate solution = 95/5-90/10) to obtain 4.9 mg (yield: 29%) of the title compound.

### Synthesis Example 7

### N-[5-(4-Cyanophenyl)thiazol-2-yl]-8-oxo-6,7-dihydro-5H-indolizine-5-carboxamide

Using the 4-(2-aminothiazol-5-yl)benzonitrile obtained in Reference Synthesis Example 13, 9.0 mg (yield: 60%) of the title compound was obtained according to the method of Synthesis Example 1.

### Synthesis Example 8

### N-[5-[4-[2-(Dimethylamino)-2-oxo-ethoxy]phenyl]thiazol-2-yl]-8-oxo-6,7-dihydro-5H-indolizine-5-carboxamide

To a mixed solution of the methyl 2-[4-[2-[(8-oxo-6,7-dihydro-5H-indolizine-5-carbonyl)amino]thiazol-5-yl]phenoxy]acetate obtained in Reference Synthesis Example 16 (84 mg, 0.197 mmol) in tetrahydrofuran (8.0 mL) and methanol (3.0 mL) was added a 5N aqueous solution of sodium hydroxide (0.20 mL, 1.0 mmol) at room temperature, followed by stirring and thereafter standing overnight. After neutralization with 5N hydrochloric acid, the solvent was distilled off under reduced pressure, to obtain a solid. To a solution of the solid obtained in N,N-dimethylformamide (3 mL) were added N,N-diisopropylethylamine (52 µL, 0.30 mmol) and a 2.0M solution of dimethylamine tetrahydrofuran (0.50 mL, 1.0 mmol) at room temperature. Thereafter, [dimethylamino(triazolo[4,5-b]pyridin-3-yloxy)methylene]dimethylammonium hexafluorophosphate (42 mg, 0.11 mmol) was further added thereto, followed by stirring for 3 hours. After the reaction solution was diluted by adding dichloromethane, a saturated aqueous solution of sodium bicarbonate was added for neutralization and washing. After the organic layers were dried over anhydrous sodium sulfate, the solvent was distilled off under reduced pressure. The residue was purified by silica-gel column chromatography (dichloromethane/methanol = 99/1-95/5) to obtain 39.1 mg (yield: 89%) of the title compound as a solid.

### Synthesis Example 9

### 8-Oxo-N-[5-[4-(trifluoromethoxy)phenyl]thiazol-2-yl]-6,7-dihydro-5H-indolizine-5-carboxamide

To a solution of commercially available 8-oxo-6,7-dihydro-5H-indolizine-5-carboxylic acid (109 mg, 0.61 mmol), the 5-[4-(trifluoromethoxy)phenyl]thiazol-2-amine obtained in Reference Synthesis Example 9 (127 mg, 0.488 mmol) and 3-hydroxytriazole [4,5-b]pyridine (128 mg, 0.941 mmol) in dichloromethane (3 mL) were added 1-ethyl-3-(3-dimethylaminopropyl)carbodiimide hydrochloride (352 mg, 1.84 mmol) and N,N-diisopropylethylamine (0.531 mL, 3.05 mmol) at room temperature, followed by stirring for 2 hours at room temperature. The reaction solution was diluted with dichloromethane and thereafter washed with water and a saturated aqueous solution of sodium chloride. The organic layers were dried over anhydrous sodium sulfate and thereafter filtered to distill off the solvent under reduced pressure. The residue was purified by silica-gel column chromatography (n-hexane/ethyl acetate solution = 3/1-0/1) to obtain 74.3 mg (yield: 29%) of the title compound as a solid.

### Synthesis Example 10

### (5R)-8-Oxo-N-[5-[4-(trifluoromethoxy)phenyl]thiazol-2-yl]-6,7-dihydro-5H-indolizine-5-carboxamide and (5S)-8-oxo-N-[5-[4-(trifluoromethoxy)phenyl]thiazol-2-yl]-6,7-dihydro-5H-indolizine-5-carboxamide

Using the 8-oxo-N-[5-[4-(trifluoromethoxy)phenyl]thiazol-2-yl]-6,7-dihydro-5H-indolizine-5-carboxamide obtained in Synthesis Example 9, optical resolution was performed by HPLC (column: YMC CHIRAL ART Cellulose-SB (5 µm) 250 x 30 mm I.D., flow rate: 31.8 ml/min, and solvent: n-hexane/ethanol = 70/30). After the first peak eluted earlier was collected, the solvent was distilled off under reduced pressure to obtain the title compound (48 mg, optical purity: 99.9%ee) as a solid. Further, after the second peak eluted later was collected, the solvent was distilled off under reduced pressure to obtain the title compound (48 mg, optical purity: 99.8%ee) as a solid.

### Synthesis Example 11

### N-[5-(4-Tert-butoxyphenyl)thiazol-2-yl]-8-oxo-6,7-dihydro-5H-indolizine-5-carboxamide

To a mixture of the N-(5-bromothiazol-2-yl)-8-oxo-6,7-dihydro-5H-indolizine-5-carboxamide obtained in Reference Synthesis Example 1 (137 mg, 0.401 mmol), commercially available (4-tert-butoxyphenyl)boronic acid (389 mg, 2.01 mmol) and cesium carbonate (670 mg, 2.06 mmol) were added N,N-dimethylformamide (2 mL) and water (1 mL). Thereafter, chloro(2-dicyclohexylphosphino-2',4',6'-triisopropyl-1,1'-biphenyl)[2-(2'-amino-1,1'-biphenyl)]palladium(II) (33.2 mg, 0.042 mmol) was added thereto, followed by stirring at 90°C under a nitrogen atmosphere for 5 hours. After water was added to the reaction solution, it was extracted with ethyl acetate. After organic layers were washed with saturated saline and dried over anhydrous sodium sulfate, the solvent was distilled off under reduced pressure. The residue was purified by silica-gel column chromatography (n-hexane/ethyl acetate = 3/1-0/1) to obtain 63.3 mg (yield: 39%) of the title compound as a solid.

### Synthesis Example 12

### N-[5-(4-Chlorophenyl)thiazol-2-yl]-8-oxo-6,7-dihydro-5H-indolizine-5-carboxamide

Using commercially available 4-chlorophenylboronic acid, 11.0 mg (yield: 6.4%) of the title compound was obtained as a solid according to the method of Synthesis Example 11.

### Synthesis Example 13

### N-[5-(3-Chlorophenyl)thiazol-2-yl]-8-oxo-6,7-dihydro-5H-indolizine-5-carboxamide

Using commercially available 5-(3-chlorophenyl)thiazol-2-amine, 158 mg (yield: 48%) of the title compound was obtained as a solid according to the method of Synthesis Example 9.

### Synthesis Example 14

### N-[5-(2-Chlorophenyl)thiazol-2-yl]-8-oxo-6,7-dihydro-5H-indolizine-5-carboxamide

Using commercially available 2-chlorophenylboronic acid, 13.2 mg (yield: 8.0%) of the title compound was obtained according to the method of Synthesis Example 11.

### Synthesis Example 15

### N-[5-(3-Fluorophenyl)thiazol-2-yl]-8-oxo-6,7-dihydro-5H-indolizine-5-carboxamide

Using commercially available 5-(3-fluorophenyl)thiazol-2-amine, 127 mg (yield: 87%) of the title compound was obtained as a solid according to the method of Synthesis Example 1.

### Synthesis Example 16

### N-[5-(4-Fluorophenyl)thiazol-2-yl]-8-oxo-6,7-dihydro-5H-indolizine-5-carboxamide

Using commercially available 5-(4-fluorophenyl)thiazol-2-amine, 198 mg (yield: 64%) of the title compound was obtained as a solid according to the method of Synthesis Example 1.

### Synthesis Example 17

### N-[5-(2-Fluorophenyl)thiazol-2-yl]-8-oxo-6,7-dihydro-5H-indolizine-5-carboxamide

Using commercially available 5-(2-fluorophenyl)thiazol-2-amine, 119 mg (yield: 82%) of the title compound was obtained as a solid according to the method of Synthesis Example 1.

### Synthesis Example 18

### 8-Oxo-N-[5-(p-tolyl)thiazol]-2-yl)-6,7-dihydro-5H-indolizine-5-carboxamide

Using commercially available 4-methylphenylboronic acid, 24.7 mg (yield: 18%) of the title compound was obtained as a solid according to the method of Synthesis Example 11.

### Synthesis Example 19

### N-[5-(o-Tolyl)thiazol-2-yl]-8-oxo-6,7-dihydro-5H-indolizine-5-carboxamide

Using commercially available 2-methylphenylboronic acid, 16.6 mg (yield: 11%) of the title compound was obtained as a solid according to the method of Synthesis Example 11.

### Synthesis Example 20

### N-[5-(m-Tolyl)thiazol-2-yl]-8-oxo-6,7-dihydro-5H-indolizine-5-carboxamide

Using commercially available 5-(m-tolyl)thiazol-2-amine, 115 mg (yield: 58%) of the title compound was obtained according to the method of Synthesis Example 9.

### Synthesis Example 21

### 8-Oxo-N-[5-[4-(trifluoromethyl)phenyl]thiazol-2-yl]-6,7-dihydro-5H-indolizine-5-carboxamide

Using commercially available [4-(trifluoromethyl)phenyl]boronic acid, 32.9 mg (yield: 17%) of the title compound was obtained according to the method of Synthesis Example 11.

### Synthesis Example 22

### N-[5-[4-(2-Methoxyphenyl)phenyl]thiazol-2-yl]-8-oxo-6,7-dihydro-5H-indolizine-5-carboxamide

Using commercially available [4-(2-methoxyphenyl)phenyl]boronic acid, 3.6 mg (yield: 16%) of the title compound was obtained according to the method of Synthesis Example 11.

### Synthesis Example 23

### 8-Oxo-N-[5-[4-(p-tolyl)phenyl]thiazol-2-yl]-6,7-dihydro-5H-indolizine-5-carboxamide

Using commercially available [4-(p-tolyl)phenyl]boronic acid, 9.0 mg (yield: 42%) of the title compound was obtained according to the method of Synthesis Example 11.

### Synthesis Example 24

### N-[5-(2-Naphthyl)thiazol-2-yl]-8-oxo-6,7-dihydro-5H-indolizine-5-carboxamide

Using commercially available 4,4,5,5-tetramethyl-2-(2-naphthyl)-1,3,2-dioxaborolane, 1.6 mg (yield: 8.2%) of the title compound was obtained according to the method of Synthesis Example 11.

### Synthesis Example 25

### 8-Oxo-N-[5-[3-(trifluoromethoxy)phenyl]thiazol-2-yl]-6,7-dihydro-5H-indolizine-5-carboxamide

Using commercially available [3-(trifluoromethoxy)phenyl]boronic acid, 9.0 mg (yield: 43%) of the title compound was obtained according to the method of Synthesis Example 11.

### Synthesis Example 26

### N-[5-(4-methoxyphenyl)thiazol-2-yl]-8-oxo-6,7-dihydro-5H-indolizine-5-carboxamide

Using commercially available (4-methoxyphenyl)boronic acid, 19.7 mg (yield: 17%) of the title compound was obtained as a solid according to the method of Synthesis Example 11.

### Synthesis Example 27

### N-[5-(3-Methoxyphenyl)thiazol-2-yl]-8-oxo-6,7-dihydro-5H-indolizine-5-carboxamide

Using commercially available (3-methoxyphenyl)boronic acid, 1.9 mg (yield: 38%) of the title compound was obtained according to the method of Synthesis Example 11.

### Synthesis Example 28

### N-[5-[2-Methyl-4-(trifluoromethoxy)phenyl]thiazol-2-yl]-8-oxo-6,7-dihydro-5H-indolizine-5-carboxamide

Using commercially available [2-methyl-4-(trifluoromethoxy)phenyl]boronic acid, 4.2 mg (yield: 19%) of the title compound was obtained according to the method of Synthesis Example 11.

### Synthesis Example 29

### N-[5-[3-Methyl-4-(trifluoromethoxy)phenyl]thiazol-2-yl]-8-oxo-6,7-dihydro-5H-indolizine-5-carboxamide

Using commercially available [3-methyl-4-(trifluoromethoxy)phenyl]boronic acid, 8.4 mg (yield: 38%) of the title compound was obtained according to the method of Synthesis Example 11.

### Synthesis Example 30

### N-[5-[3-Chloro-4-(trifluoromethoxy)phenyl]thiazol-2-yl]-8-oxo-6,7-dihydro-5H-indolizine-5-carboxamide

Using commercially available [3-chloro-4-(trifluoromethoxy)phenyl]boronic acid, 2.8 mg (yield: 12%) of the title compound was obtained according to the method of Synthesis Example 11.

### Synthesis Example 31

### N-[5-[3-(Hydroxymethyl)-4-(trifluoromethoxy)phenyl]thiazol-2-yl]-8-oxo-6,7-dihydro-5H-indolizine-5-carboxamide

Using commercially available [3-(hydroxymethyl)-4-(trifluoromethoxy)phenyl]boronic acid, 9.1 mg (yield: 40%) of the title compound was obtained according to the method of Synthesis Example 11.

### Synthesis Example 32

### N-[5-(4-Benzyloxyphenyl)thiazol-2-yl]-8-oxo-6,7-dihydro-5H-indolizine-5-carboxamide

Using commercially available (4-benzyloxyphenyl)boronic acid, 7.6 mg (yield: 34%) of the title compound was obtained according to the method of Synthesis Example 11.

### Synthesis Example 33

### N-[5-(4-Isopropoxyphenyl)thiazol-2-yl]-8-oxo-6,7-dihydro-5H-indolizine-5-carboxamide

Using commercially available (4-isopropoxyphenyl)boronic acid, 29.0 mg (yield: 24%) of the title compound was obtained as a solid according to the method of Synthesis Example 11.

### Synthesis Example 34

### N-(4-Methyl-5-phenylthiazol-2-yl)-8-oxo-6,7-dihydro-5H-indolizine-5-carboxamide

Using commercially available 4-methyl-5-phenylthiazol-2-amine, 160 mg (yield: 82%) of the title compound was obtained according to the method of Synthesis Example 9.

### Synthesis Example 35

### N-[5-(4-tert-Butoxyphenyl)-4-methylthiazol-2-yl]-8-oxo-6,7-dihydro-5H-indolizine-5-carboxamide

To a mixture of the N-(5-bromo-4-methylthiazol-2-yl)-8-oxo-6,7-dihydro-5H-indolizine-5-carboxamide obtained in Reference Synthesis Example 4 (106 mg, 0.300 mmol), commercially available (4-tert-butoxyphenyl)boronic acid (292 mg, 1.50 mmol) and cesium carbonate (596 mg, 1.83 mmol) were added N,N-dimethylformamide (1.5 mL) and water (1 mL). Thereafter, chloro(2-dicyclohexylphosphino-2',4',6'-triisopropyl-1,1'-biphenyl)[2-(2'-amino-1,1'-biphenyl)]palladium(II) (25.0 mg, 0.032 mmol) was added thereto, followed by stirring at 90°C under a nitrogen atmosphere for 5 hours. After water was added to the reaction solution, it was extracted with ethyl acetate. After organic layers were washed with saturated saline and dried over anhydrous sodium sulfate, the solvent was distilled off under reduced pressure. The residue was purified by silica-gel column chromatography (n-hexane/ethyl acetate = 3/1-0/1). Thereafter, the solid obtained was washed with diethyl ether to obtain 65.0 mg (yield: 51%) of the title compound as a solid.

### Synthesis Example 36

### N-[4-Methyl-5-[4-(trifluoromethoxy)phenyl]thiazol-2-yl]-8-oxo-6,7-dihydro-5H-indolizine-5-carboxamide

Using commercially available [4-(trifluoromethoxy)phenyl]boronic acid, 72.0 mg (yield: 49%) of the title compound was obtained according to the method of Synthesis Example 35.

### Synthesis Example 37

### N-[5-[4-(Hydroxymethyl)phenyl]thiazol-2-yl]-8-oxo-6,7-dihydro-5H-indolizine-5-carboxamide

Using commercially available [4-(hydroxymethyl)phenyl]boronic acid, 5.3 mg (yield: 29%) of the title compound was obtained according to the method of Synthesis Example 11.

### Synthesis Example 38

### N-[5-[4-(2-Methoxyethoxy)phenyl]thiazol-2-yl]-8-oxo-6,7-dihydro-5H-indolizine-5-carboxamide

Using commercially available [4-(2-methoxyethoxy)phenyl]boronic acid, 0.6 mg (yield: 2.7%) of the title compound was obtained according to the method of Synthesis Example 11.

### Synthesis Example 39

### N-[5-[4-(Difluoromethoxy)phenyl]thiazol-2-yl]-8-oxo-6,7-dihydro-5H-indolizine-5-carboxamide

Using commercially available [4-(difluoromethoxy)phenyl]boronic acid, 7.0 mg (yield: 35%) of the title compound was obtained according to the method of Synthesis Example 11.

### Synthesis Example 40

### N-[5-(2,2-Difluoro-1,3-benzodioxol-5-yl)thiazol-2-yl]-8-oxo-6,7-dihydro-5H-indolizine-5-carboxamide

Using commercially available (2,2-difluoro-1,3-benzodioxol-5-yl)boronic acid, 9.4 mg (yield: 45%) of the title compound was obtained according to the method of Synthesis Example 11.

### Synthesis Example 41

### N-[5-(4-Morpholinophenyl)thiazol-2-yl]-8-oxo-6,7-dihydro-5H-indolizine-5-carboxamide

Using commercially available 4-[4-(4,4,5,5-tetramethyl-1,3,2-dioxaborolan-2-yl)phenyl]morpholine, 27.6 mg (yield: 24%) of the title compound was obtained as a solid according to the method of Synthesis Example 11.

### Synthesis Example 42

### N-[5-[4-(Dimethylsulfamoyl)phenyl]thiazol-2-yl]-8-oxo-6,7-dihydro-5H-indolizine-5-carboxamide

Using commercially available [4-(dimethylsulfamoyl)phenyl]boronic acid, 9.7 mg (yield: 44%) of the title compound was obtained according to the method of Synthesis Example 11.

### Synthesis Example 43

### N-[5-(4-Benzyloxy-3-fluorophenyl)thiazol-2-yl]-8-oxo-6,7-dihydro-5H-indolizine-5-carboxamide

Using commercially available (4-benzyloxy-3-fluorophenyl)boronic acid, 62.8 mg (yield: 33%) of the title compound was obtained as a solid according to the method of Synthesis Example 11.

### Synthesis Example 44

### N-[5-(4-Benzyloxy-2-fluorophenyl)thiazol-2-yl]-8-oxo-6,7-dihydro-5H-indolizine-5-carboxamide

Using commercially available (4-benzyloxy-2-fluorophenyl)boronic acid, 9.7 mg (yield: 42%) of the title compound was obtained according to the method of Synthesis Example 11.

### Synthesis Example 45

### 8-Oxo-N-[5-[4-(2,2,2-trifluoroethoxy)phenyl]thiazol-2-yl]-6,7-dihydro-5H-indolizine-5-carboxamide

Using commercially available [4-(2,2,2-trifluoroethoxy)phenyl]boronic acid, 53.0 mg (yield: 28%) of the title compound was obtained as a solid according to the method of Synthesis Example 11.

### Synthesis Example 46

### 8-Oxo-N-[5-(4-phenoxyphenyl)thiazol-2-yl]-6,7-dihydro-5H-indolizine-5-carboxamide

Using commercially available (4-phenoxyphenyl)boronic acid, 53.0 mg (yield: 29%) of the title compound was obtained as a solid according to the method of Synthesis Example 11.

### Synthesis Example 47

### N-[5-(4-Bromophenyl)thiazol-2-yl]-8-oxo-6,7-dihydro-5H-indolizine-5-carboxamide

Using commercially available 5-(4-bromophenyl)thiazol-2-amine, 755 mg (yield: 59%) of the title compound was obtained as a solid according to the method of Synthesis Example 9.

### Synthesis Example 48

### tert-Butyl[4-[2-[(8-oxo-6,7-dihydro-5H-indolizine-5-carbonyl)amino]thiazol-5-yl]phenyl]carbonate

Using commercially available (4-tert-butoxycarbonyloxyphenyl)boronic acid, 1.0 mg (yield: 4.3%) of the title compound was obtained according to the method of Synthesis Example 11.

### Synthesis Example 49

### N-[5-(4-Isobutoxyphenyl)thiazol-2-yl]-8-oxo-6,7-dihydro-5H-indolizine-5-carboxamide

Using commercially available (4-isobutoxyphenyl)boronic acid, 6.5 mg (yield: 32%) of the title compound was obtained according to the method of Synthesis Example 11.

### Synthesis Example 50

### N-[5-(Cyclohexen-1-yl)thiazol-2-yl]-8-oxo-6,7-dihydro-5H-indolizine-5-carboxamide

Using commercially available 2-(cyclohexen-1-yl)-4,4,5,5-tetramethyl-1,3,2-dioxaborolane, 13.8 mg (yield: 13%) of the title compound was obtained as a solid according to the method of Synthesis Example 11.

### Synthesis Example 51

### tert-Butyl 4-[2-[(8-oxo-6,7-dihydro-5H-indolizine-5-carbonyl)amino]thiazol-5-yl]-3,6-dihydro-2H-pyridine-1-carboxylate

Using commercially available tert-butyl 4-(4,4,5,5-tetramethyl-1,3,2-dioxaborolan-2-yl)-3,6-dihydro-2H-pyridine-1-carboxylate, 7.7 mg (yield: 35%) of the title compound was obtained according to the method of Synthesis Example 11.

### Synthesis Example 52

### N-[5-(5-Chloro-6-isobutoxy-3-pyridyl)thiazol-2-yl]-8-oxo-6,7-dihydro-5H-indolizine-5-carboxamide

Using commercially available (5-chloro-6-isobutoxy-3-pyridyl)boronic acid, 3.2 mg (yield: 15%) of the title compound was obtained according to the method of Synthesis Example 11.

### Synthesis Example 53

### tert-Butyl 3-[2-[(8-oxo-6,7-dihydro-5H-indolizine-5-carbonyl)amino]thiazol-5-yl]-2,5-dihydropyrrole-1-carboxylate

Using commercially available tert-butyl 3-(4,4,5,5-tetramethyl-1,3,2-dioxaborolan-2-yl)-2,5-dihydropyrrole-1-carboxylate, 7.6 mg (yield: 35%) of the title compound was obtained according to the method of Synthesis Example 11.

### Synthesis Example 54

### N-[5-(5-Methyl-2-furyl)thiazol-2-yl]-8-oxo-6,7-dihydro-5H-indolizine-5-carboxamide

Using commercially available 4,4,5,5-tetramethyl-2-(5-methyl-2-furyl)-1,3,2-dioxaborolane, 7.3 mg (yield: 43%) of the title compound was obtained according to the method of Synthesis Example 11.

### Synthesis Example 55

### N-[5-(2,4-Dimethylthiazol-5-yl)thiazol-2-yl]-8-oxo-6,7-dihydro-5H-indolizine-5-carboxamide

Using commercially available 2,4-dimethyl-5-(4,4,5,5-tetramethyl-1,3,2-dioxaborolan-2-yl)thiazole, 3.6 mg (yield: 19%) of the title compound was obtained according to the method of Synthesis Example 11.

### Synthesis Example 56

### N-[5-(5-Chloro-2-thienyl)thiazol-2-yl]-8-oxo-6,7-dihydro-5H-indolizine-5-carboxamide

Using commercially available (5-chloro-2-thienyl)boronic acid, 1.0 mg (yield: 5.2%) of the title compound was obtained according to the method of Synthesis Example 11.

### Synthesis Example 57

### N-[5-(4-Acetylphenyl)thiazol-2-yl]-8-oxo-6,7-dihydro-5H-indolizine-5-carboxamide

Using commercially available (4-acetylphenyl)boronic acid, 6.2 mg (yield: 33%) of the title compound was obtained according to the method of Synthesis Example 11.

### Synthesis Example 58

### 1-Oxo-N-[5-[4-(trifluoromethoxy)phenyl]thiazol-2-yl]-2,3-dihydropyrrolidine-3-carboxamide

Using commercially available 1-oxo-2,3-dihydropyrrolidine-3-carboxylic acid, 71.9 mg (yield: 29%) of the title compound was obtained as a solid according to the method of Synthesis Example 9.

### Synthesis Example 59

### 8-Oxo-N-[6-[4-(trifluoromethoxy)phenyl]-1,3-benzothiazol-2-yl]-6,7-dihydro-5H-indolizine-5-carboxamide

Using the N-(6-bromo-1,3-benzothiazol-2-yl)-8-oxo-6,7-dihydro-5H-indolizine-5-carboxamide obtained in Reference Synthesis Example 5 and commercially available [4-(trifluoromethoxy)phenyl]boronic acid, 59.5 mg (yield: 46%) of the title compound was obtained as a solid according to the method of Synthesis Example 11.

### Synthesis Example 60

### N-[6-[4-(Difluoromethoxy)phenyl]-1,3-benzothiazol-2-yl]-8-oxo-6,7-dihydro-5H-indolizine-5-carboxamide

Using the N-(6-bromo-1,3-benzothiazol-2-yl)-8-oxo-6,7-dihydro-5H-indolizine-5-carboxamide obtained in Reference Synthesis Example 5 and commercially available [4-(difluoromethoxy)phenyl]boronic acid, 11.2 mg (yield: 49%) of the title compound was obtained according to the method of Synthesis Example 11.

### Synthesis Example 61

### N-[6-(2,2-Difluoro-1,3-benzodioxol-5-yl)-1,3-benzothiazol-2-yl]-8-oxo-6,7-dihydro-5H-indolizine-5-carboxamide

Using the N-(6-bromo-1,3-benzothiazol-2-yl)-8-oxo-6,7-dihydro-5H-indolizine-5-carboxamide obtained in Reference Synthesis Example 5 and commercially available (2,2-difluoro-1,3-benzodioxol-5-yl)boronic acid, 12.6 mg (yield: 54%) of the title compound was obtained according to the method of Synthesis Example 11.

### Synthesis Example 62

### 2-Methyl-8-oxo-N-[5-[4-(trifluoromethoxy)phenyl]thiazol-2-yl]-6,7-dihydro-5H-indolizine-5-carboxamide

To a mixture of the 2-bromo-8-oxo-N-[5-[4-(trifluoromethoxy)phenyl]thiazol-2-yl]-6,7-dihydro-5H-indolizine-5-carboxamide obtained in Reference Synthesis Example 18 (42.8 mg, 0.0856 mmol), commercially available 2,4,6-trimethyl-1,3,5,2,4,6-trioxatriborinane (0.127 mL, 0.908 mmol) and potassium carbonate (184 mg, 1.33 mmol) were added 1,4-dioxane (1.5 mL) and water (0.5 mL). Thereafter, chloro(2-dicyclohexylphosphino-2'4'6'-triisopropyl-1,1'-biphenyl)[2-(2'-amino-1,1'-biphenyl)]palladium(II) (15.3 mg, 0.019 mmol) was added thereto, followed by stirring at 100°C under a nitrogen atmosphere for 4 hours. Since the raw materials remained, the same operation was repeated again, and disappearance of the raw materials was confirmed. After water was added to the reaction solution, it was extracted with ethyl acetate. After the organic layers were washed with saturated saline and dried over anhydrous sodium sulfate, the solvent was distilled off under reduced pressure. The residue was purified by silica-gel column chromatography (n-hexane/ethyl acetate = 9/1-1/1) to obtain 5.6 mg (yield: 15%) of the title compound as a solid.

### Synthesis Example 63

### N-[5-(4-tert-Butoxyphenyl)thiazol-2-yl]-2-methyl-8-oxo-6,7-dihydro-5H-indolizine-5-carboxamide

Using the N-(5-bromothiazol-2-yl)-2-methyl-8-oxo-6,7-dihydro-5H-indolizine-5-carboxamide obtained in Reference Synthesis Example 19, 6.4 mg (yield: 22%) of the title compound was obtained as a solid according to the method of Synthesis Example 11.

### Synthesis Example 64

### 2-Chloro-8-oxo-N-[5-[4-(trifluoromethoxy)phenyl]thiazol-2-yl]-6,7-dihydro-5H-indolizine-5-carboxamide

To a mixed solution of the methyl 2-chloro-8-oxo-6,7-dihydro-5H-indolizine-5-carboxylate obtained in Reference Synthesis Example 20 (66.2 mg, 0.291 mmol) in ethanol (1 mL) and tetrahydrofuran (1 mL) was added a 1N sodium hydroxide aqueous solution (0.5 mL, 0.5 mmol) at room temperature, followed by stirring for 2 hours. After water was added to the reaction solution, the mixture was acidified by adding 1N hydrochloric acid and extracted with ethyl acetate. After the organic layers were washed with saturated saline and dried over anhydrous sodium sulfate, the solvent was distilled off under reduced pressure. Using the residue obtained (carboxylic acid compound 62 mg), 68.0 mg (yield: 37%) of the title compound was obtained as a solid according to the method of Synthesis Example 9.

### Synthesis Example 65

### 5-Methyl-8-oxo-N-[5-[4-trifluoromethoxy]phenyl]thiazol-2-yl]-6,7-dihydroindolizine-5-carboxamide

To a mixture of the N-(5-bromothiazol-2-yl)-5-methyl-8-oxo-6,7-dihydroindolizine-5-carboxamide obtained in Reference Synthesis Example 23 (168 mg, 0.475 mmol), commercially available [4-(trifluoromethoxy)phenyl]boronic acid (588 mg, 2.86 mmol) and cesium carbonate (1.03 g, 3.16 mmol) were added N,N-dimethylformamide (2 mL) and water (1 mL). Thereafter, chloro(2-dicyclohexylphosphino-2'4'6'-triisopropyl-1,1'-biphenyl)[2-(2'-amino-1,1'-biphenyl)]palladium(II) (38.6 mg, 0.049 mmol) was added thereto, followed by stirring at 90°C under a nitrogen atmosphere for 4 hours. After water was added to the reaction solution, it was extracted with ethyl acetate. After the organic layers were washed with saturated saline and dried over anhydrous sodium sulfate, the solvent was distilled off under reduced pressure. The residue was purified by silica-gel column chromatography (n-hexane/ethyl acetate = 3/1-0/1) to obtain 59.7 mg (yield: 29%) of the title compound as a solid.

### Synthesis Example 66

### 5-Methyl-8-oxo-N-[5-(p-tolyl)thiazol-2-yl]-6,7-dihydroindolizine-5-carboxamide

Using commercially available 4-methylphenylboronic acid, 8.6 mg (yield: 47%) of the title compound was obtained according to the method of Synthesis Example 65.

### Synthesis Example 67

### 5-Methyl-N-[5-(m-tolyl)thiazol-2-yl]-8 oxo-6,7-dihydroindolizine-5-carboxamide

Using commercially available 3-methylphenylboronic acid, 8.1 mg (yield: 44%) of the title compound was obtained according to the method of Synthesis Example 65.

### Synthesis Example 68

### N-[5-(2,2-Difluoro-l,3-benzodioxol-5-yl)thiazol-2-yl]-5-methyl-8-oxo-6,7-dihydroindolizine-5-carboxamide

Using commercially available (2,2-difluoro-1,3-benzodioxol-5-yl)boronic acid, 0.6 mg (yield: 3.0%) of the title compound was obtained according to the method of Synthesis Example 65.

### Synthesis Example 69

### N-[5-(4-Isopropoxyphenyl)thiazol-2-yl]-5-methyl-8-oxo-6,7-dihydroindolizine-5-carboxamide

Using commercially available (4-isopropoxyphenyl)boronic acid, 6.8 mg (yield: 33%) of the title compound was obtained according to the method of Synthesis Example 65.

### Synthesis Example 70

### 5-Methyl-N-[5-[3-methyl-4-(trifluoromethoxy)phenyl]thiazol-2-yl]-8-oxo-6,7-dihydroindolizine-5-carboxamide

Using commercially available [3-methyl-4-(trifluoromethoxy)phenyl]boronic acid, 8.3 mg (yield: 37%) of the title compound was obtained according to the method of Synthesis Example 65.

### Synthesis Example 71

### N-[5-[3-Chloro-4-(trifluoromethoxy)phenyl]thiazol-2-yl]-5-methyl-8-oxo-6,7-dihydroindolizine-5-carboxamide

Using commercially available [3-chloro-4-(trifluoromethoxy)phenyl]boronic acid, 5.0 mg (yield: 21%) of the title compound was obtained according to the method of Synthesis Example 65.

### Synthesis Example 72

### 5-Methyl-8-oxo-N-[5-[3-(trifluoromethoxy)phenyl]thiazol-2-yl]-6,7-dihydroindolizine-5-carboxamide

Using commercially available [3-(trifluoromethoxy)phenyl]boronic acid, 7.8 mg (yield: 36%) of the title compound was obtained according to the method of Synthesis Example 65.

### Synthesis Example 73

### 8-Oxo-N-[4-[4-(trifluoromethoxy)phenyl]phenyl]-6,7-dihydro-5H-indolizine-5-carboxamide

Using the N-(4-bromophenyl)-8-oxo-6,7-dihydro-5H-indolizine-5-carboxamide obtained in Reference Synthesis Example 2 (1.21 g, 3.62 mmol) and commercially available 4-(trifluoromethoxyphenyl)boronic acid, 1.29 g (yield: 86%) of the title compound was obtained as a solid according to the method of Synthesis Example 11.

### Synthesis Example 74

### N-[4-(2,2-Difluoro-1,3-benzodioxol-5-yl)phenyl]-8-oxo-6,7-dihydro-5H-indolizine-5-carboxamide

Using the N-(4-bromophenyl)-8-oxo-6,7-dihydro-5H-indolizine-5-carboxamide obtained in Reference Synthesis Example 2 and commercially available (2,2-difluoro-1,3-benzodioxol-5-yl)boronic acid, 10.4 mg (yield: 50%) of the title compound was obtained according to the method of Synthesis Example 11.

### Synthesis Example 75

### N-[4-[4-(Difluoromethoxy)phenyl]phenyl]-8-oxo-6,7-dihydro-5H-indolizine-5-carboxamide

Using the N-(4-bromophenyl)-8-oxo-6,7-dihydro-5H-indolizine-5-carboxamide obtained in Reference Synthesis Example 2 and commercially available [4-(difluoromethoxy)phenyl]boronic acid, 11.5 mg (yield: 58%) of the title compound was obtained according to the method of Synthesis Example 11.

### Synthesis Example 76

### N-[4-[3-Chloro-4-(trifluoromethoxy)phenyl]phenyl]-8-oxo-6,7-dihydro-5H-indolizine-5-carboxamide

Using the N-(4-bromophenyl)-8-oxo-6,7-dihydro-5H-indolizine-5-carboxamide obtained in Reference Synthesis Example 2 and commercially available [3-chloro-4-(trifluoromethoxy)phenyl]boronic acid, 7.0 mg (yield: 31%) of the title compound was obtained according to the method of Synthesis Example 11.

### Synthesis Example 77

### 3-Bromo-8-oxo-N-[4-[4-(trifluoromethoxy)phenyl]phenyl]-6,7-dihydro-5H-indolizine-5-carboxamide

To a solution of the 8-oxo-N-[4-[4-(trifluoromethoxy)phenyl]phenyl]-6,7-dihydro-5H-indolizine-5-carboxamide obtained in Synthesis Example 73 (150 mg, 0.363 mmol) in dichloromethane (15 mL) was added N-bromosuccinimide (45.9 mg, 0.258 mmol) at room temperature, followed by stirring for 4.5 hours. The reaction solution was concentrated under reduced pressure, and the residue obtained was purified by silica-gel column chromatography (n-hexane/10% methanol ethyl acetate solution = 3/1-1/1) to obtain 93.6 mg (yield: 52%) of the title compound as a solid.

### Synthesis Example 78

### N-[2-Fluoro-4-[4-(trifluoromethoxy)phenyl]phenyl]-8-oxo-6,7-dihydro-5H-indolizine-5-carboxamide

Using the N-(4-bromo-2-fluorophenyl)-8-oxo-6,7-dihydro-5H-indolizine-5-carboxamide obtained in Reference Synthesis Example 3 and commercially available [4-(trifluoromethoxy)phenyl]boronic acid, 73.4 mg (yield: 91%) of the title compound was obtained as a solid according to the method of Synthesis Example 11.

### Synthesis Example 79

### N-[4-[3-Chloro-4-(trifluoromethoxy)phenyl]-2-fluorophenyl]-8-oxo-6,7-dihydro-5H-indolizine-5-carboxamide

Using the N-(4-bromo-2-fluorophenyl)-8-oxo-6,7-dihydro-5H-indolizine-5-carboxamide obtained in Reference Synthesis Example 3 and commercially available [3-chloro-4-(trifluoromethoxy)phenyl]boronic acid, 5.0 mg (yield: 21%) of the title compound was obtained according to the method of Synthesis Example 11.

### Synthesis Example 80

### N-[4-(4-tert-Butoxyphenyl)-2-fluorophenyl]-8-oxo-6,7-dihydro-5H-indolizine-5-carboxamide

Using the N-(4-bromo-2-fluorophenyl)-8-oxo-6,7-dihydro-5H-indolizine-5-carboxamide obtained in Reference Synthesis Example 3, 75.0 mg (yield: 84%) of the title compound was obtained as a solid according to the method of Synthesis Example 11.

### Synthesis Example 81

### N-[2-Methyl-4-[4-(trifluoromethoxy)phenyl]phenyl]-8-oxo-6,7-dihydro-5H-indolizine-5-carboxamide

Using the N-(4-bromo-2-methylphenyl)-8-oxo-6,7-dihydro-5H-indolizine-5-carboxamide obtained in Reference Synthesis Example 6 and commercially available [4-(trifluoromethoxy)phenyl]boronic acid, 184 mg (yield: 92%) of the title compound was obtained as a solid according to the method of Synthesis Example 11.

### Synthesis Example 82

### 8-Oxo-N-[5-[4-(trifluoromethoxy)phenyl]-2-pyridyl]-6,7-dihydro-5H-indolizine-5-carboxamide

Using the N-(5-bromo-2-pyridyl)-8-oxo-6,7-dihydro-5H-indolizine-5-carboxamide obtained in Reference Synthesis Example 7 and commercially available [4-(trifluoromethoxy)phenyl]boronic acid, 4.0 mg (yield: 17%) of the title compound was obtained according to the method of Synthesis Example 11.

### Synthesis Example 83

### 8-Oxo-N-[2-[4-(trifluoromethoxy)phenyl]-1,3-benzotriazol-6-yl]-6,7-dihydro-5H-indolizine-5-carboxamide

Using the N-(2-chloro-1,3-benzothiazol-6-yl)-8-oxo-6,7-dihydro-5H-indolizine-5-carboxamide obtained in Reference Synthesis Example 8 and commercially available [4-(trifluoromethoxy)phenyl]boronic acid, 86.8 mg (yield: 58%) of the title compound was obtained as a solid according to the method of Synthesis Example 11.

### Synthesis Example 84

### N-[5-(2,2-Difluoro-1,3-benzodioxol-5-yl)-4-methylthiazol-2-yl]-8-oxo-6,7-dihydroindolizine-5-carboxamide

Using commercially available (2,2-difluoro-1,3-benzodioxol-5-yl)boronic acid, 10.4 mg (yield: 48%) of the title compound was obtained according to the method of Synthesis Example 35.

### Synthesis Example 85

### N-[5-(4-Benzyloxy-3-fluorophenyl)-4-methylthiazol-2-yl]-8-oxo-6,7-dihydro-5H-indolizine-5-carboxamide

Using commercially available (4-benzyloxy-3-fluorophenyl)boronic acid, 10.4 mg (yield: 44%) of the title compound was obtained according to the method of Synthesis Example 35.

### Synthesis Example 86

### N-[5-(4-Isobutoxyphenyl)-4-methylthiazol-2-yl]-8-oxo-6,7-dihydro-5H-indolizine-5-carboxamide

Using commercially available (4-isobutoxyphenyl)boronic acid, 12.2 mg (yield: 58%) of the title compound was obtained according to the method of Synthesis Example 35.

### Synthesis Example 87

### N-[5-(5-Chloro-6-isobutoxy-3-pyridyl)-4-methylthiazol-2-yl]-8-oxo-6,7-dihydro-5H-indolizine-5-carboxamide

Using commercially available (5-chloro-6-isobutoxy-3-pyridyl)boronic acid, 6.6 mg (yield: 29%) of the title compound was obtained according to the method of Synthesis Example 35.

### Synthesis Example 88

### N-[4-Methyl-5-[4-(2,2,2-trifluoroethoxy)phenyl]thiazol-2-yl]-8-oxo-6,7-dihydro-5H-indolizine-5-carboxamide

Using commercially available [4-(2,2,2-trifluoroethoxy)phenyl]boronic acid, 13.2 mg (yield: 59%) of the title compound was obtained according to the method of Synthesis Example 35.

### Synthesis Example 89

### N-[4-Methyl-5-[3-methyl-4-(trifluoromethoxy)phenyl]thiazol-2-yl]-8-oxo-6,7-dihydro-5H-indolizine-5-carboxamide

Using commercially available [3-methyl-4-(trifluoromethoxy)phenyl]boronic acid, 7.5 mg (yield: 33%) of the title compound was obtained according to the method of Synthesis Example 35.

### Synthesis Example 90

### tert-Butyl 4-[4-methyl-2-[(8-oxo-6,7-dihydro-5H-indolizine-5-carbonyl)amino]thiazol-5-yl]-3,6-dihydro-2H-pyridine-1-carboxylate

Using commercially available tert-butyl 4-(4,4,5,5-tetramethyl-1,3,2-dioxaborolan-2-yl)-3,6-dihydro-2H-pyridine-1-carboxylate, 12.8 mg (yield: 56%) of the title compound was obtained according to the method of Synthesis Example 35.

### Synthesis Example 91

### N-[5-[4-(Difluoromethoxy)phenyl]-4-methylthiazol-2-yl]-8-oxo-6,7-dihydro-5H-indolizine-5-carboxamide

Using the N-(6-bromo-1,3-benzothiazol-2-yl)-8-oxo-6,7-dihydro-5H-indolizine-5-carboxamide obtained in Reference Synthesis Example 5 and commercially available [4-(difluoromethoxy)phenyl]boronic acid, 10.9 mg (yield: 52%) of the title compound was obtained according to the method of Synthesis Example 35.

### Synthesis Example 92

### N-[6-[3-Methyl-4-(trifluoromethoxy)phenyl]-1,3-benzothiazol-2-yl]-8-oxo-6,7-dihydro-5H-indolizine-5-carboxamide

Using the N-(6-bromo-1,3-benzothiazol-2-yl)-8-oxo-6,7-dihydro-5H-indolizine-5-carboxamide obtained in Reference Synthesis Example 5 and commercially available [3-methyl-4-(trifluoromethoxy)phenyl]boronic acid, 14.0 mg (yield: 58%) of the title compound was obtained according to the method of Synthesis Example 11.

### Synthesis Example 93

### N-[6-(4-Chlorophenyl)-1,3-benzothiazol-2-yl]-8-oxo-6,7-dihydro-5H-indolizine-5-carboxamide

Using the N-(6-bromo-1,3-benzothiazol-2-yl)-8-oxo-6,7-dihydro-5H-indolizine-5-carboxamide obtained in Reference Synthesis Example 5 and commercially available 4-chlorophenylboronic acid, 2.8 mg (yield: 13%) of the title compound was obtained according to the method of Synthesis Example 11.

### Synthesis Example 94

### N-[6-(4-Fluorophenyl)-1,3-benzothiazol-2-yl]-8-oxo-6,7-dihydro-5H-indolizine-5-carboxamide

Using the N-(6-bromo-1,3-benzothiazol-2-yl)-8-oxo-6,7-dihydro-5H-indolizine-5-carboxamide obtained in Reference Synthesis Example 5 and commercially available 4-fluorophenylboronic acid, 13.0 mg (yield: 64%) of the title compound was obtained according to the method of Synthesis Example 11.

### Synthesis Example 95

### N-[5-(4-Benzoylphenyl)thiazol-2-yl]-8-oxo-6,7-dihydro-5H-indolizine-5-carboxamide

Using commercially available 4-benzoylphenylboronic acid, 75.4 mg (yield: 28%) of the title compound was obtained as a solid according to the method of Synthesis Example 11.

### Synthesis Example 96

### N-[4-Isopropyl-5-[4-(trifluoromethoxy)phenyl]thiazol-2-yl]-8-oxo-6,7-dihydro-5H-indolizine-5-carboxamide

Using the N-(5-bromo-4-isopropylthiazol-2-yl)-8-oxo-6,7-dihydro-5H-indolizine-5-carboxamide obtained in Reference Synthesis Example 24 and commercially available [4-(trifluoromethoxy)phenyl]boronic acid, 112.2 mg (yield: 66%) of the title compound was obtained according to the method of Synthesis Example 35.

### Synthesis Example 97

### N-[5-[1-(2-Methylpropanoyl)-3,6-dihydro-2H-pyridin-4-yl]thiazol-2-yl]-8-oxo-6,7-dihydro-5H-indolizine-5-carboxamide

To a solution of the tert-butyl 4-[2-[(8-oxo-6,7-dihydro-5H-indolizine-5-carbonyl)amino]thiazol-5-yl]-3,6-dihydro-2H-pyridine-1-carboxylate obtained in Synthesis Example 51 (139 mg, 0.315 mmol) in dichloromethane (3 mL) was added trifluoroacetic acid (0.6 mL) at room temperature, followed by stirring for 2 hours. The reaction solution was concentrated under reduced pressure and azeotropically concentrated several times by addition of dichloromethane to obtain an oil material (226 mg). To a solution of the obtained oil material (68 mg) and N,N-diisopropylethylamine (0.10 mL) in dichloromethane (3 mL) was added 2-methylpropanoyl chloride (0.032 mL, 0.30 mmol) under ice cooling, followed by stirring for 2 hours. The reaction solution was diluted with dichloromethane and thereafter washed with water and saturated saline. After the organic layers were dried over anhydrous sodium sulfate, the solvent was distilled off under reduced pressure. The residue was purified by silica gel chromatography (n-hexane/ethyl acetate = 4/1-1/2) to obtain 50.3 mg (yield: 81%) of the title compound as a solid.

### Synthesis Example 98

### N-[5-[1-(Isopropylcarbamoyl)-3,6-dihydro-2H-pyridin-4-yl]thiazol-2-yl]-8-oxo-6,7-dihydro-5H-indolizine-5-carboxamide

Using the tert-butyl 4-[2-[(8-oxo-6,7-dihydro-5H-indolizine-5-carbonyl)amino]thiazol-5-yl]-3,6-dihydro-2H-pyridine-1-carboxylate obtained in Synthesis Example 51 and 2-isocyanatopropane, 30.6 mg (yield: 48%) of the title compound was obtained as a solid according to the method of Synthesis Example 97.

### Synthesis Example 99

### Methyl 2-[(8-oxo-6,7-dihydro-5H-indolizine-5-carbonyl)amino]-5-[4-(2,2,2-trifluoroethoxy)phenyl]thiazole-4-carboxylate

Using the methyl 5-bromo-2-[(8-oxo-6,7-dihydro-5H-indolizine-5-carbonyl)amino]thiazole-4-carboxylate obtained in Reference Synthesis Example 25 and commercially available [4-(2,2,2-trifluoroethoxy)phenyl]boronic acid, 42.3 mg (yield: 31%) of the title compound was obtained according to the method of Synthesis Example 35.

### Synthesis Example 100

### Methyl 5-(4-tert-butoxyphenyl)-2-[(8-oxo-6,7-dihydro-5H-indolizine-5-carbonyl)amino]thiazole-4-carboxylate

Using the methyl 5-bromo-2-[(8-oxo-6,7-dihydro-5H-indolizine-5-carbonyl)amino]thiazole-4-carboxylate obtained in Reference Synthesis Example 25 and commercially available (4-tert-butoxyphenyl)boronic acid, 45.8 mg (yield: 38%) of the title compound was obtained according to the method of Synthesis Example 35.

### Synthesis Example 101

### (5R)-N-[5-(4-tert-Butoxyphenyl)-4-methylthiazol-2-yl]-8-oxo-6,7-dihydro-5H-indolizine-5-carboxamide and (5S)-N-[5-(4-tert-butoxyphenyl)-4-methylthiazol-2-yl]-8-oxo-6,7-dihydro-5H-indolizine-5-carboxamide

Using the N-[5-(4-tert-butoxyphenyl)-4-methylthiazol-2-yl]-8-oxo-6,7-dihydro-5H-indolizine-5-carboxamide obtained in Synthesis Example 35 and YMC CHIRAL ART Cellulose-SC (10 µm) 250 x 10 mm I.D., flow rate: 2.3 ml/min, solvent: n-hexane/ethanol = 40/60), optical resolution was performed. After the first peak eluted earlier was collected, the solvent was distilled off under reduced pressure, to obtain the title compound (53 mg, optical purity: > 99%ee) as an amorphous material. Further, after the second peak eluted later was collected, the solvent was distilled off under reduced pressure, to obtain the title compound (53 mg, optical purity: > 99%ee) as an amorphous material.

Tables below show the structural formulae and physicochemical data of the compounds synthesized in the Reference Synthesis Examples and Synthesis Examples.

**[Table 1-1]**

| Reference Synthesis Example No. | Structural formula | Physicochemical data |
|---|---|---|
| 1 | 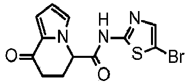 | ¹H-NMR (DMSO-D₆) δ: 12.95 (1H, br s), 7.63 (1H, s), 7.21 (1H, t, *J* = 2.1 Hz), 6.85 (1H, dd, *J* = 3.9, 1.5 Hz), 6.27 (1H, dd, *J* = 3.9, 2.1 Hz), 5.35 (1H, dd, *J* = 5.4, 3.6 Hz), 2.60-2.46 (2TT, m), 2.43-2.38 (2H, m); [M+H]+ = 340. |
| 2 | 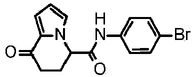 | ¹H-NMR (DMSO-D₆) δ: 10.57 (1H, s), 7.58 (2H, d, *J* = 9.1 Hz), 7.52 (2H, d, *J* = 9.1 Hz), 7.18 (1H, t, *J* = 2.1 Hz), 6.83 (1H, dd, *J* = 3.9, 1.5 Hz), 6.26 (1H, dd, *J* = 3.9, 2.1 Hz), 5.18 (1H, t, *J* = 3.9 Hz), 2.58-2.41 (4H, m); [M+H]+ = 333. |
| 3 | 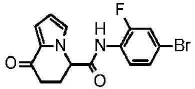 | ¹H-NMR (DMSO-D₆) δ: 10.34 (1H, s), 7.85 (1H, t, *J* = 8.5 Hz), 7.65 (1H, dd, *J* = 10.6, 2.1 Hz), 7.40 (1H, dd, *J* = 8.5, 2.1 Hz), 7.18 (1H, t, *J* = 1.8 Hz), 6.83 (1H, dd, *J* = 3.6, 1.2 Hz), 6.26 (1H, dd, *J* = 3.6, 1.8 Hz), 5.34 (1H, t, *J* = 3.6 Hz), 2.50-2.45 (4H, m); [M+H]+ = 351 |
| 4 | 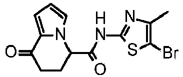 | ¹H-NMR (DMSO-D₆) δ: 12.88 (1H, br s), 7.20 (1H, t, *J* = 2.1 Hz), 6.85 (1H, dd, *J* = 3.9, 1.5 Hz), 6.27 (1H, dd, *J* = 3.9, 2.1 Hz), 5.33 (1H, t, *J* = 4.2 Hz), 2.61-2.44 (2H, m), 2.42-2.32 (2H, m), 2.25 (3H, s); [M+H]+ = 354. |
| 5 | 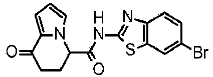 | ¹H-NMR (DMSO-D₆) δ: 13.02 (1H, br s), 8.29 (1H, d, *J* = 1.8 Hz), 7.73 (1H, d, *J* = 8.5 Hz), 7.60 (1H, dd, *J* = 8.5, 1.8 Hz), 7.26 (1H, t, *J* = 2.1 Hz), 6.87 (1H, dd, *J* = 3.9, 1.5 Hz), 6.29 (1H, dd, *J* = 3.9, 2.1 Hz), 5.41 (1H, t, *J* = 3.9 Hz), 2.62-2.55 (2H, m), 2.45-2.40 (2H, m); [M+H]+ = 390. |
| 6 | 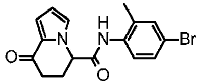 | ¹H-NMR (DMSO-D₆) δ: 9.79 (1H, s), 7.47 (1H, s), 7.40-7.35 (2H, m), 7.19 (1H, t, *J* = 1.5 Hz), 6.83 (1H, dd, *J* = 3.9, 1.5 Hz), 6.27 (1H, dd, *J* = 3.9, 2.7 Hz), 5.27 (1H, t, *J* = 4.2 Hz), 2.56-2.44 (4H, m), 2.22 (3H, s); [M+H]+ = 347. |
| 7 | 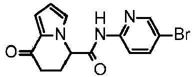 | ¹H-NMR (DMSO-D₆) δ: 11.19 (1H, s), 8.50 (1H, s), 8.05-8.00 (2H, m), 7.20 (1H, t, *J* = 1.8 Hz), 6.83 (1H, dd, *J* = 3.9, 1.2 Hz), 6.26 (1H, dd, *J* = 3.9, 1.8 Hz), 5.32 (1 H, br s), 2.50-2.39 (4H, m); [M+H]+ = 334. |
| 8 | 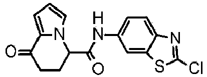 | ¹H-NMR (DMSO-D₆) δ: 10.76 (1H, s), 8.51 (1H, d, *J* = 1.8 Hz), 7.93 (1H, d, *J* = 8.8 Hz), 7.62 (1H, dd, *J* = 8.8, 1.8 Hz), 7.20 (1H, d, *J* = 1.8 Hz), 6.85 (1H, dd, *J* = 3.9, 1.5 Hz), 6.27 (1H, dd, *J* = 3.9, 1.8 Hz), 5.24 (1H, t, *J* = 3.9 Hz), 2.61-2.43 (4H, m); [M+H]+ = 346. |
| 9 | 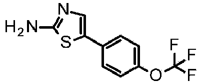 | ¹H-NMR (CDCl₃) δ: 7.43-7.42 (2H, m), 7.29 (1H, s), 7.19 (2H, d, *J* = 7.9 Hz), 5.00 (2H, br s); [M+H]+ = 261. |
| 10 | 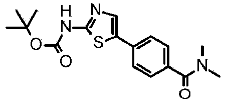 | ¹H-NMR (CDCl₃) δ: 11.38 (1H, br s), 7.62 (1H, s), 7.55-7.53 (211, m), 7.46-7.44 (2H, m), 3.12 (3H, br s), 3.04 (3H, br s), 1.62 (911, s); [M+H]+ = 348. |

**[Table 1-2]**

| | | |
|---|---|---|
| 11 | 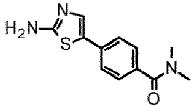 | ¹H-NMR (CDCl₃) δ: 7.45-7.40 (4H, m), 7.36 (1H, s), 5.01 (2H, br s), 3.12 (3H, br s), 3.02 (3H, br s). |
| 12 | 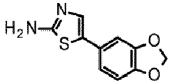 | ¹H-NMR (CDCl₃) δ: 7.16 (1H, s), 6.92 (1H, d, *J* = 2.0 Hz), 6.87 (1H, dd, *J* = 7.8, 2.0 Hz), 6.79 (1H, d, *J* = 7.8 Hz), 5.98 (2H, s), 4.90 (2H, br s); [M+H]+ = 221. |
| 13 | 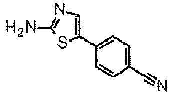 | ¹H-NMR (CDCl₃) δ: 7.61 (2H, dt, *J* = 8.5, 1.8 Hz), 7.48 (2H, dt, *J* = 8.5, 1.8 Hz), 7.45 (1H, s), 5.10 (2H, br s); [M+H]+ = 202. |
| 14 | 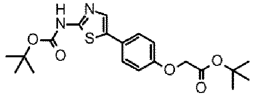 | ¹H-NMR (CDCl₃) δ: 10.63 (1H, br s), 7.45 (1H, s), 7.43 (2H, d, *J* = 8.2 Hz), 6.91 (2H, d, *J* = 8.2 Hz), 4.54 (2H, s), 1.60 (9H, s), 1.50 (9H, s); [M+H]+ = 407. |
| 15 | 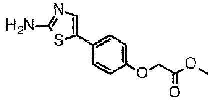 | ¹H-NMR (CDCl₃) δ: 7.35 (2H, td, *J* = 6.0, 3.4 Hz), 7.19 (1H, s), 6.89 (2H, td, *J* = 6.0, 3.4 Hz), 4.86 (2H, br s), 4.65 (2H, s), 3.82 (3H, s); [M+H]+ = 265. |
| 16 | 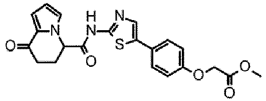 | ¹H-NMR (CDCl₃) δ: 8.75 (1H, br s), 7.76-7.44 (3H, m), 7.19 (1H, dd, *J* = 3.9, 1.5 Hz), 6.99 (1H, dd, *J* = 2.4, 1.5 Hz), 6.93 (2H, dt, *J* = 9.0, 2.4 Hz), 6.49 (1H, dd, *J* = 3.9, 2.4 Hz), 5.14 (1H, dd, *J* = 5.1, 3.2 Hz), 4.67 (2H, s), 3.82 (3H, s), 2.90-2.85 (1H, m), 2.69-2.56 (3H, m); [M+H]+ = 426. |
| 17 | 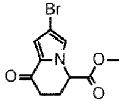 | ¹H-NMR (CDCl₃) δ: 7.03 (1H, d, *J* = 1.8 Hz), 6.88 (1H, d, *J* = 1.8 Hz), 4.90-4.89 (1H, m), 3.81 (3H, s), 2.62-2.55 (4H, m); [M+H]+ = 272. |
| 18 | 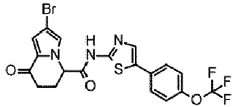 | ¹H-NMR (DMSO-D₆) δ: 12.86 (1H, br s), 8.01-7.97 (1H, m), 7.75-7.71 (2H, m), 7.43-7.41 (3H, m), 6.91 (1H, d, *J* = 1.8 Hz), 5.36 (1H, t, *J* = 4.2 Hz), 2.65-2.50 (2H, m). 2.46-2.33 (2H, m); [M+H]+ = 500, 502. |
| 19 | 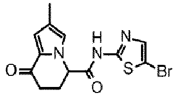 | ¹H-NMR (CDCl₃) δ: 8.40 (1H, br s), 7.35 (1H, s), 6.98 (1H, s), 6.75 (1H, s), 5.02 (1H, dd, *J* = 5.1, 2.7 Hz), 2.84-2.82 (1H, m), 2.65-2.54 (2H, m), 2.49-2,41 (1H, m), 2.17 (3H, s); [M+H]+ = 355. |

**[Table 1-3]**

| | | |
|---|---|---|
| 20 | 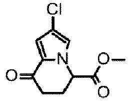 | ¹H-NMR (CDCl₃) δ: 6.94 (111, d, *J* = 1.8 Hz), 6.83 (1H, d, *J* = 1.8 Hz), 4.87 (1H, dd, *J* = 4.2, 2.4 Hz), 3.81 (3H, s), 2.65-2.53 (4H, m); [M+H]+ = 228. |
| 21 | 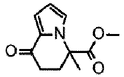 | 1295¹H-NMR (CDCl₃) δ: 7.09 (1H, dd, J = 3.9, 1.5 Hz), 7.04 (1H, t, J = 2.1 Hz), 6.35 (1H, dd, J = 3.9, 2.1 Hz), 3.73 (3H, s), 2.71-2.62 (1H, m), 2.60-2.48 (2H, m), 2.35-2.27 (1H, m), 1.89 (3H, s); [M+H]+ = 208. |
| 22 | 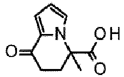 | ¹H-NMR (CDCl₃) δ: 7.10 (1H, d, J = 3.6 Hz), 7.06 (1H, d, *J* = 1.2 Hz), 6.36 (1H, t, J = 3.6 Hz), 2.72-2.60 (2H, m), 2.38-2.30 (2H, m), 1.93 (3H, s). |
| 23 | 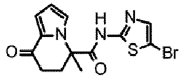 | ¹H-NMR (CDCl₃) δ: 8.01 (1H, br s), 7.31 (1H, s), 7.21 (1H, dd, *J* = 3.9, 1.5 Hz), 7.12 (1H, dd, *J* = 2.7, 1.5 Hz), 6.53 (1H, dd, *J* = 3.9, 2.7 Hz), 2.89-2.84 (1H, m), 2.65 (1H, dt, *J* = 17.3, 3.2 Hz), 2.51-2.33 (2H, m), 1.98 (3H, s): [M+H]+ = 355. |
| 24 | 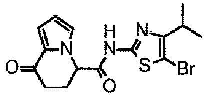 | ¹H-NMR (CDCl₃) δ: 8.36 (1H, br s), 7.21 (1H, dd, *J* = 4.2, 1.5 Hz), 6.99 (1H, dd, *J* = 2.4, 1.5 Hz), 6.52 (1H, dd, *J* = 4.2, 2.4 Hz), 5.11 (1H, dd, *J* = 4.8, 2.4 Hz), 3.18-3.08 (1H, m), 2.89-2.83 (1H, m), 2.69-2.48 (3H, m), 1.15 (6H, d, *J* = 7.3 Hz); [M+H]+ = 382. |
| 25 | 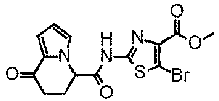 | ¹H-NMR (CDCl₃) δ: 7.17 (1H, dd, *J* = 4.2, 1.8 Hz), 6.95 (1H, t, *J* = 2.1 Hz), 6.48 (1H, dd, *J* = 4.2, 2.4 Hz), 5.15 (1H, dd, *J* = 5.1, 2.7 Hz), 3.92 (3H, s), 2.88-2.81 (1H, m), 2.68-2.57 (2H, m), 2.53-2.47 (1H, m); [M+H]+ = 398. |

**[Table 2-1]**

| Synthesis Example No. | Structural formula | Physicochemical data |
|---|---|---|
| 1 | 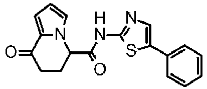 | ¹H-NMR (CDCl₃) δ: 8.96 (1H, br s), 7.57 (1H, s), 7.55-7.52 (2H, m), 7.41-7.38 (2H, m), 7.33-7.32 (1H, m), 7.20 (1H, dd, J = 3.9, 1.6 Hz), 7.00 (1H, dd, J = 2.7, 1.6 Hz), 6.50 (1H, dd, J = 3.9, 2.7 Hz), 5.16 (1H, dd, J = 4.9, 3.3 Hz), 2.89-2.87 (1H, m), 2.71-2.53 (311, m); [M+II]+ = 338. |
| 2 | 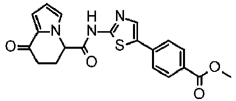 | ¹H-NMR (DMSO-D₆) δ: 12.89 (1H, br s), 8.13 (1H, s), 7.97 (2H, d, J = 8.5 Hz), 7.76 (2H, d, J = 7.9 Hz), 7.24 (1H, t, J = 1.8 Hz), 6.86 (1H, dd, J = 4.3, 1.2 Hz), 6.28 (1H, dd, J = 3.7, 1.8 Hz), 5.38 (1H, t, J = 4.3 Hz), 3.85 (3H, s), 2.64-2.52 (2H, m), 2.45-2.42 (2H, m); └M+II┘+ = 396. |
| 3 | 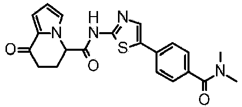 | ¹H-NMR (CDCI₃) δ: 8.60 (1H, br s), 7.63 (1H, s), 7.56 (2H, dt, J = 8.5, 1.8 Hz), 7.46 (2H, dt, J = 8.5, 1.8 Hz), 7.20 (1H, dd, J = 3.9, 1.6 Hz), 7.00 (1H, dd, J = 2.5, 1.6 Hz), 6.51 (1H, dd, J = 3.9, 2.5 Hz), 5.16 (1H, dd, J = 4.9, 2.9 Hz), 3.13 (3H, br s), 3.02 (3H, br s), 2.92-2.87 (1H, m), 2.70-2.54 (3H, m); [M+H]+ = 409. |
| 4 | 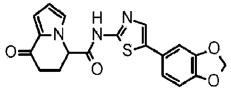 | ¹H-NMR (CDCl₃) δ: 7.44 (1H, s), 7.19 (1H, dd, J = 4.1, 1.4 Hz), 7.01-6.98 (3H, m), 6.83 (1H, dd, J = 5.9, 2.7 Hz), 6.49 (1H, dd, J = 4.1, 2.5 Hz), 6.00 (2H, s), 5.14 (1H, t, J = 4.1 Hz), 2.66-2.60 (4H, m); └M+H┘+ = 382. |
| 5 | 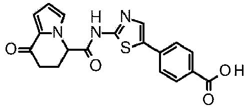 | ¹H-NMR (DMSO-D₆) δ: 12.91 (211, br s), 8.10 (1H, s), 7.95 (2H, d, J = 7.9 Hz), 7.74 (2H, d, J = 7.9 Hz), 7.24 (1H, s), 6.87 (1H, d, J = 2.4 Hz), 6.29 (1H, s), 5.39 (1H, s), 2.58-2.44 (4H, m); [M+H]+ = 382. |
| 6 | 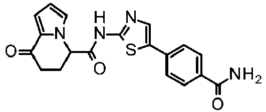 | ¹H-NMR (DMSO-D₆) δ: 12.84 (1H, s), 8.07 (1H, s), 8.00 (1H, s), 7.90 (2H, d, *J* = 7.9 Hz), 7.69 (2H, d, *J* = 7.9 Hz), 7.40 (1H, s), 7.24 (1H, t, *J* = 2.1 Hz), 6.86 (1 H, dd, *J* = 4.0, 1.5 Hz), 6.28 (1H, dd, *J* = 4.0, 2.1 Hz), 5.38 (1H, t, *J* = 4.3 Hz), 2.55-2.42 (411, m); [M+H]+ = 381. |
| 7 | 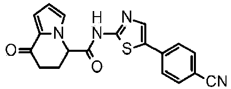 | ¹H-NMR (DMSO-D₆) δ: 12.91 (1H, br s), 8.17 (1H, s), 7.86 (211, d, J = 8.5 Hz), 7.80 (2H, d, J = 8.5 Hz), 7.23 (1H, dd, J = 2.4, 1.5 Hz), 6.86 (1H, dd, J = 4.2, 1.5 Hz), 6.28 (1H, dd, J = 4.2, 2.4 Hz), 5.37 (1H, t, J = 4.2 Hz), 2.61-2.53 (2H, m), 2.45-2.41 (2H, m).; [M+H]+ = 363. |

**[Table 2-2]**

| | | |
|---|---|---|
| 8 | 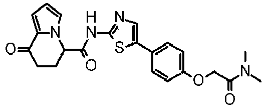 | 'H-NMR (CDCl₃) δ: 8.57 (1H, br s), 7.45 (1H, s), 7.45-7.42 (2H, m), 7.19 (1H, dd, J = 3.9, 1.5 Hz), 6.99 ((III, dd, J = 2.4, 1.5 Hz), 6.97-6.96 (2H, m), 6.50 (1H, dd, J = 3.9, 2.4 Hz), 5.14-5.13 (1H, m), 4.73 (2H, s), 3.10 (3H, s), 2.99 (3H, s), 2.89-2.87 (1H, m), 2.68-2.55 (3H, m); ┌M+H┐+=439. |
| 9 | 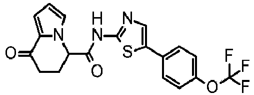 | 'H-NMR (DMSO-D₆) δ: 12.83 (1H, br s), 7.99 (1H, s), 7.73 (2H, d, J = 7.9 Hz), 7.42 (2H, d, J = 7.9 Hz), 7.23 (1H, t, J = 2.1 Hz), 6.86 (111, dd, J = 3.9, 2.1 Hz), 6.28 (1H, dd, J = 3.9, 2.7 Hz), 5.37 (1H, t, J= 4.2 Hz), 2.63-2.50 (2H, m), 2.46-2.41 (2H, m); [M+H]+ = 422. |
| 10a | First peak | HPLC measurement conditions column : YMC CHIRAL ART Cellulose-SB (5um), 250 × 4.6 mm I.D., column temperature: 25°C, flow rate: 0.5 ml/min, mobile phase: n-hexane/ethanol =70/30. detectioin wavelength : 293 nm, retention time : tR = 14.9 min |
| | Optically active form of compound of Synthesis Example 9 | |
| 10b | Second peak | HPLC measurement conditions column : YMC CHIRAL ART Cellulose-SB (5um), 250 × 4.6 mm I.D., column temperature : 25°C, flow rate: 0.5 ml/min, mobile phase: n-hexane/ethanol =70/30, detectioin wavelength : 293 nm, retention time : tR = 21.6min |
| | Optically active form of compound of Synthesis Example 9 | |
| 11 | 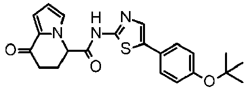 | ¹H-NMR (DMSO-D₆) δ: 12.73 (1H, br s), 7.83 (1H, s), 7.51 (2H, d, J = 8.5 Hz), 7.23 (1H, t, J = 2.1 Hz), 7.01 (2H, d, J = 8.5 Hz), 6.86 (1H, dd, J = 4.2, 1.8 Hz), 6.28 (1H, dd, J = 4.2, 2.1 Hz), 5.36 (III, t, J =4.2 Hz), 2.59-2.50 (211, m), 2.45-2.44 (2H, m), 1.31 (9H, s); [M+H]+ = 410. |
| 12 | 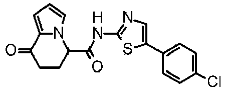 | ¹H-NMR (DMSO-D₆) δ: 12.82 (1H, br s), 7.98 (1H, s), 7.63 (2H, d, J = 8.5 Hz), 7.47 (2H, d, J = 8.5 Hz), 7.23 (1H, s), 6.86 (1H, dd, J =3.9, 1.5 Hz), 6.28 (1H, dd, J = 3.9, 2.1 Hz), 5.37 (1H, t, J = 3.9 Hz), 2.61-2.50 (2H, m), 2.45-2.41 (2H, m); ┌M+H┐+ = 372. |
| 13 | 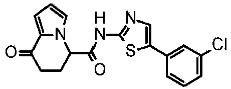 | 'H-NMR (DMSO-D₆) δ: 12.84 (1H, br s), 8.05 (1H, s), 7.73 (1H, s), 7.53 (1H, d, J = 7.9 Hz), 7.44 (1H, t, J = 7.9 Hz), 7.36 (1H, d, J = 7.9 Hz), 7.23 (1H t, J = 2.2 Hz), 6.86 (1H, dd, J = 4.2, 1.2 Hz), 6.28 (1H, dd, J = 4.2, 2.2 Hz), 5.38 (1H, t, J = 4.2 Hz), 2.61-2.50 (2H, m), 2.46-2.41 (2H, m); [M+H]+ = 372. |
| 14 | 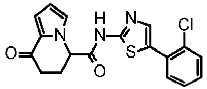 | ¹H-NMR (CDCl₃) δ: 8.95 (1H, br s), 7.62 (1H, s), 7.50-7.48 (2H, m), 7.31-7.29 (2H, m), 7.20 (1H, dd, J - 3.9, 1.5 Hz), 6.99 (1H,dd, J = 2.7, 1.5 Hz), 6.50 (1H, dd, J = 3.9, 2.7 Hz), 5.16 (1H, t, J = 3.9 Hz), 2.91-2.86 (1H, m), 2.71-2.57 (3H, m); ┌M+H┐+ = 372. |

**[Table 2-3]**

| | | |
|---|---|---|
| 15 | 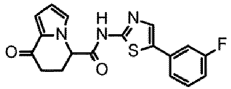 | ¹H-NMR (DMSO-D₆) δ: 12.83 (1H, s), 8.04 (1H, s), 7.54-7.51 (1H, m), 7.48-7.39 (2H, m), 7.23 (1H, t, J = 1.8 Hz), 7.17-7.11 (1H, m), 6.86 (1H, dd, J =3.9, 1.5 Hz), 6.28 (1H, dd, J = 3.9, 1.8 Hz), 5.38 (1H, t, J = 4.2 Hz), 2.62-2.50 (2H, m), 2.45-2.41 (2H, m); [M+H]+ = 356. |
| 16 | 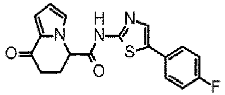 | ¹H-NMR (DMSO-D₆) δ: 12.78 (1H, br s), 7.90 (1H, s), 7.67-7.62 (2H, m), 7.29-7.23 (3H, m), 6.86 (1H, dd, J = 3.9, 1.5 Hz), 6.28 (1H, dd, J = 3.9, 2.7 Hz), 5.37 (1H, t, J = 4.2 Hz), 2.62-2.50 (2H, m), 2.45-2.42 (2H, m); [M+H]+ = 356. |
| 17 | 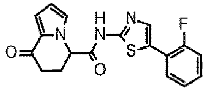 | ¹H-NMR (DMSO-D₆) δ: 12.84 (1H, s), 8.01 (1H,s),7.78 (1H, td, J - 7.9, 1.2 Hz), 7.38-7.26 (3H, m), 7.24 (1H, t, J = 2.1 Hz), 6.86 (1H, dd, J = 3.9, 1.8 Hz), 6.28 (1H, dd, J = 3.9, 2.1 Hz), 5.38 (1H, t, J = 3.9 Hz), 2.58-2.53 (2H, m), 2.45-2.42 (2H, m); [M+H]+ = 356. |
| 18 | 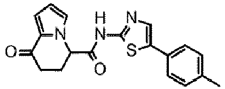 | 'H-NMR (DMSO-D₆) δ: 12.73 (1H, br s), 7.87 (1 H, s), 7.48 (2H, d,*J*= 7.9 Hz), 7.22 (3H, d,*J=* 7.9 Hz), 6.86 (1H, dd,*J*= 3.6, 1.2 Hz), 6.28 (1H, dd, *J* = 3.9, 2.7 Hz), 5.36 (1H, t,*J*= 4.2 Hz), 2.61-2.50 (2H, m), 2.45-2.42 (2H, m), 2.31 (3H, s); [M+H]+ = 352. |
| 19 | 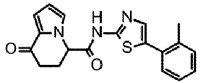 | ¹H-NMR (CDCl₃) δ: 9.38 (1H, br s), 7.36-7.21 (5H, m), 7.19 (1H, dd, J = 3.9, 0.9 Hz), 6.99 (1H, br s), 6.49 (1H, t, J = 3.0 Hz), 5.16 (1H, t, J = 3.9 Hz), 2.89-2.84 (1H, m), 2.71-2.59 (3H, m), 2.41 (3H, s); [M+H]+ = 352. |
| 20 | 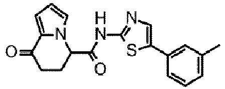 20 | ¹H-NMR (DMSO-D₆) δ: 12.76 (1H, br s), 7.91 (1H, s), 7.43 (1H, br s), 7.39 (1H, d, J = 7.8 Hz), 7.30 (1H, t, J = 7.8 Hz), 7.23 (1H, dd, J = 2.3, 1.6 Hz), 7.12 (1H, d, J = 7.8 Hz), 6.86 (1H, dd, J = 3.9, 1.6 Hz), 6.28 (1H, dd, J = 3.9, 2.3 Hz), 5.37 (1H, t, J = 4.3 Hz), 2.59-2.49 (2H, m), 2.47-2.41 (2H, m), 2.33 (3H, s); [M+H]+ = 352. |
| 21 | 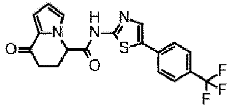 | ¹H-NMR(DMSO-D₆)δ:12.89(1H,br s),8.11 (1H,s),7.82(2H,d, J = 8.4 Hz), 7.75 (2H, d, J = 8.4 Hz), 7.22 (1H, t, J = 2.3 Hz), 6.86 (1H, dd, J = 3.8, 1.5 Hz), 6.28 (1H, dd, J =3.8, 2.3 Hz), 5.36 (1H, t, J = 4.2 Hz), 2.56-2.52 (2H, m), 2.45-2.43 (2H, m); [M+H]+ = 406. |

**[Table 2-4]**

| | | |
|---|---|---|
| 22 | 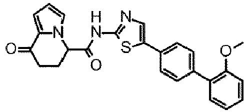 | ¹H-NMR (DMSO-D₆) δ: 12.79 (1H, br s), 7.94 (1H, s), 7.62 (2H, d, J = 8.4 Hz), 7.51 (2H, d, J = 8.4 Hz), 7.37-7.31 (2H, m), 7.22 (1H, t, J = 2.3 Hz), 7.12 (1H, d, J = 7.6 Hz), 7.05-7.02 (1H, m), 6.86 (1H, dd, J =3.8, 1.5 Hz), 6.28 (1H, dd, J = 3.8, 2.3 Hz), 5.34 (1H, t, J =4.2 Hz), 3.78 (3H, s), 2.57-2.50 (2H, m), 2.46-2.44 (2H, m); [M+H]+ = 444. |
| 23 | 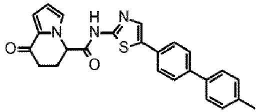 | ¹H-NMR (DMSO-D₆) δ: 12.80 (1H, br s), 7.97 (1H, s), 7.70-7.66 (4H, m), 7.59 (2H, d, J = 8.4 Hz), 7.28 (2H, d, J = 8.4 Hz), 7.23 (1H, t, J = 2.3 Hz), 6.86 (1H, dd, J = 3.8, 1.5 Hz), 6.28 (1H, t, J = 3.8 Hz), 5.36 (1H, t, J = 4.2 Hz), 2.59-2.50 (2H, m), 2.47-2.42 (2H, m), 2.34 (3H, s); [M+H]+ = 428. |
| 24 | 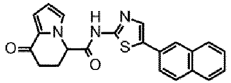 | ¹H-NMR (DMSO-D₆) δ: 12.80 (1H, s), 8.08-8.06 (2H, m), 7.96-7.89 (3H, m), 7.83 (1H, dd, *J* = 8.4, 2.3 Hz), 7.52-7.50 (2H, m), 7.23 (1H, t, *J* = 1.9 Hz), 6.87 (1H, dd, *J* = 3.8, 1.5 Hz), 6.28 (1H, dd, J = 3.8, 1.9 Hz), 5.35 (1H, t, *J*= 4.2 Hz), 2.60-2.45 (4H, m); [M+H]+ = 388. |
| 25 | 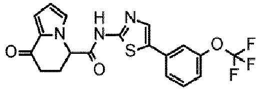 | ¹H-NMR (DMSO-D₆) δ: 12.86 (1H, br s), 8.06 (1H, s), 7.62-7.60 (211, m), 7.54 (1H, t, J = 8.0 Hz), 7.29 (1H, dd, J = 9.2, 1.5 Hz), 7.22 (1H, t, J = 2.3 Hz), 6.86 (1H, dd, J = 3.8, 1.5 Hz), 6.28 (1H, dd, J = 3.8, 2.3 Hz), 5.36 (1H, t, J = 4.2 Hz), 2.58-2.50 (2H, m), 2.45-2.42 (2H, m); [M+H]+ = 422. |
| 26 | 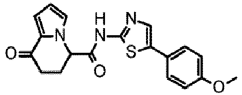 | ¹H-NMR (DMSO-D₆) δ: 12.70 (1H, br s), 7.79 (1H, s), 7.53-7.52 (2H, m), 7.22 (1H, t, J = 2.1 Hz), 7.00-6.96 (2H, m), 6.86 (1H, dd, J = 3.9, 1.5 Hz), 6.28 (1H, dd, J = 3.9, 2.1 Hz), 5.35 (1H, t, J = 4.2 Hz), 3.77 (3H, s), 2.59-2.53 (2H, m), 2.45-241 (2H, m); [M+H]+ = 368. |
| 27 | 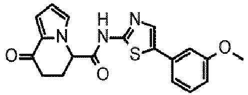 | ¹H-NMR (DMSO-D₆) δ: 12.78 (1H, br s), 7.96 (1H, s), 7.32 (1H, t, J = 8.0 Hz), 7.23 (1H, t, J = 1.9 Hz), 7,17-7.14 (2H, m), 6.89-6.87 (1H, m), 6.86 (1H, dd, J = 3.8, 1.5 Hz), 6.28 (1H, dd, J = 3.8, 2.4 Hz), 5.36 (1H, t, J = 4.2 Hz), 3.80 (3H, s), 2.59-2.50 (2H, m), 2.46-2.42 (2H, m); [M+H]+ = 368. |
| 28 | 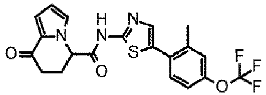 | 'H-NMR (DMSO-D₆) δ: 12.81 (1H, br s), 7.62 (1H, s), 7.49 (1H, d, J = 8.4 Hz), 7.36 (1H, s), 7.25 (1H, d, J = 8.4 Hz), 7.22 (1H, t, J = 1.9 Hz), 6.85 (1H, dd, J = 3.8, 1.5 Hz), 6.27 (1H, dd, J =3.8, 1.9 Hz), 5.36 (1H, t, J = 4.6 Hz), 2.58-2,50 (2H, m), 2.45-2.43 (2H, m), 2.41 (3H, s); [M+H]+ = 436. |

**[Table 2-5]**

| | | |
|---|---|---|
| 29 | 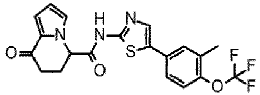 | ¹H-NMR (DMSO-D₆) δ: 12.81 (1H, br s), 7.95 (1H, s), 7.66 (1H, d, J = 1.8 Hz), 7.54 (1H, dd, J =8.5, 1.8 Hz), 7.36-7.34 (1H, m), 7.23 (1H, t, J = 2.7 Hz), 6.86 (1H, dd, J = 4.0, 2.0 Hz), 6.28 (1H, dd, J = 4.0, 2.7 Hz), 5.36 (1H, t, J = 4.3 Hz), 2.61-2.48 (2H, m), 2.47-2.42 (2H, m), 2.30 (3H, s); [M+H]+ = 436. |
| 30 | 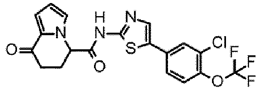 | ¹H-NMR (DMSO-D₆) δ: 12.88 (1H, br s), 8.07 (1H, s), 7.99 (1H, d, J = 2.3 Hz), 7.64 (1H, dd, J = 8.4, 2.3 Hz), 7.59 (1H, dd, J = 8.4, 1.5 Hz), 7.22 (1H, t, J = 2.3 Hz), 6.86 (1H, dd, J = 3.8, 1.5 Hz), 6.28 (1H, dd, J= 3.8, 2.3 Hz), 5.35 (1H, t, J = 4.2 Hz), 2.59-2.50 (2H, m), 2.46-2.42 (2H, m); [M+H]+ = 456. |
| 31 | 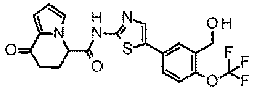 | ¹H-NMR (DMSO-D₆) δ: 12.83 (1H, br s), 7,94 (1H, s), 7.75 (1H, d, J = 2.3 Hz), 7.64 (1H, dd, J = 8.4, 2.3 Hz), 7.36 (1H, dd, J = 8.4, 1.5 Hz), 7.23 (1H, t, J = 2.3 Hz), 6.86 (1H, dd, J = 4.2, 1.5 Hz), 6.28 (1H, dd, J = 4.2, 2.3 Hz), 5.46 (1H, t, J = 5.4 Hz), 5.36 (1H, t, J = 4.2 Hz), 4.58 (2H, d, J = 5.4 Hz), 2.59-2.50 (2H, m), 2.46-2.43 (2H, m); [M+H]+ = 452. |
| 32 | 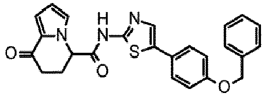 | ¹H-NMR (DMSO-D₆) δ: 12.71 (1H, br s), 7.78 (1H, s), 7.52 (2H, td, J = 6.1, 3.6 Hz), 7.45 (2H, d, J = 7.3 Hz), 7.39 (2H, t, J = 7.3 Hz), 7.33 (1H, tt, J = 7.3, 1.8 Hz), 7.22 (1H, t, J = 2.3 Hz), 7.06 (2H, td, J = 6.1, 3.6 Hz), 6.86 (1H, dd, J = 3.8, 1.5 Hz), 6.28 (1H, dd, J = 3.8, 2.3 Hz), 5.34 (1H, t, J = 4.2 Hz), 5.14 (2H, s), 2.59-2.50 (2H, m), 2.44-2.43 (2H, m); [M+H]+ = 444. |
| 33 | 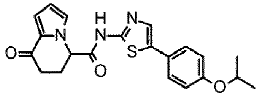 | ¹H-NMR (DMSO-D₆) δ: 12.70 (1H, br s), 7.78 (1H, s), 7.49 (2H, d, J = 8.0 Hz), 7.23 (1H, s), 6.95 (2H, d, J = 8.0 Hz), 6.96 (1H, d, J = 3.0 Hz), 6.28 (1H, s), 5.36 (1H, s), 4.66-4.60 (1H, m), 2.49-2.46 (4H, m), 1.26 (6H, d, J = 6.0 Hz); [M+H]+ = 396. |
| 34 | 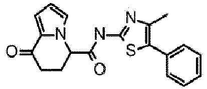 | ¹H-NMR (DMSO-D₆) δ: 12.72 (1H, s), 7.45-7.45 (4H, m), 7.38-7.33 (1H, m), 7.22 (1H, t, J = 1.5 Hz), 6.85 (1H, dd, J = 4.0, 1.5 Hz), 6.28 (1H, dd, J = 4.0, 2.4 Hz), 5.34 (1H, t, J = 4.0 Hz), 2.56-2.52 (2H, m), 2.44-2.41 (2H, m), 2.37 (3H, s); [M+H]+ - 352. |
| 35 | 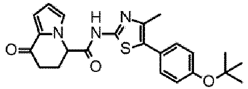 | ¹H-NMR (DMSO-D₆) δ: 12.67 (1H, br s), 7.35 (2H, d, *J* = 8.5 Hz), 7.21 (1H, br s), 7.04 (2H, d, *J* = 8.5 Hz), 6.85 (1H, br s), 6.27 (1H, d, *J* = 3.6 Hz), 5.33 (1H, br s), 2.50-2.43 (4H, br m). 2.35 (3H, s), 1.32 (9H, s); [M+H]+ = 424. |

**[Table 2-6]**

| | | |
|---|---|---|
| 36 | 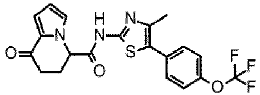 | ¹H-NMR (DMSO-D₆) δ: 12.77 (1H, br s), 7.58 (2H, dt, *J* = 8.5, 1.8 Hz), 7.45 (2H, d, *J* = 8.5 Hz), 7.21 (1H, t, *J* = 1.8 Hz), 6.85 (1H, dd, *J* = 3.9, 1.8 Hz), 6.27 (1H, dd, *J* = 3.9, 2.4 Hz), 5.35 (1H, t, *J* = 3.9 Hz), 2.58-2.48 (2H, m), 2.45-2.41 (2H, m), 2.38 (3H, s); [M+H]+ = 436. |
| 37 | 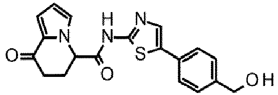 | 1H-NMR (DMSO-D₆) δ: 12.74 (1H, br s), 7.88 (1H, s), 7.55 (2H, t, J = 4.2 Hz), 7.34 (2H, d, J = 8.4 Hz), 7.22 (1H, t, J = 1.9 Hz), 6.85 (1H, dd, J = 3.8, 1.5 Hz), 6.27 (1H, dd, J = 3.8, 1.9 Hz), 5.34 (1H, t, J = 4.6 Hz), 5.21 (1H, t, J = 5.4 Hz), 4.49 (2H, d, J = 4.6 Hz), 2.56-2.49 (2H, m), 2.45-2.43 (2H, m); [M+H]+ = 368. |
| 38 | 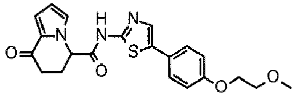 | ¹H-NMR (DMSO-D₆) δ: 7.74 (1H, s), 7.49 (2H, dt, J = 8.4, 1.5 Hz), 7.20 (1H, t, J = 2.3 Hz), 6.98 (2H, dt, J = 8.4, 1.5 Hz), 6.84 (1H, dd, J = 3.8, 1.5 Hz), 6.26 (1H, dd, J = 3.8, 2.3 Hz), 5.29 (1H, t, J = 4.2 Hz), 4.11-4.09 (211, m), 3.66-3.65 (2H, m), 3.30 (3H, s), 2.54-2.49 (2H, m), 2.45-2.43 (2H, m); [M+H]+ = 412. |
| 39 | 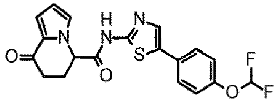 | ¹H-NMR (DMSO-D₆) δ: 12.79 (1H, br s), 7.91 (1H, s), 7.65 (2H, dd, J = 8.4, 2.3 Hz), 7.26 (1H, t, J = 74.2 Hz), 7.23-7.21 (3H, m), 6.86 (1H, dd, J = 3.8, 1.5 Hz), 6.28 (1H, dd, J = 3.8, 2.3 Hz), 5.35 (1H, t, J =4.6 Hz), 2.58-2.49 (2H, m), 2.45-2.42 (2H, m); [M+H]+ = 404. |
| 40 | 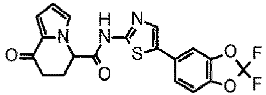 | ¹H-NMR (DMSO-D₆) δ: 12.82 (1H, br s), 7.94 (1H, s), 7.77 (1H, d, J = 1.5 Hz), 7.45 (1H, d, J = 8.4 Hz), 7.39 (1H, dd, J = 8.4, 1.5 Hz), 7.22 (1H, t, J = 2.7 Hz), 6.86 (1H, dd, J = 4.2, 1.5 Hz), 6.28 (1H, dd, J = 4.2, 2.7 Hz), 5.36 (1H, t, J = 4.2 Hz), 2.60-2.50 (2H, m), 2.45-2.42 (2H, m); [M+H]+ = 418. |
| 41 | 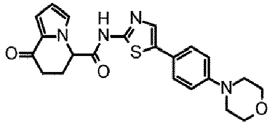 | ¹H-NMR (DMSO-D₆) δ: 12.67 (1H, br s), 7.75 (1H, s), 7.45 (2H, d, J = 9.1 Hz), 7.22 (1H, t, J = 2.1 Hz), 6.98 (2H, d, J = 9.1 Hz), 6.86 (1H, dd, J = 3.9, 1.5 Hz), 6.28 (1H, dd, J = 3.9, 2.1 Hz), 5.35 (1H, t, J = 4.2 Hz), 3.73 (4H, t, J = 4.8 Hz), 3.13 (4H, t, J = 4.8 Hz), 2.60-2.50 (2H, m), 2.46-2.41 (2H, m); [M+H]+ = 423. |
| 42 | 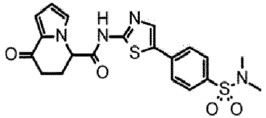 | ¹H-NMR (DMSO-D₆) δ: 12.91 (1H, br s), 8.16 (1H, s), 7.87 (2H, dd, J = 8.4, 2.3 Hz), 7.76 (2H, d, J = 8.4 Hz), 7.24 (1H, t, J = 1.9 Hz), 6.87 (1H, dd, J = 4.2, 1.5 Hz), 6.29 (1H, dd, J = 4.2, 1.9 Hz), 5.39 (1H, t, J = 4.2 Hz), 2.63 (6H, s), 2.61-2.54 (2H, m), 2.47-2.42 (2H, m); [M+H]+ = 445. |

**[Table 2-7]**

| | | |
|---|---|---|
| 43 | 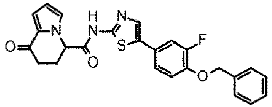 | ¹H-NMR (DMSO-D₆) δ: 12.76 (1H, br s), 7.88 (1H, s), 7.59-7.56 (1H, m), 7.46 (2H, d, J = 7.9 Hz), 7.41 (2H, t, J = 7.9 Hz), 7.37-7.26 (3H, m), 7.22 (1H, t, J = 1.8 Hz), 6.86 (1H, dd, J = 4.2, 1.8 Hz), 6.28 (1H, dd, J = 4.2, 2.4 Hz), 5.36 (1H, t, J = 3.9 Hz), 5.21 (2H, s), 2.59-2.50 (2H, m), 2.45-2.41 (2H, m); [M+H]+ = 462. |
| 44 | 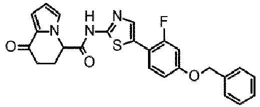 | ¹H-NMR (DMSO-D₆) δ: 12.76 (1H, br s), 7.85 (1H, s), 7.66 (1H, t, J = 8.5 Hz), 7.47-7.32 (5H, m), 7.22 (1H, t, J = 1.8 Hz), 7.07 (1H, dd, J = 12.8, 2.4 Hz), 6.94 (1H, dd, J = 8.5, 2.4 Hz), 6.86 (1H, dd, J = 4.3, 1.5 Hz), 6.28 (1H, dd, J = 4.3, 1.8 Hz), 5.37 (1H, t, J = 4.3 Hz), 5.16 (2H, s), 2.60-2.52 (2H, m), 2.46-2.41 (2H, m); [M+H]+ = 462. |
| 45 | 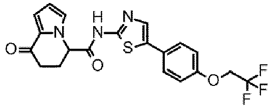 | ¹H-NMR (DMSO-D₆) δ: 12.73 (1H, br s), 7.85 (1H, s), 7.57 (2H, d, J = 8.5 Hz), 7.23 (1H, t, J = 2.1 Hz), 7.11 (2H, d, J = 8.5 Hz), 6.86 (1H, dd, J = 3.6, 1.2 Hz), 6.28 (1H, dd, J = 3.6, 2.1 Hz), 5.36 (1H, t, J = 3.9 Hz), 4.80 (2H, q, J = 8.9 Hz), 2.59-2.53 (2H, m), 2.45-2.42 (2H, m); [M+H]+ = 436. |
| 46 | 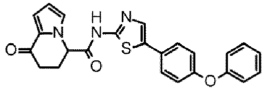 | ¹H-NMR (DMSO-D₆) δ: 12.76 (1H, br s), 7.86 (1H, s), 7.61 (2H, d, J = 8.5 Hz), 7.41 (2H, t, J = 79 Hz), 7.23 (1H, t, J = 2.1 Hz), 7.17 (1H, t, J = 7.9 Hz), 7.07-7.03 (4H, m), 6.86 (1H, dd, J = 4.2, 1.5 Hz), 6.28 (1H, dd, J = 4.2, 2.1 Hz), 5.36 (1H, t, J = 4.2 Hz), 2.59-2.50 (2H, m), 2.45-2.42 (2H, m); [M-H]- = 428. |
| 47 | 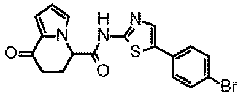 | ¹H-NMR (DMSO-D₆) δ: 12.82 (1H, br s), 7.99 (1H, s), 7.61 (2H, d, J = 8.5 Hz), 7.56 (211, d, J = 8.5 Hz), 7.23 (1H, t, J = 2.1 Hz), 6.86 (1H, dd, J = 3.9, 1.5 Hz), 6.28 (1H, dd, J= 3.9, 2.1 Hz), 5.37 (1H, t, J = 3.9 Hz), 2.60-2.50 (2H, m), 2.45-2.41 (2H, m): [M+H]+ = 416. |
| 48 | 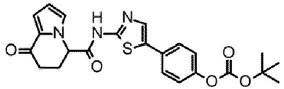 | ¹H-NMR (DMSO-D₆) δ: 7.89 (1H, s), 7.64-7.60 (2H, m), 7.25-7.20 (3H, m), 6.85 (1H, dd, *J* = 4.0, 1.5 Hz), 6.27 (1H, dd, *J* = 4.0, 2.4 Hz), 5.31 (1H, t, J = 4.3 Hz), 2.55-2.50 (2H, m), 2.46-2.42 (2H, m). 1.49 (9H, s); [M+H]+ = 454. |
| 49 | 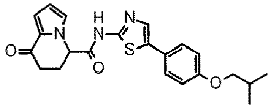 | ¹H-NMR (DMSO-D₆) δ: 12.69 (1H, br s), 7.77 (1H, s), 7.52-7.48 (2H, m), 7.22 (1H, dd, J = 2.4, 1.5 Hz), 6.99-6.95 (2H, m), 6.85 (1H, dd, J = 4.0, 1.5 Hz), 6.27 (1H, dd, J = 4.0, 2.4 Hz), 5.34 (1H, t, J = 4.0 Hz), 3.76 (2H, d, J = 6.7 Hz), 2.58-2.50 (2H, m), 2.45-2.42 (2H, m), 2.06-1.96 (1H, m), 0.97 (6H, d, J = 6.7 Hz), [M+H]+ = 410. |

**[Table 2-8]**

| | | |
|---|---|---|
| 50 | 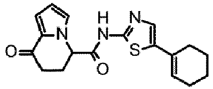 | ¹H-NMR (CDCl₃) δ: 8.77 (1H, s), 7.18-7.17 (2H, m), 6.97 (1H, dd, J = 2.7, 1.5 Hz), 6.48 (1H, dd, J = 3.9, 2.7 Hz), 6.09 (1H, t, J = 3.9 Hz), 5.11 (1H, t, J=4.2 Hz), 2.88-2.83 (1H, m), 2.67-2.53 (3H, m), 2.38-2.32 (2H, m), 2.21-2.16 (2H, m), 1.79-1.73 (2H, m), 1.68-1.62 (2H, m); [M+H]+ = 342. |
| 51 | 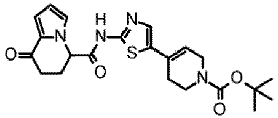 | ¹H-NMR (DMSO-D₆) δ: 12.67 (1H, br s), 7.45 (1H s), 7.20 (1H, dd, J = 2.4, 1.2 Hz), 6.84 (1H, dd, J = 4.0, 1.5 Hz), 6.26 (1H, dd, J = 4.0, 2.4 Hz), 5.92 (1H, br s), 5.30 (1H, t, J = 4.0 Hz), 3.95 (2H, br s), 3.51 (2H, t, J = 5.5 Hz), 2.58-2.49 (2H, m), 2.46-2.39 (4H, m), 1.41 (9H, s); [M-H]- = 441. |
| 52 | 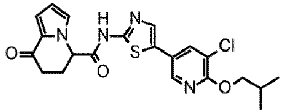 | ¹H-NMR (DMSO-D₆) δ: 12.83 (1H, br s), 8.29 (1H, d, J = 2.4 Hz), 8.22 (1H, d, J = 2.4 Hz), 7.95 (1H, s), 7.21 (1H, t, J = 1.8 Hz), 6.85 (1H, dd, J =4.0, 1.5 Hz), 6.27 (1H, dd, J=4.0. 1.8 Hz), 5.34 (1H, t, J = 4.3 Hz). 4.14 (211, d, J = 6.7 Hz), 2.58-2.53 (2H, m), 2.45-2.41 (2H, m), 2.11-2.01 (1H, m), 0.98 (6H, d, J = 6.7 Hz); [M+H]+=445 . |
| 53 | 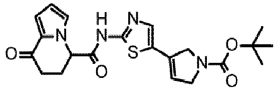 | ¹H-NMR (DMSO-D₆) δ: 12.79 (1H, br s), 7.48 (1H, s), 7.21 (1H, s), 6.85 (1H, dd, J = 4.0, 1.5 Hz), 6.27 (1H, dd, J = 4.0, 2.4 Hz), 5.98 (1H, d, J = 17.1 Hz), 5.33 (1H, t, J = 3.7 Hz), 4.38-4.33 (2H, br m), 4.17-4.12 (2H, br m), 2.57-2.50 (2H, m), 2.43-2.39 (2H, m), 1.44 (9H, d, J = 6.1 Hz); [M+H]+ = 429. |
| 54 | 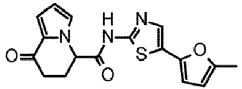 | ¹H-NMR (DMSO-D₆) δ: 12.78 (1H, br s), 7.67 (1H, s), 7.22 (1H, dd, J = 2.4,1.2 Hz), 6.86 (1H, dd, J = 4,0,1.2 Hz), 6.58 (1H, d, J = 3.1 Hz), 6.28 (1H, dd, J = 4.0, 2.4 Hz), 6.18 (1H, dd, J = 3.1, 1.2 Hz), 5.35 (1H, t, J = 4.3 Hz), 2.62-2.50 (2H, m), 2.46-2.41 (2H, m), 2.30 (3H, s); [M+H]+ = 342. |
| 55 | 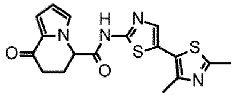 55 | ¹H-NMR (DMSO-D₆) δ: 12.86 (1H, br s), 7.59 (1H, s), 7.20 (1H, dd, J = 2.4, 1.2 Hz), 6.85 (1H, dd, J = 4.0, 1.2 Hz), 6.27 (1H, dd, J = 4.0, 2.4 Hz), 5.33 (1H, t, J = 4.3 Hz), 2.60 (3H, s), 2.56-2.50 (2H, m), 2.45-2.41 (2H, m), 2.40 (3H, s): [M+H]+ = 373. |
| 56 | 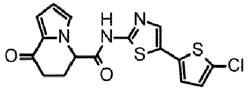 | ¹H-NMR (DMSO-D₆) δ: 7.69 (1H, s), 7.19 (1H, t, J = 1.5 Hz), 7.10 (2H, d, J = 4.0 Hz), 6.84 (1H, dd, J = 4.0, 1.5 Hz), 6.26 (1H, dd, J = 4.3, 2.4 Hz), 5.29 (1H, t, J = 4.0 Hz), 2.55-2.50 (2H, m), 2.44-2.40 (2H, m): [M+H]+ = 378. |

**[Table 2-9]**

| | | |
|---|---|---|
| 57 | 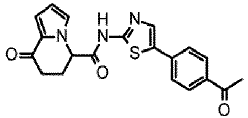 | ¹H-NMR (DMSO-D₆) δ: 12.89 (1H, br s), 8,13 (1H, s), 7.98 (2H, dt, J = 8.7, 2.0 Hz), 7.75 (2H, dt, J = 8.7, 2.0 Hz), 7.23 1H, t, J = 2.1 Hz), 6.86 (1H, dd, J = 4.0, 1.5 Hz), 6.28 (1H, dd, J = 4.0, 2.1 Hz), 5.38 (1H, t, J = 4.3 Hz), 2.58 (3H, s), 2.56-2.52 (2H, m), 2.45-2.42 (2H, m); [M+H]+ = 380. |
| 58 | 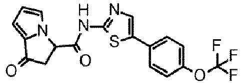 | ¹H-NMR (DMSO-D₆) δ: 12.91 (1H, br s), 8.00 (1H, s), 7.74 (2H, d, J = 8.5 Hz), 7.42 (2H, d, J = 8.5 Hz), 7.39 (1H, d, J = 1.8 Hz), 6.67 (1H, d, J = 4.2 Hz), 6.54 (1H, dd, J = 4.2, 1.8 Hz), 5.50 (1H, dd, J = 8.5, 3.0 Hz), 3.44 (1H, dd, J = 18.1, 8.5 Hz), 3.09 (1H, dd, J = 18.1, 3.0 Hz); [M+H]+ = 408. |
| 59 | 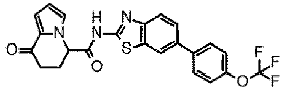 | ¹H-NMR (DMSO-D₆) δ: 13.00 (1H, br s), 8.37 (1H, d, J = 1.8 Hz), 7.89-7.84 (3H, m), 7.78 (1H, dd, J = 8.5, 1.8 Hz), 7.47 (2H, d, J = 8.5 Hz), 7.28 (1H, t, J = 2.1 Hz), 6.88 (1H, dd, J = 3.8, 1.5 Hz), 6.30 (1H, dd, J = 3.8, 2.1 Hz), 5.43 (1H, t, J = 4.2 Hz), 2.66-2.56 (2H, m), 2.47-2.38 (2H, m); [M+H]+ = 472. |
| 60 | 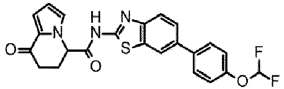 | ¹H-NMR (DMSO-D₆) δ: 12.97 (1H, br s), 8.33 (1H, d, J = 1.5 Hz), 7.85 (1H, d, J = 8.4 Hz), 7.80-7.76 (3H, m), 7.38 (1H, t, J = 74.9 Hz), 7.28-7.28 (3H, m), 6.88 (1H, dd, J = 3.8, 1.5 Hz), 6.30 (1H, dd, J = 3.8, 2.3 Hz), 5.43 (1H, t, J = 4.2 Hz), 2.64-2.56 (2H, m), 2.44-2.42 (2H, m); [M+H]+ = 454. |
| 61 | 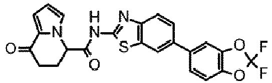 | ¹H-NMR (DMSO-D₆) δ: 12.99 (1H, br s), 8.33 (1H, d, J = 1.5 Hz), 7.84 (1H, d, J = 8.4 Hz), 7.82 (1H, d, J = 1.5 Hz), 7.76 (1H, dd, J = 8.4, 1.9 Hz), 7.58 (1H, dd, J = 8.4, 1.5 Hz), 7.51 (1H, d, J = 8.4 Hz), 7.27 (1H, t, J = 1.9 Hz), 6.87 (1H, dd, J = 3.8, 1.9 Hz), 6.29 (1H, dd, J = 3.8, 2.7 Hz), 5.42 (1H, t, J = 4.2 H z), 2.62-2.56 (2H, m), 2,47-2.40 (2H, m); [M+H]+ = 468. |
| 62 | 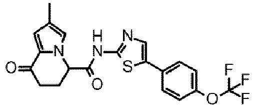 | ¹H-NMR (CDCl₃) δ: 8.64 (1H, br s), 7.56-7.54 (3H, m), 7.25 (2H, d, J = 8.5 Hz), 6.99 (1H, s), 6.77 (1H, s), 5.06-5.05 (1H, m), 2.87-2.84 (1H, m), 2.66-2.46 (3H, m), 2.17 (3H, s); [M+H]+ = 436. |
| 63 | 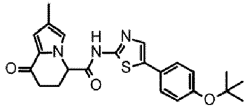 | ¹H-NMR (CDCl₃) δ: 8.72 (1H, br s), 7.50 (1H, s), 7.45-7.41 (2H, m), 7.03-7.00 (211, m), 6.98 (1H, s), 6.77 (1H, s), 5.05 (1H, t, J = 3.9 Hz), 2.89-2.83 (1H, m), 2.65-2.50 (3H, m), 2.17 (311, s), 1.37 (9H, s); [M+H]+ = 424. |
| 64 | 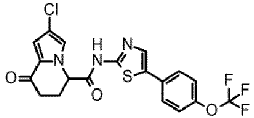 | ¹H-NMR (DMSO-D₆) δ: 12.86 (1H, br s), 7.99 (1H, s), 7.76-7.72 (2H, m), 7.43-7.41 (3H, m), 6.86 (1H, d, J = 1.8 Hz), 5.35 (1H, t, J = 3.9 Hz), 2.64-2.50 (2H, m), 2,47-2.35 (2H, m); |

**[Table 2-10]**

| | | |
|---|---|---|
| 65 | 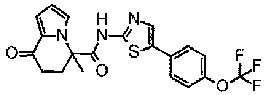 | ¹H-NMR (CDCl₃) δ: 8.07 (1H, br s), 7.55 (1H, s), 7.54 (2H, d, J = 8.5 Hz), 7.26-7.24 (3H, m), 7.16 (1H, s), 6.56 (1H, t, J = 3.3 Hz), 2.90 (1H, dt, J = 13.5, 3.6 Hz), 2.68 (1H, dt, J = 17.5, 3.6 Hz), 2.54-2.48 (1H, m), 2.39 (1H, td, J = 13.5, 4.4 Hz), 2.01 (3H, s); [M+H]+ = 436. |
| 66 | 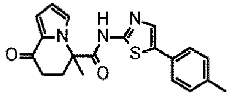 | ¹H-NMR (DMSO-D₆) δ: 12.47 (1H, br s), 7.85 (1H, s), 7.47 (2H, d, J = 8.4 Hz), 7.33 (1H, t, J = 2.3 Hz), 7.22 (2H, d, J = 8.4 Hz), 6.87 (1H, dd, J = 3.8, 1.5 Hz), 6.31 (1H, dd, J = 3.8, 2.3 Hz), 2.83 (1H, d, J = 14.5 Hz), 2.50-2.39 (2H, m), 2.30 (3H, s), 2.28-2.21 (1H, m), 2.00 (3H, s); [M+H]+ = 366. |
| 67 | 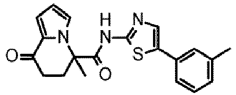 | ¹H-NMR (DMSO-D₆) δ: 12.49 (1H, br s), 7.89 (1H, s), 7.42 (1H, s), 7.38 (1H, d, J = 7.6 Hz), 7.33 (1H, s), 7.29 (1H, t, J = 7,6 Hz), 7.11 (1H, d, J = 7.6 Hz), 6.87 (1H, d, J = 3.4 Hz), 6.31 (1H, t, J = 3.4 Hz), 2.83 (1H, d, J = 14.5 Hz), 2.50-2.39 (2H, m), 2.32 (3H, s), 2.29-2.22 (1H, m), 2.01 (3H, s); [M+H]+ = 366. |
| 68 | 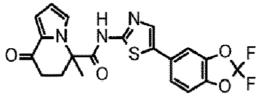 | ¹H-NMR (DMSO-D₆) δ: 7.86 (1H, s), 7.70 (1H, d, J = 1.5 Hz), 7.41 (1H, d, J = 8.4 Hz), 7.33 (1H, dd, J = 8.4, 2.7 Hz), 7.30 (1H, t, J = 2.7 Hz), 6.84 (1H, dd, J = 3.8, 1.5 Hz), 6.28 (1H, t, J = 3.8 Hz), 2.50-2.42 (2H, m), 2.36-2.26 (2H, m), 1.95 (3H, s); [M+H]+ = 432. |
| 69 | 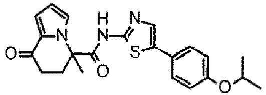 69 | ¹H-NMR (DMSO-D₆) δ: 12.39 (1H, br s), 7.77 (1H, s), 7.49-7.46 (2H, m), 7.33 (1H, t, J = 1.5 Hz), 6.96-6.93 (2H, m), 6.87 (1H, dd, J = 4.2, 1.5 Hz), 6.31 (1H, dd, J = 4.2, 2.3 Hz), 4.67-4.59 (1H, m), 2.83 (1H, d, J = 14.5 Hz), 2.47 (1H, t, J = 3.8 Hz), 2.43-2.37 (1H, m), 2.27-2.20 (1H, m), 2.00 (3H, s), 1.26 (6H, d, J = 6.1 Hz); [M+H]+ = 410. |
| 70 | 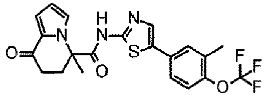 | ¹H-NMR (DMSO-D₆) δ: 12.54 (1H, br s), 7.95 (1H, s), 7.65 (111, d, J = 2.3 Hz), 7.52 (1H, dd, J = 9.2, 2.3 Hz), 7.35-7.34 (2H, m), 6.87 (1H, t, J = 2.3 Hz), 6.31 (1H, dd, J = 3.8, 2.3 Hz), 2.83 (1H, d, J = 12.2 Hz), 2.83 (3H, s), 2.47-2.36 (2H, m), 2.31-2.21 (1H, m), 2.01 (3H, s); [M+H]+ = 450. |
| 71 | 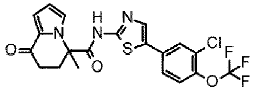 | ¹H-NMR (DMSO-D₆) δ: 12.61 (1H, br s), 8.10 (1H, s), 7.99 (1H, d, J = 2.3 Hz), 7.64-7.60 (2H, m), 7.33 (1H, t, J = 1.5 Hz), 6.88 (1H, dd, J = 3.8, 1.5 Hz), 6.32 (1H, dd, J = 4.2, 2.7 Hz), 2.83 (1H, d, J = 13.8 Hz), 2.50-2,38 (2H, m), 2.28-2.21 (1H, m), 2.02 (3H, s); [M+H]+ = 470. |

**[Table 2-11]**

| | | |
|---|---|---|
| 72 | 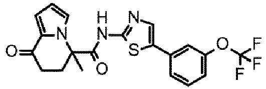 | ¹H-NMR (DMSO-D₆) δ: 12.57 (1H, br s), 8.09 (1H, s), 7.62-7.60 (2H, m), 7.54 (1H, t, J = 8.4 Hz), 7.34 (1H, t, J = 2.3 Hz), 7.30 (1H, d, J = 8.4 Hz), 6.88 (1H, dd, J = 3.8, 1.5 Hz), 6.32 (1H, t, J = 3.8 Hz), 2.83 (1H, d, J = 13.0 Hz), 2.50-2.38 (2H, m), 2.28-2.21 (1H, m), 2.02 (3H, s); [M+H]+ = 436. |
| 73 | 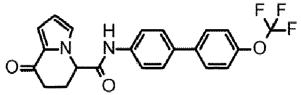 | ¹H-NMR (DMSO-D₆) δ: 10.62 (1H, br s), 7.78 (2H, d, J = 9.1 Hz), 7.73 (2H, d, J = 9.1 Hz), 7.68 (2H, d, J = 9.1 Hz), 7.43 (2H, d, J = 9.1 Hz), 7.20 (1H, t, J = 1.8 Hz), 6.85 (1H, dd, J = 3.9, 1.8 Hz), 6.27 (1H, dd, J = 3.9, 2.4 Hz), 5.23 (1H, t, J = 4.2 Hz), 2.60-2.43 (4H, m); [M+H]+ = 415 . |
| 74 | 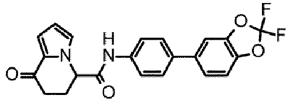 | ¹H-NMR (DMSO-D₆) δ: 10.57 (1H, br s), 7.73 (1H, d, *J* = 1.5 Hz), 7.71-7.65 (4H, m), 7.51-7.46 (2H, m), 7.20 (1H, t, *J* = 1.9 Hz), 6.85 (1H, dd, *J =* 3.8, 1.5 Hz), 6.27 (1H, dd, *J* = 3.8, 1.9 Hz), 5.21 (1H, t, *J* = 4.6 Hz), 2.59-2.44 (4H, m); [M+H]+ = 411. |
| 75 | 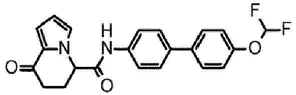 | ¹H-NMR (DMSO-D₆) δ: 10.55 (1H, s), 7,72-7,68 (4H, m), 7.67-7.63 (2H, m), 7.35 (1H, t, J = 74.2 Hz), 7.26-7.23 (2H, m), 7.19 (1H, t, J = 1.9 Hz), 6.84 (1H, dd, J = 3.8, 1.5 Hz), 6.27 (1H, dd, J = 3.8, 1.9 Hz), 5.21 (1H, t, J = 4.2 Hz), 2.60-2.44 (4H, m); [M+H]+ = 397. |
| 76 | 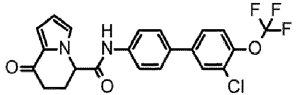 | ¹H-NMR (DMSO-D₆) δ: 10.62 (1H, br s), 7.98 (1H, d, J = 2.3 Hz), 7.77-7.71 (511, m), 7.62 (1H, dd, J = 8.0, 2.3 Hz), 7.20 (1H, t, J = 1.9 Hz), 6.85 (1H, dd, J = 3.8, 1.5 Hz), 6.27 (1H, dd, J = 3.8, 1.9 Hz), 5.22 (1H, t, J = 3.8 Hz), 2.59-2.54 (1H, m), 2.51-2.44 (3H, m); [M+H]+ = 449. |
| 77 | 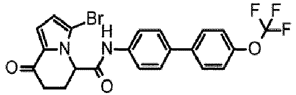 | ¹H-NMR (DMSO-D₆) δ: 10.75 (1H, s), 7.77 (2H, d, J = 9.1 Hz), 7.71 (2H, d, J = 9.1 Hz), 7.68 (2H, d, J = 9.1 Hz), 7.44 (2H, d, J = 9.1 Hz), 6.92 (1H, d, J = 4.2 Hz), 6.48 (1H, d, J = 4.2 Hz), 5.27 (1H, d, J = 3.6 Hz), 2.72-2.66 (1H, m), 2.56-2.45 (311, m); [M+H]+ = 494. |
| 78 | 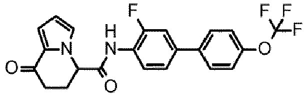 | ¹H-NMR (CDCl₃) δ: 8.31 (1H, t, J = 8.2 Hz), 7.53 (2H, d, J = 8.2 Hz), 7.35-7.17 (6H, m), 7.04 (111, s), 6.52 (1H, s), 5.06 (1H, s), 2.93-2.88 (1H, m), 2.68-2.61 (311, m); [M+H]+ = 433. |

**[Table 2-12]**

| | | |
|---|---|---|
| 79 | 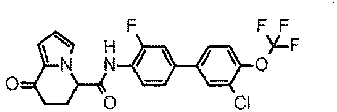 | ¹H-NMR (DMSO-D₆) δ: 10.38 (1H, s), 8.06 (1H, d, J = 2.4 Hz), 8.02 (1H, t, J = 8.2 Hz), 7.82 (1H, dd, J = 8.5, 2.4 Hz), 7.78 (1H, dd, J = 12.2, 2.4 Hz), 7.64 (1H, dd, J = 8.5, 1.8 Hz), 7.60 (1H, dd, J = 8.5, 1.8 Hz), 7.20 (1H, t, J = 2.4 Hz), 6.84 (1H, dd, J = 4.0, 1.5 Hz), 6.28 (1H, dd, J = 4.0, 2.4 Hz), 5.39 (1H, t, J = 3.7 Hz), 2.57-2.44 (4H, m); [M+H]+ = 467. |
| 80 | 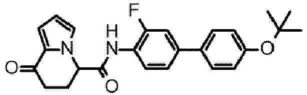 | ¹H-NMR (DMSO-D₆) δ: 10.29 (1H, s), 7.93 (1H, t, J = 8.5 Hz), 7.63-7.59 (3H, m), 7.48 (1H, dd, J = 8.5, 1.8 Hz), 7.20 (1H, t, J = 2,1 Hz), 7,05 (2H, d, J = 8.5 Hz), 6.84 (1H, dd, J = 4.2, 1.5 Hz), 6.27 (1H, dd, J = 4.2, 2.1 Hz), 5.37 (1H, t, J = 3.9 Hz), 2.57-2.44 (4H, m), 1.33 (9H, s); [M+H]+ = 421. |
| 81 | 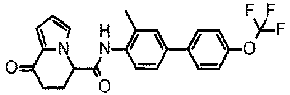 | ¹H-NMR (DMSO-D₆) δ: 9.81 (1H, s), 7.78 (2H, d, J = 8.5 Hz), 7.58 (1H, s), 7.56-7.49 (2H, m), 7.44 (2H, d, J = 8.5 Hz), 7.21 (1H, t, J = 2.1 Hz), 6.84 (1H, dd, J = 4.2, 1.8 Hz), 6.29 (1H, dd, J = 4.2, 2.1 Hz), 5.31 (1H, t, J = 3.9 Hz), 2.60-2.46 (4H, m), 2.30 (3H, s); [M+H]+ = 429. |
| 82 | 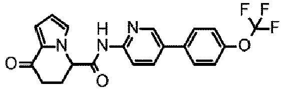 | ¹H-NMR (DMSO-D₆) δ: 11.15 (1H, s), 8.73 (1H, dd, J = 2.1, 0.9 Hz), 8.16 (1H, dd, J = 8.5, 2.4 Hz), 8.13 (1H, d, J = 8.5 Hz), 7.87-7.86 (2H, m), 7.48 (211, d, J = 7.9 Hz), 7.22 (1H, dd, J = 2.4, 1.2 Hz), 6.85 (1H, dd, J = 4.0, 1.2 Hz), 6.27 (1H, dd, J = 4.0, 2.4 Hz), 5.36 (1H, 1, J = 3.7 Hz), 2.56-2.41 (4H, m); [M+H]+ = 416. |
| 83 | 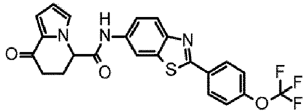 | ¹H-NMR (DMSO-D₆,) δ: 10.77 (1H, s), 8.58 (1H, d, *J* = 1.8 Hz), 8.20 (2H, d, *J* = 9.1 Hz), 8.05 (1H, d, *J* = 9.1 Hz), 7.63 (1H, dd, *J =* 9.1, 1.8 Hz), 7.57 (2H, d, *J* = 9.1 Hz), 7.22 (1H, t, *J* = 2.1 Hz), 6.85 (1H, dd, *J* = 3.9, 1.5 Hz), 6.28 (1H, dd, *J* = 3.9, 2.1 Hz), 5.26 (1H, t*, J* = 4.2 Hz), 2.62-2.45 (4H, m); [M+H]+ - 472, |
| 84 | 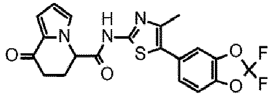 | ¹H-NMR (DMSO-D₆) δ: 12.76 (1H, br s), 7.54 (1H, d, J = 1.8 Hz), 7.48 (1H, d, J = 8.5 Hz), 7.26 (1H, dd, J = 8.5, 1.8 Hz), 7.21 (1H, t, J = 2.4 Hz), 6.85 (1H, dd, J = 4.0, 1.5 Hz), 6.27 (1H, dd, J = 4.0, 2.4 Hz), 5.33 (1H, t, J =4.3 Hz), 2.59-2.54 (2H, m), 2.43-2.40 (2H, m), 2.35 (3H, s); [M+H]+ = 432. |
| 85 | 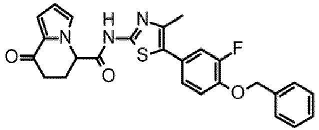 | ¹H-NMR (DMSO-D₆) δ: 12.70 (1H, br s), 7.48-7.47 (2H, m), 7.43-7.40 (2H, m), 7.37-7.29 (3H, m), 7.20-7.17 (2H, m), 6.85 (1H, dd, J = 4.0, 1.5 Hz), 6.27 (1H, dd, J = 4.0, 2.0 Hz), 5.32 (1H, t, J = 4.0 Hz), 5.22 (2H, s), 2.56-2.54 (2H, m), 2.44-2.39 (2H, m), 2.34 (3H, s); [M+H]+ = 476. |

**[Table 2-13]**

| | | |
|---|---|---|
| 86 | 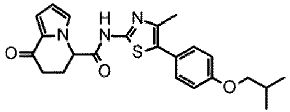 | ¹H-NMR (DMSO-D₆) δ: 12.64 (111, br s), 7.34-7.33 (2H, m), 7.20 (1H, t, J = 2.1 Hz), 7.01-6.99 (2H, m), 6.85 (1H, dd, J = 4.0, 1.5 Hz), 6.27 (1H, dd, J = 4.0, 2.1 Hz), 5.32 (1H, t, J = 4.0 Hz), 3.77 (2H, d, J = 6.7 Hz), 2.57-2.53 (2H, m), 2.43-2.41 (2H, m), 2.33 (3H, s), 2.07-1.97 (1H, m), 0.98 (6H, d, J = 6.7 Hz); [M+H]+ = 424. |
| 87 | 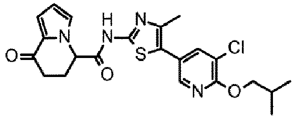 | ¹H-NMR (DMSO-D₆) δ: 12.77 (1H, br s), 8.18 (1H, d, J = 2.0 Hz), 7.98 (1H, d, J = 2.0 Hz), 7.2] (1H, t, J = 2.1 Hz), 6.85 (1H, dd, J = 4.0, 1.5 Hz), 6.27 (1H, dd, J = 4.0, 2.1 Hz), 5.34 (1H, t, J = 4.0 Hz), 4.15 (2H, d, J = 6.7 Hz), 2.59-2.54 (2H, m), 2.44-2.39 (2H, m), 2.33 (3H, s), 2.12-2.02 (1H, m), 0.99 (6H, d, J = 6.7 Hz); [M+H]+ = 460. |
| 88 | 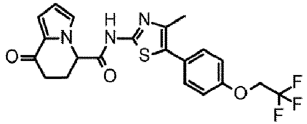 | ¹H-NMR (DMSO-D₆) δ: 12.68 (1H, br s), 7.41-7.40 (2H, m), 7.20 (1H, t, J = 2.4 Hz), 7.14-7.13 (2H, m), 6.85 (1H, dd, J = 40, 1.5 Hz), 6.27 (1H, dd, J = 4.0, 2.4 Hz), 5.33 (1H, t, J = 4.3 Hz), 4.80 (2H, q, J = 8.7 Hz), 2.58-2.51 (2H, m), 2.44-2.40 (2H, m), 2.34 (3H, s); [M+H]+ = 450. |
| 89 | 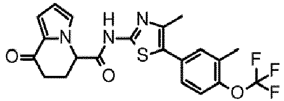 | ¹H-NMR (DMSO-D₆) δ: 12.75 (1H, br s), 7.47 (1H, s), 7.38 (2H, s), 7.20 (1H, t, J = 2.0 Hz), 6.85 (1H, dd, J = 4.0, 1.5 Hz), 6.27 (1H, dd, J = 4.0, 2.0 Hz), 5.32 (1H, t, J = 4.0 Hz), 2.58-251 (2H, m), 2.43-2.41 (2H, m), 2.37 (3H, s), 2.31 (3H, s); [M+H]+ = 450. |
| 90 | 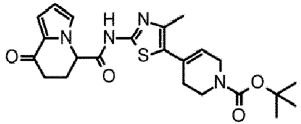 | ¹H-NMR (DMSO-D₆) δ: 12.60 (1H, br s), 7.18 (1H, t, J = 2.1 Hz), 6.84 (1H, dd, J = 4.0, 1.5 Hz), 6.26 (1H, dd, J = 4.0, 2.1 Hz), 5.84 (1H, br s), 5.29 (1H, t, J = 4.3 Hz), 3.97 (2H, br s), 3.50 (2H, t, J = 5.5 Hz), 2.54-2.45 (2H, m), 2.42-2.33 (4H, m), 2.30 (3H, s), 1.42 (911, s); [M+H]+ = 457. |
| 91 | 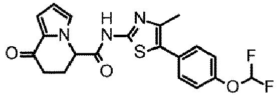 | ¹H-NMR (DMSO-D₆) δ: 12.72 (1H, br s), 7.52-7.48 (2H, m), 7.28 (1H, t, J = 74.5 Hz), 7.27-7.24 (2H, m), 7.21 (1H, t, J = 2.1 Hz), 6.85 (1H, dd, J = 4.0, 1.5 Hz), 6.27 (1H, dd, J = 4.0, 2.1 Hz), 5.34 (1H, t, J = 4.0 Hz), 2.58-2.52 (211, m), 2.44-2.40 (2H, m), 2.35 (3H, s); [M+H]+= 418. |
| 92 | 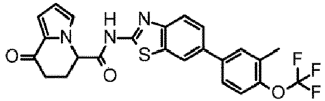 92 | ¹H-NMR (DMSO-D₆) δ: 12.98 (1H, br s), 8.35 (1H, d, J = 1.8 Hz), 7.86 (1H, d, J = 8.5 Hz), 7.79-7.77 (2H, m), 7.67 (1H, dd, J = 8.5, 2.4 Hz), 7.40 (111, dd, J = 8.5, 1.2 Hz), 7.27 (111, t, J = 2.1 Hz), 6.87 (1H, dd, J = 4.0, 1.5 Hz), 6.29 (1H, dd, J = 4.0, 2.1 Hz), 5.43 (1H t, J = 4.0 Hz), 2.63-2.57 (2H, m), 2.45-2.42 (2H, m), 2.36 (3H, s); [M+H]+ = 486. |

**[Table 2-14]**

| | | |
|---|---|---|
| 93 | 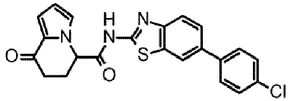 | ¹H-NMR (DMSO-D₆) δ: 12.98 (1H, br s), 8.35 (1H, d, J = 1.8 Hz), 7.85 (1H, d, J = 8.5 Hz), 7.78-7.76 (3H, m), 7.55-7.51 (2H, m), 7.27 (1H, t, J = 2.1 Hz), 6.87 (1H, dd, J = 4.0, 1.5 Hz), 6.29 (1H, dd, J = 4.0, 2.1 Hz), 5.42 (1H, t, J = 4.3 Hz), 261-2.57 (2H, m), 2.46-2.41 (2H, m); [M+H]+ = 422. |
| 94 | 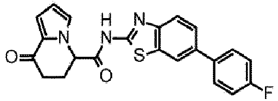 | ¹H-NMR (DMSO-D₆) δ: 12.97 (1H, br s), 8.30 (1H, d, J = 1.8 Hz), 7.83 (1H, d, J = 8.5 Hz), 7.79-7.72 (3H, m), 7.30 (2H, t, J = 8.5 Hz), 7.26 (1H, t, J=2.1 Hz), 6.87 (1H, dd, J = 4.0, 1.5 Hz), 6.29 (1H, dd, J = 4.0, 2.1 Hz), 5.40 (1H, t, J = 4.0 Hz), 2.62-2.56 (2H, m), 2.47-2.42 (2H, m); [M+H]+ = 406. |
| 95 | 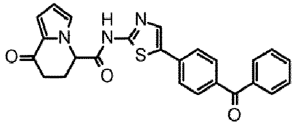 | ¹H-NMR (DMSO-D₆) δ: 12.91 (1H, s), 8.15 (1H, s), 7.82-7.74 (6H, m), 7.69 (1H, t, J = 6.7 Hz), 7.58-7.57 (2H, m), 7.24 (1H, br s), 6.87 (1H, d, J = 3.6 Hz), 6.29 (1H, t, J = 3.6 Hz), 5.39 (1H, t, J = 3.9 Hz), 2.63-2.53 (2H, m), 2.45-2.43 (2H, m); [M+H]+ = 442. |
| 96 | 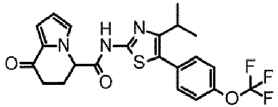 | ¹H-NMR (DMSO-D₆) δ: 12.85 (1H, br s), 7.51 (2H, d, J = 8.5 Hz), 7.46 (2H, d, J = 8.5 Hz), 7.20 (1H, s), 6.85 (1H, d, J = 3.6 Hz). 6.27 (1H, t, J = 2.7 Hz), 5.34 (1H, t, J = 4.2 Hz), 3.11-3.04 (1H, m), 2.57-2.55 (2H, br m), 2.44-2.42 (2H, br m), 1.23 (6H, d, J = 6.7 Hz); [M+H]+ = 464. |
| 97 | 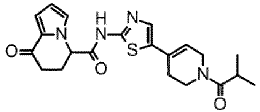 | ¹H-NMR (DMSO-D₆) δ: 12.68 (1H, br s), 7.48 (1H, s), 7.21 (1H, t, J = 1.5 Hz), 6.85 (1H, dd, J = 3.9, 1.5 Hz), 6.27 (1H, dd, J = 3.9, 2.4 Hz), 5.98-5.94 (1H, br m), 5.33 (1H, t, J = 4.2 Hz), 4.18 (1H, br s), 4.05 (1H, br s), 3.69-3.63 (2H, m), 2.99-2.81 (1H, m), 2.59-2.49 (4H, m), 2.44-2.39 (2H, br m), 1.01 (3H, d, J = 6.7 Hz), 0.98 (3H, d, J = 6.7 Hz); [M+H]+ = 413. |
| 98 | 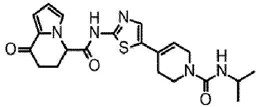 | ¹H-NMR (DMSO-D₆) δ: 12.67 (1H, br s), 7.48 (1H, s), 7.21 (1H, t, J = 1.8 Hz), 6.85 (1H, dd, J = 3.9, 1.8 Hz), 6.27 (1H, dd, J = 3.9, 2.7 Hz), 6.18 (1H, d, J = 7.3 Hz), 5.94 (1H, t, J = 3.6 Hz), 5.33 (1H, t, J = 4.2 Hz), 3.94-3.90 (2H, br m), 3.80-3.72 (1H, m), 3.49 (2H, t, J = 5.7 Hz), 259-2.49 (2H, m), 2.44-2.38 (4H, br m), 1.05 (6H, d, J = 6.0 Hz); [M+H]+ = 428. |
| 99 | 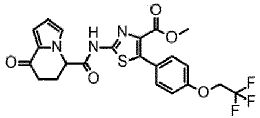 | ¹H-NMR (CDCl₃) δ: 8.82 (1H, br s), 7.49-7.45 (2H, m), 7.18 (1H, dd, J = 4.2, 1.2 Hz), 7.01-6.96 (3H, m), 6.49 (1H, dd, J = 4.2, 2.4 Hz), 5.16 (1H, dd, J = 5.1, 2.7 Hz), 4.40 (2H, q, J = 8.1 Hz), 3.79 (3H, s), 2.88-2.82 (1H, m), 2.69-2.51 (3H, m); [M+H]+ = 494. |

**[Table 2-15]**

| | | |
|---|---|---|
| 100 | 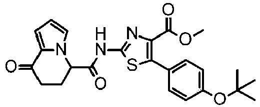 | ¹H-NMR (CDCl₃) δ: 8.80 (1H, br s), 7.42-7.39 (2H, m), 7.18 (1H, dd, J = 4.2, 1.2 Hz), 7.04-7.02 (2H, m), 6.97 (1H, t, J = 2.4 Hz), 6.48 (1H, dd, J = 4.2, 2.4 Hz), 5.16 (1H, dd, J = 4.8, 2.4 Hz), 3.78 (3H, s), 2.87-2.82 (1H, m), 2.69-2.52 (3H, m), 1.40 (9H, s); [M+H]+ = 468. |
| 101a | First peak | HPLC measurement conditions column : YMC CHIRAL ART Cellulose-SB (5um), 250 × 4,6 mm I.D., column temperature: 25°C, flow rate: 0.5 ml/min, mobile phase: n hexane/ethanol = 40/60, detectioin wavelength : 288 nm, retention time : tR = 13.6 min |
| | Optically active form of compound of Synthesis Example 35 | |
| 101b | Second peak | HPLC measurement conditions column : YMC CHIRAL ART Cellulose-SB (5um), 250 × 4.6 mm I.D., column temperature : 25°C, flow rate : 0.5 ml/min, mobile phase: n-hexane/ethanol = 40/60, detectioin wavelength : 288 nm, retention time : tR = 23.4 min |
| | Optically active form of compound of Synthesis Example 35 | |

### [Reference Example 1]

### Evaluation of effect to promote differentiation from human iPS cells into insulin-producing cells

The evaluation system of the effect (efficacy) to promote differentiation from human iPS cells into insulin-producing cells was constructed with reference to known information (Non Patent Document 6). Further, the medium used in each differentiation stage was also produced with reference to known information (differentiation media A to E (Media A to E) described in Non Patent Document 6 were used respectively for stages 1 to 5; however, a medium free from GLP-1 receptor agonist and nicotinamide was used as differentiation medium E).

In order to evaluate the efficacy of each compound, the compound of Synthesis Example 1 was used as a positive control, and dimethylsulfoxide (DMSO) (SIGMA, D2650) with a final concentration of 0.1% was used as a control untreated with compounds. Each compound was dissolved in DMSO, and two types of compound solutions were prepared so as to have final concentrations of 2 µM and 10 µM after the compound was added to the medium. In the following evaluation, the compound solutions were added to the medium to 0.1% that is the final concentration of DMSO.

First, induction from human iPS cell Toe strain (National Institutes of Biomedical Innovation, Health and Nutrition) into cells on day 7 of culture (2 days after replacement with differentiation medium C (cells in the differentiation process from FOXA2-positive primitive gut tube cells into PDX1-positive pancreatic progenitor cells)) was performed according to the method of Non Patent Document 6 for "inducing differentiation from human iPS cells into pancreatic β cells", and the cells were collected and thereafter stored in liquid nitrogen using Bambanker (NIPPON Genetics Co, Ltd.) at 1 × 10⁷ cells/mL/tube, to produce a cell stock for evaluation. The cell stock was dissolved at the start of the evaluation of efficacy, suspended in differentiation medium C for stage 3 (DMEM high glucose (Life technologies, 11965092), 0.25 µM SANT-1, 0.1 µM LDN193189 (Stemgent, 04-0074), 10µM SB431542, 2 µM Retinoic acid (Stemgent, 04-0021), 1% B27 serum free supplement (Life technologies, 17504044) and thereafter seeded in a 96-well plate (Corning, #3340) coated with Synthemax II (Corning, #5656) at 1 × 10⁵ cells/well. After culturing for 2 days, the medium was removed, and new differentiation medium C for stage 3 with the compound or only DMSO added was added thereto at 100 µL/well. After culturing for 2 days, the medium was removed, and new differentiation medium D for stage 4 (DMEM high glucose, 0.1 µM LDN193189, 5 µM TGF-β type I receptor kinase inhibitor II (Calbiochem 616452), 0.3 µM (-)-indolactam V (Enzo life science ALX-420-011-C300), 1% B27 serum free supplement) with the compound or only DMSO added was added thereto at 100 µL/well. After culturing for 2 to 3 days, the medium was removed, and new differentiation medium E for stage 5 (GLP-1 receptor agonist and nicotinamide-free; Knockout DMEM/F-20 (Life technologies, 12660012), 1% B27 serum free supplement) with the compound or only DMSO added was added thereto at 200 µL/well. After culturing for 2 days, the medium was removed, and a 4% paraformaldehyde phosphorus acid buffer (Wako, 163-20145) was added thereto at 150 µL/well and left standing for 30 to 60 minutes at room temperature to fix the cells. A phosphorus acid buffer (PBS) (Takara, T9181) containing 1% Triton X-100 (Sigma, T8787) was left standing for 15 minutes at room temperature, then washed with PBS-T (Takara, T9183), and was blocked for 1 hour using 20% Blocking One (Nacalai tesque, Tokyo, Japan) diluted with PBS-T at room temperature. After the removal of Blocking One, guinea pig anti-insulin antibody (Abcam,ab7842) diluted 200-fold with 20% Blocking One was added thereto at 50 µL/well, followed by standing at 4°C overnight. After washing with PBS-T 3 times, Alexa Fluor 548-labeled anti-guinea pig antibody (Life Technologies, A11075) diluted 1000-fold with 20% Blocking One and 6-diamidino-2-phenylindole (DAPI) (Roche Diagnostics, Basel, Switzerland) were added thereto, followed by standing at room temperature for 2 hours. After washing with PBS-T 3 times, the fluorescence images of the cells were analyzed.

The cell images were captured using a high-content imaging system Opera Phenix or Operetta (PerkinElmer). Further, the total number of insulin-positive cells and DAPI-positive cells was measured by analysis using Harmony (PerkinElmer) to calculate the ratio of the number of the insulin-positive cells with respect to the total number of the cells (insulin-positive cell rate). The compound of Synthesis Example 1 was used as a positive control, and DMSO with a final concentration of 0.1% was used as a control untreated with compounds. The increment in insulin-positive cell rate (average insulin-positive cell rate of 30 cases: 13%) of 10 µM of the compound of Synthesis Example 1 from the control untreated with compounds (average insulin-positive cell rate of 30 cases: 4.9%) was taken as 100%. The increment in insulin-positive cell rate of each compound at each concentration was converted into a percentage (%) based on the above, to obtain an activity value. The primary evaluation of the compound including the positive control was performed for each compound at two concentrations of 2 µM and 10 µM using a plurality of wells. The activity intensity was determined by comparing the sum of activity values at the two concentrations with the positive control. The case where the sum of activity values at the two concentrations was obviously higher than the control untreated with compounds while being lower than the positive control was expressed as +, the case where the sum was equivalent to the positive control was expressed as ++, and the case where the sum was higher than the positive control was expressed as +++. Compounds having a weak activity intensity were evaluated again at a concentration of 0.4, 2, 5 or 10 µM using a plurality of wells, and compounds obviously exhibiting a higher activity value than the control untreated with compounds or exhibiting an activity value of 15% or more at any concentration and exhibiting a significant difference (P < 0.05) in the t test as compared with the control untreated with compounds were determined to be effective. Compounds exhibiting comparatively strong efficacy were subjected to the secondary evaluation at a concentration from 0.01 to 10 µM using a plurality of wells, in order to investigate the concentration-dependent effect, to calculate EC50 (when the efficacy of 10 µM of the compound of Synthesis Example 1 was taken as 100%, the concentration of the compound at which the efficacy corresponding to 50% thereof can be exerted) using Sigma Plot (Systat Software).

Table 3 and Table 4 show the results of Reference Example 1.

**[Table 3]**

| Synthesis Example No. | Activity | Synthesis Example No. | Activity | Synthesis Example No. | Activity | Synthesis Example No. | Activity | Synthesis Example No. | Activity |
|---|---|---|---|---|---|---|---|---|---|
| 1 | ++ | 2 | ++ | 3 | + | 4 | ++ | 5 | + |
| 6 | + | 7 | + | 8 | ++ | 9 | +++ | 10a | +++ |
| 10b | +++ | 11 | +++ | 12 | ++ | 13 | ++ | 14 | + |
| 15 | + | 16 | ++ | 17 | ++ | 18 | ++ | 19 | ++ |
| 20 | ++ | 21 | +++ | 22 | +++ | 23 | + | 24 | + |
| 25 | +++ | 26 | ++ | 27 | ++ | 28 | +++ | 29 | +++ |
| 30 | +++ | 31 | + | 32 | +++ | 33 | +++ | 34 | ++ |
| 35 | +++ | 36 | +++ | 37 | + | 38 | +++ | 39 | +++ |
| 40 | +++ | 41 | + | 42 | + | 43 | +++ | 44 | + |
| 45 | +++ | 46 | +++ | 47 | ++ | 48 | + | 49 | +++ |
| 50 | + | 51 | +++ | 52 | +++ | 53 | ++ | 54 | + |
| 55 | + | 56 | ++ | 57 | + | 58 | ++ | 59 | +++ |
| 60 | ++ | 61 | + | 62 | +++ | 63 | +++ | 64 | ++ |
| 65 | +++ | 66 | ++ | 67 | ++ | 68 | ++ | 69 | ++ |
| 70 | ++ | 71 | +++ | 72 | +++ | 73 | + | 74 | + |
| 75 | + | 76 | + | 77 | + | 78 | ++ | 79 | + |
| 80 | + | 81 | + | 82 | + | 83 | + | 84 | +++ |
| 85 | +++ | 86 | +++ | 87 | +++ | 88 | +++ | 89 | +++ |
| 90 | +++ | 91 | +++ | 92 | +++ | 93 | +++ | 94 | +++ |
| 95 | +++ | 96 | + | 97 | ++ | 98 | ++ | 99 | ++ |
| 100 | + | 101a | + | 101b | +++ | | | | |

**[Table 4]**

| Synthesis Example No. | EC50 (*µ* M) |
|---|---|
| 1 | 5.8 |
| 9 | 0.23 |
| 10a | 1.2 |
| 10b | 0.22 |
| 11 | 0.49 |
| 21 | 0.45 |
| 30 | 1.0 |
| 35 | 0.25 |
| 36 | 0.31 |
| 40 | 0.82 |
| 43 | 0.19 |
| 45 | 0.13 |
| 46 | 0.54 |
| 58 | 1.6 |
| 59 | 0.54 |
| 65 | 1.8 |
| 88 | 0.24 |
| 93 | 0.29 |
| 101b | 0.09 |

It was found from the results of Reference Example 1 that a compound represented by formula (I) or a salt thereof could efficiently promote differentiation of pluripotent stem cells into insulin-producing cells as compared with the case without addition of the compound.

### [Example 1]

### (A) Results

### (1) Screening of novel β cell differentiation inducer

As previously reported (Sakano et al., 2014; Shiraki et al., 2008), the mouse ES cell line SK7 carrying GFP reporter gene driven by Pdxl promoter was used for establishing an assay system for screening of a small-molecule compound that enhanced β cell differentiation.

Following primary screening campaign using about 55,000 compounds, and subsequent repeated assay to confirm the reproducibility and dose dependence of effectiveness, the hit compound K-1 (compound synthesized in Synthesis Example 1) was found as a novel compound that enhanced β cell differentiation (Figure 1). K-1 increased the ratio of Pdxl-GFP+insulin+ double-positive cells and the expression of insulin 1 in a dose-dependent manner. K-1 further enhanced increase in the number of Pdxl-GFP+Ins+ double-positive cells by the γ-secretase inhibitor LY411575 (Treff et al., 2006). This result indicates that K-1 exhibits its efficacy through the mechanism of action different from that of γ-secretase inhibition which has previously been reported to promote differentiation into pancreatic endocrine.

### (2) K-1 and derivative thereof promote differentiation of β cells derived from human iPS cells.

The effectiveness of a compound for differentiation of human iPS cells into insulin-expressing β (hiPS-β) cells was examined using two different differentiation protocols and three human iPS cell lines (Toe, RPChiPSC771, and Ff-I01s01 (iPSC derived from a HLA homozygous patient)) (Figure 2-1a). The present inventors first tested treatment of Toe iPSC with K-1 from the later stage of stage 3 to the initial stage of stage 5 in monolayer culture according to differentiation protocol #1. It brought about enhanced differentiation into iPS-β cells (Figures 2-la and 2-1b). The present inventors also tested the effects of other derivatives such as K-3 (compound synthesized in Synthesis Example 9), K-4 (compound synthesized in Synthesis Example 33), and K-5 (compound synthesized in Synthesis Example 101b). K-3 and K-5 were found to exhibit stronger efficacy than that of the original compound K-1 (Figure 2-1b).

Next, the effect of K-3 was tested using another differentiation protocol recently established by the present inventors. The present inventors previously found that methionine depletion treatment for a short period before differentiation brings about enhancement in differentiation (Shiraki et al., 2014). Accordingly, the present inventors adopted methionine depletion for a short time (5 hours) and performed sphere culture of RPChiPS771 iPSC according to differentiation protocol #1 to obtain highly functional iPS-β cells (Shiraki et al., 2014). K-3 at 0.25 µM enhanced differentiation into INSULIN+PDX1+ double-positive cells (Figure 2-1c). Next, differentiation protocol #2 was used. Here, cells were pretreated by methionine depletion before differentiation, and differentiation was performed using a medium with an adjustable zinc concentration. According to this protocol, K-3 enhanced differentiation of Ff-I01s01 into INSULIN+NKX6.1+, NKX6.1+PDX1+ double-positive, and INSULIN+NKX6.1+PDX1+ triple-positive cells. They appeared at the end of stage 4 and then increased in number (Figure 2-1d). When K-3 was added in a time window including stage 3, K-3 was found to enhance differentiation most effectively. K-3 also promoted the production of NKX6.1+ and INSULIN+NKX6.1+ double-positive cells by treatment, particularly, in stages 3 to 5 (Figure 2-1d).

Subsequently, function assay of iPS-β cells produced by sphere culture of RpChiPSC771 according to protocol #1 was carried out. In this function assay, K-3 as well as K-5, K-6 (compound synthesized in Synthesis Example 45) and K-7 (compound synthesized in Synthesis Example 93) were used. The hit compounds including K-3 enhanced the glucose stimulated insulin secretion (GSIS) activity of the obtained iPS-β cells and exhibited rapid first-phase insulin secretion and long-lasting second-phase insulin secretion in step-wise time-course measurement (Figure 2-2e, left). In order to quantify GSIS activity, conventional batch-wise assay was also carried out (Figure 2-2e, right). K-3 exhibited similar effects in culture protocol #2 (Figure 2-2f). Accordingly, this indicates that their effectiveness does not depend on variability in culture protocol or cell line. The iPS-β cells treated with K-3 exhibited enhanced c-peptide secretion by stimulation with insulin secretagogues (glibenclamide, exendin 4, and KCl) (Figure 2-2g). This indicates that the compound enhances functional maturation or increases the insulin content of cells.

### (B) Method

### (1) Culture of mES and hiPS cell lines

The SK7 mouse ES cell line was established from a transgenic mouse strain having Pdx1-GFP gene, and maintained on MEF feeder cells (Stem cell technology) as previously described (Shiraki et al., 2008). The Toe human iPS cell line was obtained from the cell bank of the National Institute of Biomedical Innovation (Japan), and maintained under feeder-free xenogeneic conditions as previously described (Shahjalal et al., 2014). The RPChiPS771 cells were purchased from ReproCELL Inc., and the FfI-01s01 cells were provided from CiRA. The RPChiPS771 cells and the Ffl-01s01 cells were cultured on dishes coated with synthemax II (Invitrogen, 3535XX1) using Essential 8 (Invitrogen, A1517001) and StemFit AK03N (Ajinomoto Co., Inc.), respectively.

### (2) Differentiation of SK7 mouse ES cells and human iPS cells

The SK7 mouse ES cells were differentiated into β cells basically according to the previous report (Nakashima et al., 2015). Briefly, the cells were seeded at 5,000 cells per well in CellBIND 384-well (CORNING, 3770) cell culture plate or at 20,000 cells per well in a 96-well plate (Sigma) coated with 0.2% gelatin. Then, the cells were cultured in medium 1 from days 1 to 5. Medium 1 consisted of DMEM (high glucose) (Life Technologies, 11965092) supplemented with 0.1 mM NEAA (Gibco, 11140-50), 2 mM L-glutamine (Nacalai, 16948-04), 100 U/mL penicillin-streptomycin (Nacalai, 26252-94), 0.01 mM β-mercaptoethanol (Sigma), 1% insulin-transferrin-selenium supplement (ITS: Life Technologies, 41400045), 0.25% Albumax (Thermo Fisher, 11020021), and 10 ng/mL recombinant human activin-A (R&D Systems, 338-AC). Subsequently, the cells were cultured for 1 day in medium 2 which consisted of medium 1 supplemented with 10 µM retinoic acid (Stemgent, 04-0021). On day 6, the medium was replaced with medium 3, and the culture was continued until day 12 in order to induce differentiation into β cells. Medium 3 consisted of DMEM (low glucose) (Life Technologies, 11885084) supplemented with 0.1 mM NEAA, 2 mM L-glutamine, 100 U/mL penicillin-streptomycin, 0.1 mM β-mercaptoethanol, 1% ITS, 0.25% Albumax, and 3 mM nicotinamide (Sigma-Aldrich, N0636-500G). A test compound was added on day 8, and the cells were fixed in 4% paraformaldehyde for analysis using a high-content imager.

The Toe human iPS cells were seeded in CellBIND 6-well (CORNING, CLS3335) cell culture plate coated with synthemax II, and differentiated into primitive gut tube cells by the previously described method (Shahjalal et al., 2014). The cells were dissociated with TrypLE Select (Thermo Fisher, 12563011) and cryopreserved in BanBanker (Nippon genetics) at a density of 1.0 to 2.0 × 10⁷ cells/mL until use. One day before compound addition, the cells were thawed in a medium for stage 3 (DMEM (high glucose), 0.25 µM SANT1 (Wako, 197-16351), 0.1 µM LDN193189 (Stemgent, 04-0074), 10 µM SB431542, 2 µM retinoic acid, 1% B27-free serum supplement (Life Technologies, 17504044), and seeded at 150,000 cells per well in CellBIND 96-well (Corning, 3340) cell culture plated coated with synthemax II. The cells were cultured for 1 day in a medium for stage 3 containing a test compound or a negative control (0.01% DMSO). The cells were further cultured for 2 days and 1 day in media for stages 4 and 5, respectively, containing the test compound or the negative control. On the next day, the cells were fixed in 4% paraformaldehyde for analysis using a high-content imager.

The RPChipS771 cells were maintained as described above, and differentiated according to protocol #1. Briefly, on day 0, the cells were dissociated with TrypLE Select, transferred at a concentration of 1 × 10⁶ cells/mL in Essential 8 medium to a low-attachment 6-well plate (Greiner, 657185), and cultured on a rotary shaker (95 rpm). On day 1, the medium was replaced with methionine-depleted medium KA01 (Ajinomoto), and the cells were cultured for 5 hours. The medium was replaced with differentiation medium 1 (DMEM (high glucose), L-Gln, NEAA, 0.01 mM β-mercaptoethanol, 100 ng/mL activin A, B27 supplement, 3 µM CHIR99021), and the cells were cultured for 1 day (stage 1-1). Then, the medium was replaced with medium 1 free from CHIR99021, and the cells were cultured for 2 days (stage 1-2). The cell culture was continued for 2 days in medium 2 (RPMI, L-Gln, NEAA, 0.01 mM β-mercaptoethanol, insulin-depleted B27 supplement, 50 ng/mL FGF10, 0.25 µM SANT1) (stage 2), for 6 days in medium 3 (DMEM (high glucose), L-Gln, NEAA, 0.01 mM β-mercaptoethanol, 0.15 µM SANT1, 2 µM retinoic acid, 0.1 µM LDN193189, B27 supplement) (stage 3), for 2 days in medium 4 (DMEM (high glucose), L-Gln, NEAA, 0.01 mM β-mercaptoethanol, 5 µM ALK5 inhibitor (Calbiochem, 616452), 0.3 µM indolactam V, 0.1 µM LDN193189, B27 supplement) (stage 4), and for 13 days in medium 5 (KO DMEM/F12, L-Gln, NEAA, 0.01 mM β-mercaptoethanol, 50 ng/mL exendin 4, 10 mM nicotinamide, 10 µM ZnSO₄, 1 mM N-acetyl-L-cysteine, B27 supplement) (stage 5). In the case of evaluating the effectiveness of a compound for a differentiation rate and a cell function, a test compound and a negative control (0.01% DMSO) were treated between stages 3 and 4 (days 6 to 14), and immunostaining was performed on day 19 in order to confirm an effect on positivity to insulin. GSIS (glucose stimulated insulin secretion) assay was carried out on day 27.

The FfI-01s01 cells were maintained as described above and then differentiated according to protocol #2. Briefly, the cells were dissociated with TrypLE Select, transferred at a concentration of 1 × 10⁶ cells/mL in AK03N medium (Ajinomoto Co., Inc.) to a low-attachment 6-well plate, and cultured for 24 hours on a rotary shaker (95 rpm). Then, the medium was replaced with AK03N-based methionine-depleted medium KA01, and the cells were cultured for 5 hours. Then, the medium was replaced with M1-1 AKM medium, and the cells were cultured for 24 hours (stage 1-1). The cells were cultured for 2 days in Ml-2 AKM medium (stage 1-2), subsequently cultured for 2 days in M2 AKM medium (stage 2-1), cultured for 2 days in S2 medium (stage 2-2), cultured for 2 days in S3 medium (stage 3), cultured for 5 days in S4 medium (stage 4), cultured for 4 days in S5-1 medium (stage 5-1), and cultured for 3 days in S5-2 medium (stage 5).

AKM medium is insulin- and Zn²⁺-depleted StemFit Basic 03 medium.
M1-1 AKM medium: AKM (100 ng/mL IGF1, 0.5 µM Zn) medium supplemented with 100 ng/mL activin A and 3 µM CHIR990221
Ml-2 AKM medium: AKM (100 ng/mL IGF1, 0.5 µM Zn) medium supplemented with 100 ng/mL activin A
M2 AKM medium: AKM (0.5 µM Zn) medium supplemented with 50 ng/mL FGF10 and 250 nM SANT1
S2 medium: StemFit Basic 03 supplemented with 50 ng/mL KGF and 44 µg/mL vitamin C
S3 medium: StemFit Basic 03 supplemented with 50 ng/mL KGF, 50 nM indolactam V, 2 µM retinoic acid, 250 nM SANT1, and 44 µg/mL vitamin C
S4 medium: StemFit Basic 03 supplemented with 50 ng/mL KGF, 100 nM retinoic acid, 250 nM SANT1, 44 µg/mL vitamin C, and 100 nM LDN193189
S5-1 medium: StemFit Basic 03 supplemented with 10 µM ALK5 inhibitor, 10 µM DAPT, 33.3 ng/mL EGF, 100 nM retinoic acid, 1 µM T3, and 44 µg/mL vitamin C
S5-2 medium: StemFit Basic 03 supplemented with 10 µM ALK5 inhibitor, 10 µM DAPT, 33.3 ng/mL EGF, 25 nM retinoic acid, and 1 µM T3

In the case of evaluating the effectiveness of a compound for a differentiation rate and a cell function, a test compound and a negative control (0.01% DMSO) were treated between stages 3 and 4 (days 5 to 13), and immunostaining was carried out on days 14 (at the completion of stage 4) and 21 (at the completion of stage 5) in order to confirm an effect on positivity to insulin, NKX6.1, and PDX1. GSIS assay was carried out on day 21.

### (3) Screening of compound that enhances β cell differentiation and quantitative analysis by high-content imaging

A chemical library consisting of about 55,000 compounds was screened for a substance that induced β cell differentiation. For primary screening, a compound was dissolved at a concentration of 2 mM in DMSO, added at 1:1000 on day 8 in the process of differentiation of the SK-7 mouse ES cells, and the medium was replaced on day 10. The cells were assayed by immunostaining with guinea pig anti-insulin and rabbit anti-GFP on day 12. Fluorescence images were quantified using Operetta high-content imaging system and Harmony image analysis software (PerkinElmer, Germany). The nuclei were identified by DAPI staining, and the positivity of antibody staining was determined from the fluorescence intensity of a cytoplasmic region surrounding the nucleus. The number of cells positive to both insulin and GFP was counted and normalized with the total number of DAPI-positive cells or a positive area. A median of quadruple data was calculated, and screening hits were defined as states in which the number of insulin- and GFP-positive cells was increased as compared with a DMSO control. As described herein, primary hit compounds were further tested for dose dependence and reproducibility. The activity of candidate compounds was confirmed using resynthesized ones. The activity of the selected candidate compounds was further analyzed with Toe human iPS cell assay system in order to select a hit compound having effectiveness for both differentiation into mouse β cells and differentiation into human β cells. A compound was added as described above, and its effectiveness was evaluated from a mRNA expression level by q-PCR in triplet experiments. Screening hits were defined as states with a dose-dependent increased insulin expression level relative to a β-actin expression level as compared with a DMSO control.

### (4) Immunocytochemistry

Immunocytochemistry was carried out according to a general protocol using the following antibodies: rabbit anti-MAFA (Abcam; ab26405; 1/100x), goat anti-PDX1 (R&D systems; AF2419; 1/100x), mouse anti-NKX6.1 (Developmental Studies Hybridoma Bank, University of Iowa; F64A6B4; 1/100x), guinea pig anti-INSULIN (Dako; IR002; 1/10x), mouse anti-INSULIN (Sigma-Aldrich; 12018; 1/1000x), rabbit anti-C-peptide (Cell Signaling; 4593; 1/100x), rabbit anti-GFP (MBL International Corp; 598; 1/1000x), and mouse anti-GLUCAGON (Sigma-Aldrich; G2654; 1/1000x). The secondary antibodies used were Alexa 488-conjugated goat anti-mouse IgG (Invitrogen, A11029, 1/1000x), Alexa 488-conjugated donkey anti-guinea pig IgG (Jackson ImmunoResearch Laboratory, 706-546-148), AF647-conjugated donkey anti-rabbit IgG (Jackson ImmunoResearch Laboratory, 711-606-152), AF568-conjugated donkey anti-mouse IgG (BIOTIUM, 20105), Alexa 568-conjugated goat anti-guinea pig IgG (Invitrogen, A11075, 1/1000x), Alexa 568-conjugated goat anti-mouse IgG (Invitrogen, A11031, 1/1000x), Alexa 568-conjugated goat anti-rabbit IgG (Invitrogen, A11036, 1/1000x), and Alexa 633-conjugated goat anti-rabbit IgG (Invitrogen, A21072, 1/1000x). The cells were counterstained with DAPI (Thermo Fisher, D1306).

### (5) Quantitative real-time PCR

cDNA of mouse ES cells was prepared using VILO master mix, and real-time PCR analysis was conducted using Taqman qPCR assay (Applied Biosystems TaqMan gene expression assay ID: mouse Hprt1, Mm03024075_m1; mouse insulin 1, Mm01950294_s1) according to manufacturer's instruction. Gene expression levels were calculated using a calibration curve of each gene prepared by use of appropriate dilution series of a mixture of test samples. The expression level of each gene was corrected with a Hprt1 expression level.

(6) Measurement of glucose stimulated insulin secretion (GSIS) reaction of β cells derived from human iPS cells Time-dependent GSIS activity measurement was performed as described below. An aliquot of the obtained human iPS-β cells was washed several times for 10 minutes in low-glucose (LG: 3 mM glucose) HKRB buffer (COSMOBIO, PMC-PNIMG). The cells were further incubated for 30 minutes in LG-HKRB buffer in Transwell permeable support in a 24-well plate (CORNING, 3415), and only a portion of the supernatant was sampled for c-peptide measurement (LG 30 min). An additional amount of glucose was added to the cell suspension so that the final glucose concentration was increased from 3 to 20 mM to start high-glucose stimulation. The supernatant was sampled in a time-course manner (10, 30, and 60 minutes after the start of HG stimulation; HG 10 min, HG 30 min, and HG 60 min, respectively). Subsequently, 100 µM exendin 4 and 20 mM KCl were added in order, followed by incubation for 30 minutes (Ex4 30 min and KCl 30 min, respectively).

Batch-mode GSIS assay was carried out as described below. An aliquot of the obtained human iPS-β cells was washed with LG-KRBH as described above. Then, the cells were incubated for 30 minutes or 1 hour in LG-HKRB buffer and subsequently incubated for the same time thereas in HG-HKRB buffer. In some experiments, after washing of the cells, the cells were dispensed into some aliquots and incubated for 1 hour in HG-HKRB buffer containing 0.01% DMSO or several types of insulin secretagogues. The C-peptide concentration of a sample was measured using alphaLISA C-peptide kit (PerkinElmer, AL299F) or C-peptide ELISA and corrected with the DNA content of the cells used in the GSIS assay.

### (7) Measurement of amount of DNA

After GSIS assay, the cells were collected, and DNA thereof was purified using AllPrep DNA/RNA Micro Kit (Qiagen, 80284). Then, the concentration of the DNA was measured using Qubit assay (Lifescience technologies, Q32854).

### [References]

Nakashima, R., Morooka, M., Shiraki, N., Sakano, D., Ogaki, S., Kume, K. and Kume, S. (2015). Neural cells play an inhibitory role in pancreatic differentiation of pluripotent stem cells. Genes Cells 1028-1045.
Sakano, D., Shiraki, N., Kikawa, K., Yamazoe, T., Kataoka, M., Umeda, K., Araki, K., Mao, D., Matsumoto, S., Nakagata, N., et al. (2014). VMAT2 identified as a regulator of late-stage β-cell differentiation. Nat. Chem. Biol. 10, 141-8.
Shahjalal, H. M., Shiraki, N., Sakano, D., Kikawa, K., Ogaki, S., Baba, H., Kume, K. and Kume, S. (2014). Generation of insulin-producing β-like cells from human iPS cells in a defined and completely xeno-free culture system. J. Mol. Cell Biol. 0, 1-15.
Shiraki, N., Yoshida, T., Araki, K., Umezawa, A., Higuchi, Y., Goto, H., Kume, K. and Kume, S. (2008). Guided differentiation of embryonic stem cells into Pdx1-expressing regional-specific definitive endoderm. Stem Cells 26, 874-85.
Shiraki, N., Shiraki, Y., Tsuyama, T., Obata, F., Miura, M., Nagae, G., Aburatani, H., Kume, K., Endo, F. and Kume, S. (2014). Methionine metabolism regulates maintenance and differentiation of human pluripotent stem cells. Cell Metab. 19, 780-794.
Treff, N. R., Vincent, R. K., Budde, M. L., Browning, V. L., Magliocca, J. F., Kapur, V. and Odorico, J. S. (2006). Differentiation of Embryonic Stem Cells Conditionally Expressing Neurogenin 3. Stem Cells 24, 2529-2537.

All publications, patents and patent applications cited herein are incorporated herein by reference in their entirety.

## Claims

1. A method for producing insulin-producing cells by differentiating pluripotent stem cells into insulin-producing cells, comprising the step of three-dimensionally culturing cells in a medium containing a compound represented by formula (I): 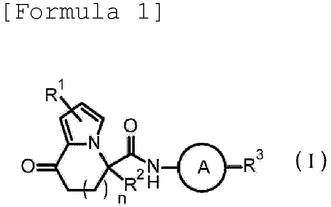 wherein each substituent is defined as follows:
R¹ represents a hydrogen atom, a halogen atom, or a C1-C6 alkyl group;
R² represents a hydrogen atom or a C1-C6 alkyl group;
R³ represents an aryl group optionally substituted with one to four substituents independently selected from a substituent group α, a C5-C10 cycloalkenyl group optionally substituted with one to four substituents independently selected from the substituent group α, or a heterocyclyl group optionally substituted with one to four substituents independently selected from the substituent group α;
the substituent group α includes a halogen atom, a cyano group, a carboxy group, a C1-C6 alkyl group, a Cl-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, a phenoxy group optionally substituted with one to four substituents independently selected from a substituent group β, a phenyl group optionally substituted with one to four substituents independently selected from the substituent group β, and a benzoyl group optionally substituted with one to four substituents independently selected from the substituent group β;
the substituent group β includes a halogen atom, a C1-C6 alkyl group, a C1-C6 alkoxy group, a halo-C1-C6 alkyl group, a halo-C1-C6 alkoxy group, and a (C1-C6 alkoxy)carbonyl group;
n represents 0 or 1; and
A represents a group represented by any one of formulae (i) to (iv) below: 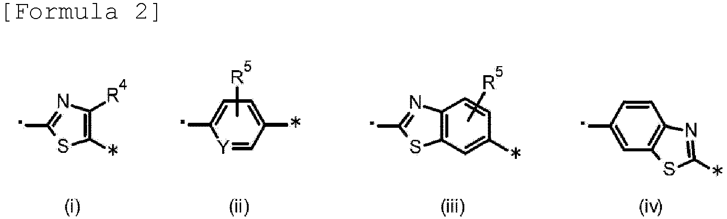 wherein each substituent is defined as follows:
• and * each represent a bond, where · is bonded to a nitrogen atom in an amido group of formula (I), and * is bonded to R³;
R⁴ represents a hydrogen atom, a C1-C6 alkyl group, a halo-C1-C6 alkyl group, or a (C1-C6 alkoxy)carbonyl group;
R⁵ represents a hydrogen atom, a halogen atom, or a C1-C6 alkyl group; and
Y represents N or CH; or a salt thereof.

2. A method for producing insulin-producing cells according to claim 1, wherein
R³ in the compound represented by formula (I) represents a naphthyl group, a 1,3-benzodioxolyl group, a 2,2-dihalo-1,3-benzodioxolyl group, a C5-C10 cycloalkenyl group, a phenyl group optionally substituted with one or two substituents independently selected from a substituent group α1, or a 5- or 6-membered heterocyclyl group optionally substituted with one or two substituents independently selected from the substituent group α1;
the substituent group α1 includes a halogen atom, a cyano group, a carboxy group, a phenoxy group, a benzoyl group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a 5- or 6-membered non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, and a phenyl group optionally substituted with one or two substituents independently selected from a substituent group β1; and
the substituent group β1 includes a halogen atom, a C1-C6 alkyl group, a C1-C6 alkoxy group, and a (C1-C6 alkoxy)carbonyl group.

3. A method for producing insulin-producing cells according to claim 1, wherein
R³ in the compound represented by formula (I) represents a naphthyl group, a 1,3-benzodioxolyl group, a 2,2-dihalo-1,3-benzodioxolyl group, a C5-C10 cycloalkenyl group, a phenyl group optionally substituted with one or two substituents independently selected from a substituent group α2, or a 5- or 6-membered heterocyclyl group optionally substituted with one or two substituents independently selected from a substituent group γ2;
the substituent group α2 includes a halogen atom, a cyano group, a carboxy group, a phenoxy group, a benzoyl group, a C1-C6 alkyl group, a C1-C6 alkoxy group, a halo-C1-C6 alkyl group, a halo-C1-C6 alkoxy group, a hydroxy C1-C6 alkyl group, a C1-C6 alkoxy C1-C6 alkoxy group, a (C1-C6 alkyl)carbonyl group, a (C1-C6 alkoxy)carbonyl group, a (C1-C6 alkoxy)carbonyloxy group, a phenyl C1-C6 alkoxy group, a 5- or 6-membered non-aromatic heterocyclyl group, a carbamoyl group optionally substituted with one or two C1-C6 alkyl groups, a C1-C6 alkoxy group substituted by a carbamoyl group optionally substituted with one or two C1-C6 alkyl groups, a sulfamoyl group substituted with one or two C1-C6 alkyl groups, and a phenyl group optionally substituted with one or two substituents independently selected from a substituent group β2;
the substituent group β2 includes a halogen atom, a C1-C6 alkyl group, and a C1-C6 alkoxy group; and
the substituent group γ2 includes a halogen atom, a C1-C6 alkyl group, a C1-C6 alkoxy group, a (C1-C6 alkyl)carbonyl group, and a (C1-C6 alkoxy)carbonyl group.

4. A method for producing insulin-producing cells according to claim 1, wherein
R³ in the compound represented by formula (I) represents a naphthyl group, a 1,3-benzodioxolyl group, a 2,2-difluoro-1,3-benzodioxolyl group, a C5-C8 cycloalken-1-yl group, a phenyl group optionally substituted with one or two substituents independently selected from a substituent group α3, or a 5- or 6-membered heterocyclyl group optionally substituted with one or two substituents independently selected from a substituent group γ3;
the substituent group α3 includes a halogen atom, a cyano group, a carboxy group, a phenoxy group, a benzoyl group, a C1-C4 alkyl group, a C1-C4 alkoxy group, a halo-C1-C2 alkyl group, a halo-C1-C2 alkoxy group, a hydroxy C1-C4 alkyl group, a C1-C2 alkoxy C1-C2 alkoxy group, a (C1-C4 alkyl)carbonyl group, a (C1-C4 alkoxy)carbonyl group, a (C1-C4 alkoxy)carbonyloxy group, a phenyl C1-C4 alkoxy group, a morpholin-1-yl group, a carbamoyl group optionally substituted with one or two C1-C4 alkyl groups, a C1-C2 alkoxy group substituted by a carbamoyl group optionally substituted with one or two C1-C4 alkyl groups, a sulfamoyl group substituted with one or two C1-C4 alkyl groups, and a phenyl group optionally substituted with one or two substituents independently selected from a substituent group β3;
the substituent group β3 includes a fluorine atom, a chlorine atom, a C1-C4 alkyl group, and a C1-C4 alkoxy group; and
the substituent group γ3 includes a halogen atom, a C1-C4 alkyl group, a C1-C4 alkoxy group, a (C1-C4 alkyl)carbonyl group, and a (C1-C4 alkoxy)carbonyl group.

5. A method for producing insulin-producing cells according to any one of claims 1 to 4, wherein R¹ in the compound represented by formula (I) represents a hydrogen atom, a chlorine atom, or a methyl group.

6. A method for producing insulin-producing cells according to any one of claims 1 to 5, wherein R² in the compound represented by formula (I) represents a hydrogen atom or a methyl group.

7. A method for producing insulin-producing cells according to any one of claims 1 to 6, wherein A in the compound represented by formula (I) represents a group represented by formula (i), and R⁴ represents a hydrogen atom, a C1-C6 alkyl group, a halo-C1-C6 alkyl group, or a (C1-C6 alkoxy)carbonyl group.

8. A method for producing insulin-producing cells according to claim 7, wherein R⁴ in the compound represented by formula (I) represents a hydrogen atom, a methyl group, or a trifluoromethyl group.

9. A method for producing insulin-producing cells according to any one of claims 1 to 6, wherein A in the compound represented by formula (I) represents a group represented by formula (ii), and R⁵ represents a hydrogen atom, a halogen atom, or a C1-C6 alkyl group.

10. A method for producing insulin-producing cells according to claim 9, wherein R⁵ in the compound represented by formula (I) represents a hydrogen atom, a fluorine atom, or a methyl group.

11. A method for producing insulin-producing cells according to any one of claims 1 to 6, wherein A in the compound represented by formula (I) represents a group represented by formula (iii), and R⁵ represents a hydrogen atom, a fluorine atom, or a methyl group.

12. A method for producing insulin-producing cells according to any one of claims 1 to 6, wherein A in the compound represented by formula (I) represents a group represented by formula (iv).

13. A method for producing insulin-producing cells according to any one of claims 1 to 12, wherein n in the compound represented by formula (I) represents 1.

14. A method for producing insulin-producing cells according to any one of claims 1 to 13, wherein R³ in the compound represented by formula (I) represents a 2,2-difluoro-1,3-benzodioxolyl group, a 1-tert-butoxycarbonyl-3,6-dihydro-2H-pyridin-4-yl group, or a phenyl group optionally substituted with one or two substituents independently selected from the group consisting of a fluorine atom, a chlorine atom, a trifluoromethyl group, a tert-butoxy group, a trifluoromethoxy group, a 2,2,2-trifluoroethoxy group, a benzyloxy group, and a phenoxy group.

15. A method for producing insulin-producing cells according to any one of claims 1 to 13, wherein R³ in the compound represented by formula (I) represents a phenyl group, or a phenyl group substituted at m position or p position with any one substituent selected from the group consisting of a fluorine atom, a chlorine atom, a trifluoromethyl group, a tert-butoxy group, a trifluoromethoxy group, a 2,2,2-trifluoroethoxy group, a benzyloxy group, and a phenoxy group.

16. A method for producing insulin-producing cells according to claim 1, wherein the compound represented by formula (I) is any one selected from the compound group shown below:

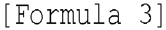

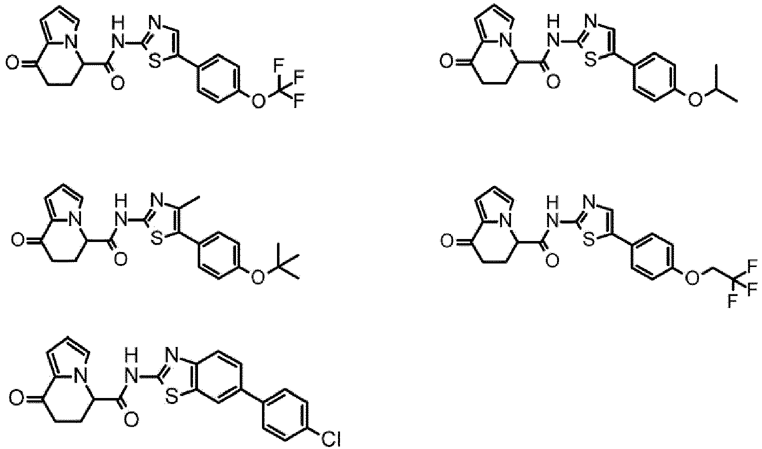

17. A method for producing insulin-producing cells according to claim 1, wherein the pluripotent stem cells are human ES cells or human iPS cells.

18. A method for producing insulin-producing cells according to claim 1, wherein the three-dimensional culture is performed in a low-adhesive or non-adhesive culture container.

19. A method for producing insulin-producing cells according to any one of claims 1 to 18, wherein the differentiation process from pluripotent stem cells into insulin-producing cells comprises steps 1 to 5 below, and at least one step selected from the group consisting of step 3, step 4 and step 5 comprises culturing cells in a medium containing the compound represented by formula (I) or a salt thereof:
step 1 of inducing definitive endoderm cells from pluripotent stem cells;
step 2 of inducing primitive gut tube cells from the definitive endoderm cells;
step 3 of inducing pancreatic progenitor cells from the primitive gut tube cells;
step 4 of inducing pancreatic endocrine progenitor cells from the pancreatic progenitor cells; and
step 5 of inducing insulin-producing cells from the pancreatic endocrine progenitor cells.

20. A method for producing insulin-producing cells according to claim 19, wherein in step 3, step 4 and step 5, cells are cultured in a medium containing the compound represented by formula (I) or a salt thereof.

21. A method for producing insulin-producing cells according to claim 19, wherein in step 1, pluripotent stem cells pretreated with a methionine-depleted medium is used.

22. Insulin-producing cells derived from pluripotent stem cells, the insulin-producing cells being produced by a method according to any one of claims 1 to 21.

23. A therapeutic drug for a disease caused by abnormal insulin secretion or insulin secretory disorder, comprising insulin-producing cells derived from pluripotent stem cells, the insulin-producing cells being produced by a method according to any one of claims 1 to 21.

24. The therapeutic drug according to claim 23, wherein the disease caused by abnormal insulin secretion or insulin secretory disorder is type 1 diabetes or type 2 diabetes.
